(19) Europäisches Patentamt
European Patent Office
Office européen des brevets

(11) EP 4 692 094 A1

(12) EUROPEAN PATENT APPLICATION
published in accordance with Art. 153(4) EPC

(43) Date of publication:
**11.02.2026 Bulletin 2026/07**

(21) Application number: **24778042.2**

(22) Date of filing: **27.03.2024**

(51) International Patent Classification (IPC):
***C07D 498/04*** *(2006.01)* ***C07D 493/00*** *(2006.01)*
***C07D 487/00*** *(2006.01)* ***A61K 31/5365*** *(2006.01)*
***A61P 35/00*** *(2006.01)*

(52) Cooperative Patent Classification (CPC):
**A61K 31/5365; A61P 35/00; C07D 487/00; C07D 493/00; C07D 498/04**

(86) International application number:
**PCT/CN2024/083976**

(87) International publication number:
**WO 2024/199254 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.03.2023 CN 202310308970**
**16.11.2023 CN 202311533391**

(71) Applicant: **Beijing Konruns Pharmaceutical Co., Ltd.**
**Beijing 101500 (CN)**

(72) Inventors:
- **YANG, Yang**
  **Beijing 101500 (CN)**
- **ZHANG, Lei**
  **Beijing 101500 (CN)**
- **PENG, Yong**
  **Beijing 101500 (CN)**
- **ZHANG, Xianjun**
  **Beijing 101500 (CN)**
- **ZHANG, Chen**
  **Beijing 101500 (CN)**
- **FENG, Kaiyu**
  **Beijing 101500 (CN)**
- **LI, Yuhua**
  **Beijing 101500 (CN)**
- **NIE, Xin**
  **Beijing 101500 (CN)**
- **SHI, Zijun**
  **Beijing 101500 (CN)**
- **MI, Zhen**
  **Beijing 101500 (CN)**
- **ZHANG, Yinyin**
  **Beijing 101500 (CN)**

(74) Representative: **Gille Hrabal Partnerschaftsgesellschaft mbB**
**Patentanwälte**
**Brucknerstraße 20**
**40593 Düsseldorf (DE)**

(54) **SULFONAMIDE COMPOUND, PHARMACEUTICAL COMPOSITION THEREOF AND USE THEREOF**

(57) The present disclosure relates to a sulfonyl compound, and a pharmaceutical composition thereof and a use thereof. The sulfonamide compound comprises a compound as shown in formula I. The sulfonamide compound competitively binds to KAT6A/KAT6B with the substrate at the binding pocket of the substrate acetyl coenzyme of KAT6A/KAT6B, inhibits acetyl coenzyme from binding to KAT6A/KAT6B to catalyze histone related sites, prevents gene expression that induces cancer cell growth, and is further used to treat tumor diseases related to KAT6A/KAT6B gene amplification or KAT6A/KAT6B protein overexpression.

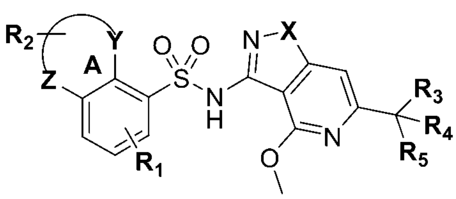

(I)

EP 4 692 094 A1

## Description

The present application claims priority to the following prior patent applications: a prior patent application with the application No. 202310308970.7 filed with the China National Intellectual Property Administration on Mar. 27, 2023 and entitled "SULFONAMIDE COMPOUND, PHARMACEUTICAL COMPOSITION THEREOF AND USE THEREOF", and a prior patent application with the application No. 202311533391.9 filed with the China National Intellectual Property Administration on Nov. 16, 2023 and entitled "SULFONAMIDE COMPOUND, PHARMACEUTICAL COMPOSITION THEREOF AND USE THEREOF", which are incorporated herein by reference in their entirety.

## TECHNICAL FIELD

The present disclosure pertains to the technical field of pharmaceuticals, and particularly relates to a sulfonamide compound and a pharmaceutical composition and use thereof.

## BACKGROUND

Histone acetyltransferases (HATs) are enzymes that acetylate specific sites of histones using acetyl-CoA (Ac-CoA) as a substrate, thereby playing a key role in a series of epigenetic pathways. Based on the structural features of the lysine acetyltransferase domain, these histone acetyltransferases can be classified into three major families: (i) p300/CBP; (ii) GNAT; and (iii) MYST. Among them, the largest family of HATs are MYST proteins, which are responsible for histone acetylation and involve in a wide range of cellular events such as DNA repair, replication, transcriptional activation, and the like, accounting for about one-third of the HATs in the human genome. Proteins in the MYST family have a conserved "MYST" domain, including KAT6A (MOZ/MYST3), KAT6B (Morf/MYST4/Qkf), KAT8 (Mof/MYST1), KAT5 (Tip60), and KAT7 (Hbo1/MYST2).

KAT6A and KAT6B are paralogous genes, and their protein products can form protein complexes with ING5, EAF6, BRPF1, BRPF2, or BRPF3 to acetylate the H3K23 site using the substrate acetyl-CoA (Ac-CoA). KAT6A is a target for recurrent chromosomal translocations and is a characteristic site that causes acute myeloid leukemia (AML). Translocations of KAT6A and KAT6B have been found in many patients with acute myeloid leukemia (AML), producing other HATs such as EP300 (the gene encodes the adenovirus E1A-associated cellular p300 transcriptional co-activator protein), NCOA2 (the protein encoded by the gene functions as nuclear hormone receptor 2), and NCOA3. In 2021, the M. Andrés Blanco research group found that KAT6A can epigenetically regulate the expression of key genes for AML, thereby promoting the development of AML. Therefore, inhibition of KAT6A activity could be a potential therapeutic for AML. Analysis of KAT genes in breast cancer clinical database shows that about 10% of patients with breast cancer, including those with the subtype three-negative breast cancer, have KAT6 gene amplification or overexpression of KAT6A and KAT6B proteins, and it is presumed that KAT6A or KAT6B is a breast cancer susceptibility gene. Moreover, studies have shown that a proportion of patients in a number of cancer types (including cervical cancer, ovarian cancer, lung adenocarcinoma, colorectal cancer, medulloblastoma, and prostate cancer) have 8p11 amplification and KAT6A overexpression, with KAT6A ranking as the 12th most frequently amplified gene in cancers.

In conclusion, the development of inhibitors targeting KAT6A and KAT6B can not only meet the unmet clinical requirements for ER+ breast cancer, triple-negative breast cancer, and acute myeloid leukemia (AML), but also has wide clinical value in other cancer types. However, only PF-07248144 (NCT04606446) from Pfizer is currently in clinical phase I. Moreover, as can be seen from the disclosed clinical data, the excessively long half-life of this molecule, combined with its very high exposure, leads to severe *in-vivo* accumulation and produce serious toxic and side effects. Therefore, extensive research efforts are still required for the development and optimization of KAT6A and KAT6B inhibitors to address or ameliorate the safety risks resulting from the severe accumulation.

## SUMMARY

The present disclosure provides a sulfonamide compound and a pharmaceutical composition and use thereof. The sulfonamide compound can be used for treating related cancer conditions by inhibiting histone acetylation, inhibiting transcription of related genes, and inhibiting cell growth.

In a first aspect, the present disclosure provides a sulfonamide compound or a stereoisomer, an isotopic derivative, a hydrate, a solvate, a prodrug, or a pharmaceutically acceptable salt thereof, wherein the sulfonamide compound has a structure represented by the following formula (I):

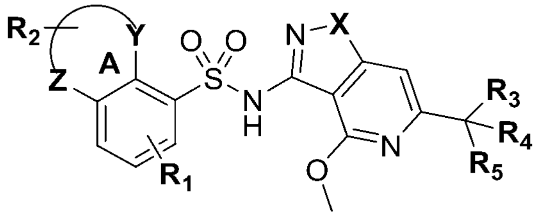

(I)

in formula (I): ring A is selected from a five-membered saturated ring, a six-membered saturated ring, a seven-membered saturated ring, a five-membered unsaturated ring, a six-membered unsaturated ring, and a C3-C8 spiro ring, bridged ring, aza-spiro ring, or aza-bridged ring;

$R_1$ is selected from absent, methoxy, ethoxy, halogen, cyano, nitro, hydroxyl, alkynyl, alkynyl containing an $R_6$ substituent, aryl, aryl containing an $R_6$ substituent, heteroaryl, heteroaryl containing an $R_6$ substituent, alkyl, alkyl containing an $R_6$ substituent, cycloalkyl, cycloalkyl containing an $R_6$ substituent, alkoxy, alkoxy containing an $R_6$ substituent, alkoxyalkyl, alkoxyalkyl containing an $R_6$ substituent, alkylamino, alkylamino containing an $R_6$ substituent, alkylmercapto, alkylmercapto containing an $R_6$ substituent, cycloalkoxy, cycloalkoxy containing an $R_6$ substituent, cycloalkylmercapto, cycloalkylmercapto containing an $R_6$ substituent, sulfonamido, sulfonamido containing an $R_6$ substituent, phosphoryl, and phosphoryl containing an $R_6$ substituent, wherein $R_6$ is selected from halogen, cyano, nitro, hydroxyl, alkynyl, aryl, heteroaryl, alkyl, cycloalkyl, alkoxy, alkoxyalkyl, alkylamino, alkylmercapto, cycloalkoxy, cycloalkylmercapto, sulfonamido, and phosphoryl, and $R_1$ and $R_6$ are different;

$R_2$ is selected from absent, halogen, cyano, nitro, hydroxyl, aryl, heteroaryl, alkyl, cycloalkyl, alkoxy, alkoxyalkyl, alkylamino, alkylmercapto, cycloalkoxy, cycloalkylmercapto, sulfonamido, and phosphoryl;

$R_3$ is selected from methyl, fluoro, and hydrogen;

$R_4$ is selected from methyl, fluoro, and hydrogen;

$R_5$ is selected from alkyl, cycloalkyl, azacyclyl, alkoxy, alkoxyalkyl, alkylamino, alkylcycloalkoxy, oxy, oxy substituted with $R_7$, amino, amino substituted with $R_7$, cycloalkyl, cycloalkyl substituted with $R_7$, azacycloalkyl, azacycloalkyl substituted with $R_7$, aryl, aryl substituted with $R_7$, heteroaryl, and heteroaryl substituted with $R_7$, wherein $R_7$ is selected from methyl, fluoro, difluoro, cyano, alkyl, cycloalkyl, alkoxy, alkylamino, alkylmercapto, and hydrogen, and $R_5$ and $R_7$ are different; Y is selected from C, CH, O, NH, and N-$R_8$, and Z is selected from C, CH, O, NH, and N-$R_8$, wherein $R_8$ is selected from hydrogen, methyl, ethyl, cyclobutyl, hydroxyethyl, hydroxymethyl, and -$CH_2OCH_3$;

X is selected from O and S.

In a second aspect, the present disclosure provides a sulfonamide compound or a stereoisomer, an isotopic derivative, a hydrate, a solvate, a prodrug, or a pharmaceutically acceptable salt thereof, wherein the sulfonamide compound has a structure represented by the following formula (II):

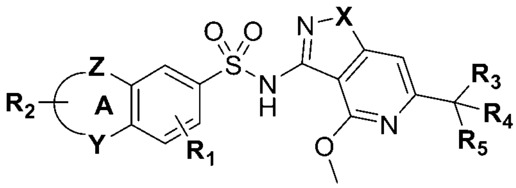

(II)

in formula (II): ring A is selected from a five-membered saturated ring, a six-membered saturated ring, a seven-membered saturated ring, a five-membered unsaturated ring, a six-membered unsaturated ring, and a C3-C8 spiro ring, bridged ring, aza-spiro ring, or aza-bridged ring;

$R_1$ is selected from absent, methoxy, ethoxy, halogen, cyano, nitro, hydroxyl, alkynyl, alkynyl containing an $R_6$ substituent,

aryl, aryl containing an $R_6$ substituent, heteroaryl, heteroaryl containing an $R_6$ substituent, alkyl, alkyl containing an $R_6$ substituent, cycloalkyl, cycloalkyl containing an $R_6$ substituent, alkoxy, alkoxy containing an $R_6$ substituent, alkoxyalkyl, alkoxyalkyl containing an $R_6$ substituent, alkylamino, alkylamino containing an $R_6$ substituent, alkylmercapto, alkylmercapto containing an $R_6$ substituent, cycloalkoxy, cycloalkoxy containing an $R_6$ substituent, cycloalkylmercapto, cycloalkylmercapto containing an $R_6$ substituent, sulfonamido, sulfonamido containing an $R_6$ substituent, phosphoryl, and phosphoryl containing an $R_6$ substituent, wherein $R_6$ is selected from halogen, cyano, nitro, hydroxyl, alkynyl, aryl, heteroaryl, alkyl, cycloalkyl, alkoxy, alkoxyalkyl, alkylamino, alkylmercapto, cycloalkoxy, cycloalkylmercapto, sulfonamido, and phosphoryl, and $R_1$ and $R_6$ are different;

$R_2$ is selected from absent, halogen, cyano, nitro, hydroxyl, aryl, heteroaryl, alkyl, cycloalkyl, alkoxy, alkoxyalkyl,

alkylamino, alkylmercapto, cycloalkoxy, cycloalkylmercapto, sulfonamido, and phosphoryl;

$R_3$ is selected from methyl, fluoro, and hydrogen;

$R_4$ is selected from methyl, fluoro, and hydrogen;

$R_5$ is selected from alkyl, cycloalkyl, azacyclyl, alkoxy, alkoxyalkyl, alkylamino, alkylcycloalkoxy, oxy, oxy substituted with $R_7$, amino, amino substituted with $R_7$, cycloalkyl, cycloalkyl substituted with $R_7$, azacycloalkyl, azacycloalkyl substituted with $R_7$, aryl, aryl substituted with $R_7$, heteroaryl, and heteroaryl substituted with $R_7$, wherein $R_7$ is selected from methyl, fluoro, difluoro, cyano, alkyl, cycloalkyl, alkoxy, alkylamino, alkylmercapto, and hydrogen, and $R_5$ and $R_7$ are different; Y is selected from C, CH, O, NH, and N-$R_8$, and Z is selected from C, CH, O, NH, and N-$R_8$, wherein $R_8$ is selected from hydrogen, methyl, ethyl, cyclobutyl, hydroxyethyl, hydroxymethyl, and -$CH_2OCH_3$;

X is selected from O and S.

In a third aspect, the present disclosure provides a sulfonamide compound or a stereoisomer, an isotopic derivative, a hydrate, a solvate, a prodrug, or a pharmaceutically acceptable salt thereof, wherein the sulfonamide compound has a structure represented by the following formula (III):

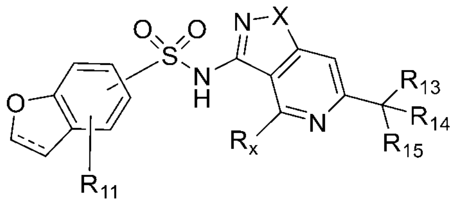

(III)

in formula (III), $\overline{- - -}$ is selected from a single bond and a double bond;

$R_{11}$, $R_{13}$, $R_{14}$, and $R_x$ are each independently selected from hydrogen, halogen, hydroxyl, substituted or unsubstituted C1-C6 alkyl, and substituted or unsubstituted C1-C6 alkoxy, wherein substituents of the C1-C6 alkyl and C1-C6 alkoxy are each independently selected from hydrogen, halogen, hydroxyl, and cyano. Preferably, $R_{11}$, $R_{13}$, $R_{14}$, and $R_x$ are each independently selected from substituted or unsubstituted C1-C3 alkyl and substituted or unsubstituted C1-C3 alkoxy, wherein substituents of the C1-C3 alkyl and C1-C3 alkoxy are each independently selected from hydrogen, halogen, hydroxyl, and cyano. Further preferably, $R_{11}$, $R_{13}$, $R_{14}$, and $R_x$ are each independently selected from substituted or unsubstituted methyl, ethyl, methoxy, and ethoxy, wherein substituents of the methyl, ethyl, methoxy, or ethoxy are each independently selected from halogen, hydroxyl, and cyano.

$R_{15}$ is selected from substituted or unsubstituted 5- to 6-membered heteroaryl, and preferably, $R_{15}$ is selected from substituted or unsubstituted 5-membered heteroaryl.

X is selected from O and S.

In a fourth aspect, the present disclosure provides a pharmaceutical composition comprising any one of the sulfonamide compound represented by any one of formulas (I) to (III) described above or the stereoisomer, the isotopic derivative, the hydrate, the solvate, the prodrug, or the pharmaceutically acceptable salt thereof, and an enantiomer, a diastereoisomer, a pharmaceutical salt, and a solvate of the sulfonamide compound represented by any one of formulas (I) to (III), as well as a pharmaceutically acceptable carrier.

In a fifth aspect, the present disclosure also provides use of the pharmaceutical composition as a medicament for the treatment of a disease associated with KAT6 amplification or KAT6 overexpression.

The technical solution of the present disclosure has at least the following beneficial effects: The present disclosure provides a sulfonamide compound having a benzoheterocyclic structure and a pyridoisoxazole structure, such that the sulfonamide compound competes with acetyl coenzyme, a substrate of KAT6A/KAT6B, for targeted binding to KAT6A/KAT6B at the binding pocket for the substrate. In the compound, the benzoheterocyclic structure can effectively improve the electron cloud density of the sulfonamide moiety, enhance the ability of the two oxygen atoms in the sulfonyl group to act as hydrogen bond acceptors, and increase the binding affinity of the compound for the pocket; after the phenyl ring is replaced by pyridine, the pyridoisoxazole structure effectively reduces the electron cloud density of N-O in the isoxazole, reduces the space and the degree of freedom of lone pair electrons of the oxygen, minimizes steric clashes, and makes the compound bind to the pocket better. The binding of the two significantly improves the ability of the compound to compete with the acetyl coenzyme for binding to KAT6A/KAT6B, inhibits the binding of the acetyl coenzyme to KAT6A/KAT6B to catalyze related sites of histones, prevents gene expression that induces the growth of cancer cells, and is further used to treat tumor diseases associated with KAT6A/KAT6B gene amplification or KAT6A/KAT6B protein overexpression.

In particular, the compound in the examples of the present disclosure has relatively good target selectivity; for example, the compound of the present disclosure has better selectivity against KAT6A and/or KAT6B, as well as better

bioavailability and higher tumor inhibitory activity, relative to other subtypes of KAT6.

## DETAILED DESCRIPTION

In order to better illustrate the content of the present disclosure and to facilitate understanding of the technical solutions of the present disclosure, the present disclosure is further illustrated in detail below. It should be understood that the described embodiments or examples are only some, but not all, embodiments or examples of the present disclosure. Based on the embodiments or examples in the present disclosure, all other embodiments or examples obtained by those of ordinary skill in the art without creative efforts shall fall within the protection scope of the claims in the present disclosure.

The present disclosure provides a sulfonamide compound, wherein the sulfonamide compound is selected from a compound represented by the following formula (I) or a stereoisomer, an isotopic derivative, a hydrate, a solvate, a prodrug, or a pharmaceutically acceptable salt thereof:

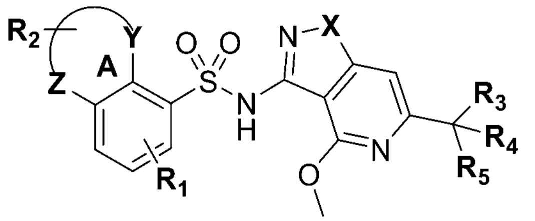

(I)

in formula (I): ring A is selected from a five-membered saturated ring, a six-membered saturated ring, a seven-membered saturated ring, a five-membered unsaturated ring, a six-membered unsaturated ring, and a C3-C8 spiro ring, bridged ring, aza-spiro ring, or aza-bridged ring;

$R_1$ is selected from absent, methoxy, ethoxy, halogen, cyano, nitro, hydroxyl, alkynyl, alkynyl containing an $R_6$ substituent, aryl, aryl containing an $R_6$ substituent, heteroaryl, heteroaryl containing an $R_6$ substituent, alkyl, alkyl containing an $R_6$ substituent, cycloalkyl, cycloalkyl containing an $R_6$ substituent, alkoxy, alkoxy containing an $R_6$ substituent, alkoxyalkyl, alkoxyalkyl containing an $R_6$ substituent, alkylamino, alkylamino containing an $R_6$ substituent, alkylmercapto, alkylmercapto containing an $R_6$ substituent, cycloalkoxy, cycloalkoxy containing an $R_6$ substituent, cycloalkylmercapto, cycloalkylmercapto containing an $R_6$ substituent, sulfonamido, sulfonamido containing an $R_6$ substituent, phosphoryl, and phosphoryl containing an $R_6$ substituent, wherein $R_6$ is selected from halogen, cyano, nitro, hydroxyl, alkynyl, aryl, heteroaryl, alkyl, cycloalkyl, alkoxy, alkoxyalkyl, alkylamino, alkylmercapto, cycloalkoxy, cycloalkylmercapto, sulfonamido, and phosphoryl, and $R_1$ and $R_6$ are different.

$R_2$ is selected from absent, halogen, cyano, nitro, hydroxyl, aryl, heteroaryl, alkyl, cycloalkyl, alkoxy, alkoxyalkyl, alkylamino, alkylmercapto, cycloalkoxy, cycloalkylmercapto, sulfonamido, and phosphoryl;

$R_3$ is selected from methyl, fluoro, and hydrogen; $R_4$ is selected from methyl, fluoro, and hydrogen;

$R_5$ is selected from alkyl, cycloalkyl, azacyclyl, alkoxy, alkoxyalkyl, alkylamino, alkylcycloalkoxy, oxy, oxy substituted with $R_7$, amino, amino substituted with $R_7$, cycloalkyl, cycloalkyl substituted with $R_7$, azacycloalkyl, azacycloalkyl substituted with $R_7$, aryl, aryl substituted with $R_7$, heteroaryl, and heteroaryl substituted with $R_7$, wherein $R_7$ is selected from methyl, fluoro, difluoro, cyano, alkyl, cycloalkyl, alkoxy, alkylamino, alkylmercapto, and hydrogen, and $R_5$ and $R_7$ are different;

Y is selected from C, CH, O, NH, and N-$R_8$, and Z is selected from C, CH, O, NH, and N-$R_8$, wherein $R_8$ is selected from hydrogen, methyl, ethyl, cyclobutyl, hydroxyethyl, hydroxymethyl, and $-CH_2OCH_3$;

X is selected from O and S.

In some embodiments, the sulfonamide compound is selected from a compound represented by the following formula (II) or a stereoisomer, an isotopic derivative, a hydrate, a solvate, a prodrug, or a pharmaceutically acceptable salt thereof:

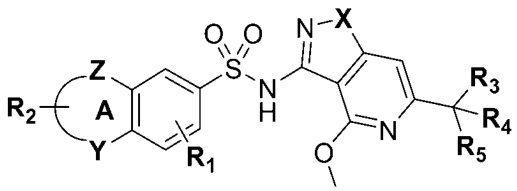

(II)

in formula (II): ring A is selected from a five-membered saturated ring, a six-membered saturated ring, a seven-membered saturated ring, a five-membered unsaturated ring, a six-membered unsaturated ring, and a C3-C8 spiro ring, bridged ring, aza-spiro ring, or aza-bridged ring;

$R_1$ is selected from absent, methoxy, ethoxy, halogen, cyano, nitro, hydroxyl, alkynyl, alkynyl containing an $R_6$ substituent, aryl, aryl containing an $R_6$ substituent, heteroaryl, heteroaryl containing an $R_6$ substituent, alkyl, alkyl containing an $R_6$ substituent, cycloalkyl, cycloalkyl containing an $R_6$ substituent, alkoxy, alkoxy containing an $R_6$ substituent, alkoxyalkyl, alkoxyalkyl containing an $R_6$ substituent, alkylamino, alkylamino containing an $R_6$ substituent, alkylmercapto, alkylmercapto containing an $R_6$ substituent, cycloalkoxy, cycloalkoxy containing an $R_6$ substituent, cycloalkylmercapto, cycloalkylmercapto containing an $R_6$ substituent, sulfonamido, sulfonamido containing an $R_6$ substituent, phosphoryl, and phosphoryl containing an $R_6$ substituent, wherein $R_6$ is selected from halogen, cyano, nitro, hydroxyl, alkynyl, aryl, heteroaryl, alkyl, cycloalkyl, alkoxy, alkoxyalkyl, alkylamino, alkylmercapto, cycloalkoxy, cycloalkylmercapto, sulfonamido, and phosphoryl, and $R_1$ and $R_6$ are different;

$R_2$ is selected from absent, halogen, cyano, nitro, hydroxyl, aryl, heteroaryl, alkyl, cycloalkyl, alkoxy, alkoxyalkyl, alkylamino, alkylmercapto, cycloalkoxy, cycloalkylmercapto, sulfonamido, and phosphoryl;

$R_3$ is selected from methyl, fluoro, and hydrogen; $R_4$ is selected from methyl, fluoro, and hydrogen;

$R_5$ is selected from alkyl, cycloalkyl, azacyclyl, alkoxy, alkoxyalkyl, alkylamino, alkylcycloalkoxy, oxy, oxy substituted with $R_7$, amino, amino substituted with $R_7$, cycloalkyl, cycloalkyl substituted with $R_7$, azacycloalkyl, azacycloalkyl substituted with $R_7$, aryl, aryl substituted with $R_7$, heteroaryl, and heteroaryl substituted with $R_7$, wherein $R_7$ is selected from methyl, fluoro, difluoro, cyano, alkyl, cycloalkyl, alkoxy, alkylamino, alkylmercapto, and hydrogen, and $R_5$ and $R_7$ are different;

Z is selected from C, CH, O, NH, and N-$R_8$, and Y is selected from C, CH, O, NH, and N-$R_8$, wherein $R_8$ is selected from hydrogen, methyl, ethyl, cyclobutyl, hydroxyethyl, hydroxymethyl, and $-CH_2OCH_3$; X is selected from O and S.

In one embodiment of the present disclosure, the sulfonamide compound is a compound represented by formula (I) or formula (II) or a stereoisomer, an isotopic derivative, a hydrate, a solvate, a prodrug, or a pharmaceutically acceptable salt thereof, wherein:

ring A is selected from a five-membered saturated ring, a six-membered saturated ring, a five-membered unsaturated ring, and a six-membered unsaturated ring;

$R_1$ is selected from hydrogen, halogen, cyano, nitro, hydroxyl, C2-C6 alkynyl containing an $R_6$ substituent, C1-C6 alkyl containing an $R_6$ substituent, and C1-C6 alkoxy containing an $R_6$ substituent, wherein the $R_6$ substituent is selected from halogen, cyano, nitro, hydroxyl, C1-C6 alkyl, C3-C6 cycloalkyl, and C1-C6 alkoxy, and $R_1$ and $R_6$ are different.

$R_2$ is selected from hydrogen, halogen, cyano, nitro, hydroxyl, C1-C6 alkyl, C3-C6 cycloalkyl, and C1-C6 alkoxy; $R_3$ is selected from methyl, fluoro, and hydrogen; $R_4$ is selected from methyl, fluoro, and hydrogen;

$R_5$ is selected from heteroaryl containing an $R_6$ substituent and aryl containing an $R_6$ substituent, wherein the $R_6$ substituent is selected from hydrogen, fluoro, cyano, C1-C6 alkyl, C3-C6 cycloalkyl, and C1-C6 alkoxy;

Y is selected from CH, $CH_2$, O, S, N, and N-$R_8$, wherein $R_8$ is selected from hydrogen, methyl, and ethyl;

Z is selected from CH, $CH_2$, O, S, N, and N-$R_8$, wherein $R_8$ is selected from hydrogen, methyl, and ethyl;

X is selected from O and S.

In another embodiment of the present disclosure, the sulfonamide compound is a compound represented by formula (I) or formula (II) or a stereoisomer, an isotopic derivative, a hydrate, a solvate, a prodrug, or a pharmaceutically acceptable salt thereof, wherein:

ring A is selected from a five-membered saturated ring, a six-membered saturated ring, a five-membered unsaturated ring, and a six-membered unsaturated ring;

$R_1$ is selected from hydrogen, halogen, cyano, nitro, hydroxyl, C2-C4 alkynyl containing an $R_6$ substituent, C1-C4 alkyl containing an $R_6$ substituent, and C1-C4 alkoxy containing an $R_6$ substituent, wherein $R_6$ is selected from halogen, hydroxyl, C1-C4 alkyl, C3-C4 cycloalkyl, and C1-C4 alkoxy, and $R_1$ and $R_6$ are different.

$R_2$ is selected from hydrogen, halogen, hydroxyl, C1-C4 alkyl, and C1-C4 alkoxy;

$R_3$ is selected from methyl, fluoro, and hydrogen; $R_4$ is selected from methyl, fluoro, and hydrogen;

$R_5$ is selected from heteroaryl containing an $R_6$ substituent and aryl containing an $R_6$ substituent, wherein the $R_6$ substituent is selected from hydrogen, fluoro, cyano, C1-C4 alkyl, C3-C4 cycloalkyl, and C1-C4 alkoxy;

Y is selected from CH, $CH_2$, O, S, N, and N-$R_8$, wherein $R_8$ is selected from hydrogen, methyl, and ethyl;

Z is selected from CH, $CH_2$, O, S, N, and N-$R_8$, wherein $R_8$ is selected from hydrogen, methyl, and ethyl;

X is selected from O and S.

In still another embodiment of the present disclosure, the sulfonamide compound is a compound represented by formula (I) or formula (II) or a stereoisomer, an isotopic derivative, a hydrate, a solvate, a prodrug, or a pharmaceutically

acceptable salt thereof, wherein:

ring A is selected from a five-membered saturated ring, a six-membered saturated ring, a five-membered unsaturated ring, and a six-membered unsaturated ring;
$R_1$ is selected from hydrogen, halogen, methoxy, ethoxy, methyl, ethyl, and C2-C4 alkynyl containing an $R_6$ substituent, wherein the $R_6$ substituent is selected from halogen, hydroxyl, methyl, ethyl, methoxy, ethoxy, cyclopropyl, and cyclobutyl;
$R_2$ is selected from hydrogen, halogen, hydroxyl, methyl, ethyl, methoxy, and ethoxy;
$R_3$ is selected from methyl, fluoro, and hydrogen; $R_4$ is selected from methyl, fluoro, and hydrogen;
$R_5$ is selected from heteroaryl containing an $R_6$ substituent and aryl containing an $R_6$ substituent, wherein the $R_6$ substituent is selected from hydrogen, fluoro, methyl, ethyl, methoxy, ethoxy, cyclopropyl, and cyclobutyl;
Y is selected from CH, $CH_2$, O, S, N, and N-$R_8$, wherein $R_8$ is selected from hydrogen, methyl, and ethyl;
Z is selected from CH, $CH_2$, O, S, N, and N-$R_8$, wherein $R_8$ is selected from hydrogen, methyl, and ethyl;
X is selected from O and S.

In some embodiments, the sulfonamide compound is a compound represented by formula (I) or formula (II) or a stereoisomer, an isotopic derivative, a hydrate, a solvate, a prodrug, or a pharmaceutically acceptable salt thereof, wherein

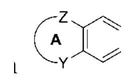

is selected from fused bicyclic ring structures as shown below:

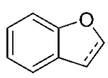 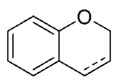 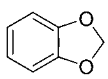 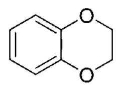 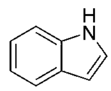 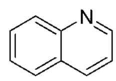

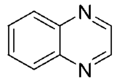 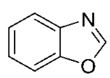 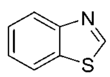 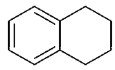 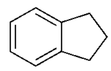

wherein - - - is selected from a single bond and a double bond;
preferably, the aminosulfonyl fragment (-S(=O)$_2$-NH-) and R1 are located on the phenyl of the fused bicyclic ring structure.
In some embodiments, the sulfonamide compound is a compound represented by formula (III) or a stereoisomer, an isotopic derivative, a hydrate, a solvate, a prodrug, or a pharmaceutically acceptable salt thereof:

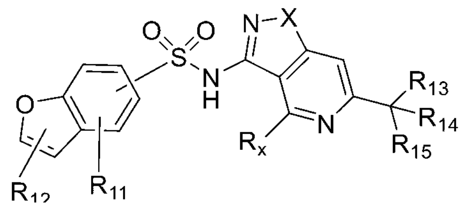 (III)

wherein - - - is selected from a single bond and a double bond;

$R_{11}$, $R_{12}$, $R_{13}$, $R_{14}$, and $R_x$ are each independently selected from hydrogen, halogen, hydroxyl, substituted or unsubstituted C1-C6 alkyl, substituted or unsubstituted C1-C6 alkoxy, and substituted or unsubstituted C2-C6 alkynyl. Preferably, $R_{11}$, $R_{12}$, $R_{13}$, $R_{14}$, and $R_x$ are each independently selected from hydrogen, halogen, hydroxyl, substituted or unsubstituted C1-C3 alkyl, substituted or unsubstituted C1-C3 alkoxy, and substituted or unsubstituted C2-C4 alkynyl. Further preferably, $R_{11}$, $R_{12}$, $R_{13}$, $R_{14}$, and $R_x$ are each independently selected from hydrogen, halogen, hydroxyl, substituted or unsubstituted methyl, substituted or unsubstituted ethyl, substituted or unsubstituted methoxy, substituted or unsubstituted ethoxy, and substituted or unsubstituted ethynyl. The substituents in the groups described above are each independently selected from halogen, hydroxyl, cyano, C1-C6 alkyl, C1-C6 alkoxy, and C3-C6 cycloalkyl. Preferably, the substituents in the groups described above are each independently selected from halogen, hydroxyl, cyano, C1-C3 alkyl, C1-C3 alkoxy, and C3-C4 cycloalkyl, and further preferably, the substituents in the groups described above are each independently selected from halogen, hydroxyl, cyano, methyl,

ethyl, methoxy, ethoxy, cyclopropyl, and cyclobutyl;

$R_{15}$ is selected from 5- to 6-membered heteroaryl containing an $R_6$ substituent. Preferably, $R_{15}$ is selected from 5-membered heteroaryl containing an $R_6$ substituent. The $R_6$ substituent is selected from hydrogen, halogen, hydroxyl, cyano, C1-C6 alkyl, C3-C6 cycloalkyl, and C1-C6 alkoxy. Preferably, $R_6$ is selected from hydrogen, halogen, hydroxyl, cyano, C1-C3 alkyl, C3-C4 cycloalkyl, and C1-C3 alkoxy. Further preferably, $R_6$ is selected from hydrogen, fluoro, methyl, ethyl, methoxy, ethoxy, cyclopropyl, and cyclobutyl;

X is selected from O and S.

In some embodiments, the aminosulfonyl fragment ($-S(=O)_2-NH-$) and $R_{11}$ in formula (III) are located on the phenyl of the oxygen-containing fused bicyclic ring. Preferably, the aminosulfonyl fragment ($-S(=O)_2-NH-$) is located at position 6 or 7 of the oxygen-containing fused bicyclic ring, and $R_{11}$ is located at position 5 or 6 of the fused bicyclic ring. Further preferably, the aminosulfonyl fragment ($-S(=O)_2-NH-$) and $R_{11}$ are located at ortho positions of the phenyl ring in the oxygen-containing fused bicyclic ring.

In one embodiment, the aminosulfonyl fragment ($-S(=O)_2-NH-$) is located at position 6 of the oxygen-containing fused bicyclic ring, and $R_{11}$ is located at position 5 of the oxygen-containing fused bicyclic ring; in another embodiment, the aminosulfonyl fragment ($-S(=O)_2-NH-$) is located at position 7 of the oxygen-containing fused bicyclic ring, and $R_{11}$ is located at position 6 of the oxygen-containing fused bicyclic ring.

In other embodiments, the sulfonamide compound is a compound having a structure of formula (III), wherein

$\overline{---}$ is selected from a single bond and a double bond;

$R_{11}$ is selected from hydrogen, C1-C6 alkoxy, C2-C6 alkynyl containing a C3-C6 cycloalkyl substituent; preferably, $R_{11}$ is selected from hydrogen, methoxy, ethoxy, ethynyl, and cyclopropyl-ethynyl.

$R_{12}$ is selected from hydrogen and C1-C6 alkyl. Preferably, $R_{12}$ is selected from hydrogen, methyl, and ethyl.

$R_{13}$ and $R_{14}$ are each independently selected from hydrogen and halogen. Preferably, $R_{13}$ and $R_{14}$ are each independently selected from hydrogen and F.

$R_x$ is selected from hydrogen and C1-C6 alkoxy. Preferably, $R_x$ is selected from hydrogen, methoxy, and ethoxy. $R_{15}$ is selected from

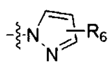
;

$R_6$ is selected from hydrogen, halogen, and C1-C6 alkyl. Preferably, $R_6$ is selected from hydrogen, F, methyl, and ethyl.

X is selected from O.

In some embodiments, $R_5$ in the compound represented by formula (I) or formula (II) and $R_{15}$ in the compound represented by formula (III) may be each independently selected from any one of the following groups, wherein $R_6$ independently has the definition in each of the structural formulas described above:

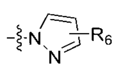 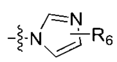 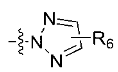 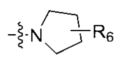 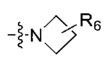 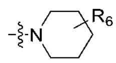

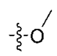 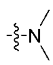 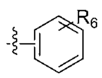 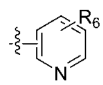 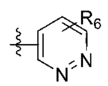 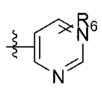 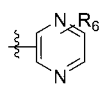

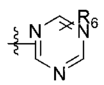 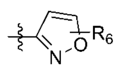 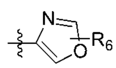 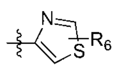 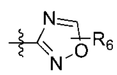 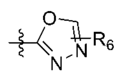

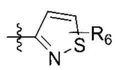
.

Preferably, $R_5$ or $R_{15}$ is independently selected from any one of the following groups:

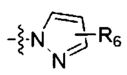 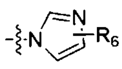 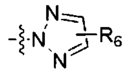

.

Further preferably, $R_5$ or $R_{15}$ is independently selected from

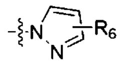.

In some embodiments, the sulfonamide compound is a compound represented by formula (IV) or a stereoisomer, an isotopic derivative, a hydrate, a solvate, a prodrug, or a pharmaceutically acceptable salt thereof:

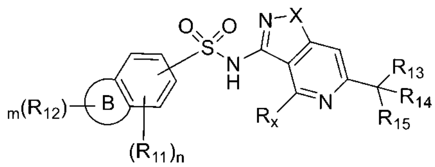

(IV)

wherein

ring B, together with the phenyl to which it is fused, forms a 9- to 10-membered fused heterocyclic ring, wherein the fused heterocyclic ring is a partially saturated or aromatic fused heterocyclic ring, and the ring B moiety contains 1-3 heteroatoms selected from N, O, and S; or the ring B moiety, together with the phenyl to which it is fused, forms a C9-C10 fused carbocyclic ring, wherein the fused carbocyclic ring is a partially saturated or aromatic fused carbocyclic ring;

$R_{11}$, $R_{12}$, $R_{13}$, $R_{14}$, and $R_x$ are identical or different, and are each independently selected from hydrogen, deuterium, halogen, hydroxyl, cyano, substituted or unsubstituted alkyl, substituted or unsubstituted alkoxy, substituted or unsubstituted cycloalkyl, and substituted or unsubstituted alkynyl; preferably, $R_{11}$, $R_{12}$, $R_{13}$, $R_{14}$, and $R_x$ are identical or different, and are each independently selected from hydrogen, deuterium, halogen, hydroxyl, cyano, substituted or unsubstituted C1-C6 alkyl, substituted or unsubstituted C1-C6 alkoxy, substituted or unsubstituted C3-C6 cycloalkyl, and substituted or unsubstituted C2-C6 alkynyl; further preferably, $R_{11}$, $R_{12}$, $R_{13}$, $R_{14}$, and $R_x$ are identical or different, and are each independently selected from hydrogen, halogen, hydroxyl, substituted or unsubstituted C1-C3 alkyl, substituted or unsubstituted C 1-C3 alkoxy, and substituted or unsubstituted C2-C4 alkynyl; more preferably, $R_{11}$, $R_{12}$, $R_{13}$, $R_{14}$, and $R_x$ are identical or different, and are each independently selected from hydrogen, halogen, hydroxyl, substituted or unsubstituted methyl, substituted or unsubstituted ethyl, substituted or unsubstituted methoxy, substituted or unsubstituted ethoxy, and substituted or unsubstituted ethynyl, wherein preferably, when the groups described above are substituted, the substituents are identical or different, and are each independently selected from halogen, hydroxyl, cyano, C1-C6 alkyl, C1-C6 alkoxy, and C3-C6 cycloalkyl; preferably, when the groups described above are substituted, the substituents are identical or different, and are each independently selected from halogen, hydroxyl, cyano, C1-C3 alkyl, C1-C3 alkoxy, and C3-C4 cycloalkyl; further preferably, when the groups described above are substituted, the substituents are identical or different, and are each independently selected from halogen, hydroxyl, cyano, methyl, ethyl, methoxy, ethoxy, cyclopropyl, and cyclobutyl;

$R_{15}$ is selected from the following groups optionally substituted with an $R_6$ substituent: hydroxyl, amino, alkoxy, heterocyclyl, and heteroaryl; preferably, $R_{15}$ is selected from the following groups optionally substituted with an $R_6$ substituent: hydroxyl, amino, C1-C6 alkoxy, 4- to 6-membered heterocyclyl, and 5- to 6-membered heteroaryl, wherein the heterocyclyl contains 1-2 heteroatoms selected from N, O, and S, and the heteroaryl contains 1, 2, or 3 heteroatoms selected from N, O, and S; when present, the $R_6$ substituent is selected from hydrogen, halogen, hydroxyl, cyano, alkyl, cycloalkyl, alkoxy, and alkylcarbonyl; the $R_6$ substituent is preferably selected from hydrogen, halogen, hydroxyl, cyano, C1-C6 alkyl, C3-C6 cycloalkyl, C1-C6 alkoxy, and C1-C6 alkylcarbonyl;

X is selected from O and S;

m and n are identical or different, and are each independently selected from integers selected from 0, 1, 2, and 3.

In one embodiment of the compound represented by formula (IV), ring B, together with the phenyl to which it is fused, forms a 9- to 10-membered fused heterocyclic ring, wherein the fused heterocyclic ring is a partially saturated or aromatic fused heterocyclic ring, the ring B moiety contains 1-2 heteroatoms selected from N, O, and S, and the heteroatom is linked to the fused phenyl; or the ring B moiety, together with the phenyl to which it is fused, forms a

C9-C10 fused carbocyclic ring, wherein the fused carbocyclic ring is a partially saturated or aromatic fused carbocyclic ring, and preferably, the fused carbocyclic ring is a partially saturated fused carbocyclic ring.

In one embodiment of the compound represented by formula (IV),

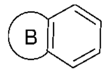

,

is selected from fused bicyclic ring structures as shown below:

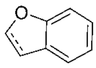
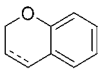
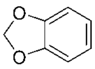
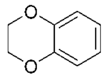
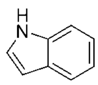
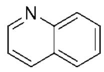

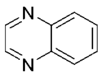
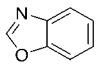
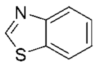
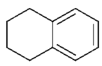
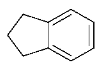

wherein is selected from a single bond and a double bond; preferably, both the aminosulfonyl fragment (-S(=O)$_2$-NH-) and $R_{11}$ are bonded to the phenyl in the fused bicyclic ring structure

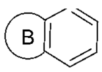

; more preferably, in the case that $R_{11}$ is not hydrogen, the aminosulfonyl fragment (-S(=O)$_2$-NH-) and at least one $R_{11}$ are bonded to ortho positions of the phenyl in the fused bicyclic ring structure

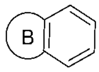

. As an example, the sulfonyl in the aminosulfonyl fragment (-S(=O)$_2$-NH-) may be bonded to a non-fused common carbon atom of the phenyl ring in

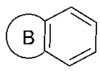

, e.g., the carbon atom at position a, b, c, or d in the following formula:

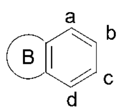

In one embodiment of the compound represented by formula (IV), $R_x$ is selected from substituted or unsubstituted C1-C6 alkoxy, preferably substituted or unsubstituted C1-C3 alkoxy, and more preferably substituted or unsubstituted methoxy.

In one embodiment of the compound represented by formula (IV), $R_{11}$ is selected from hydrogen, deuterium, halogen, cyano, substituted or unsubstituted C1-C6 alkyl, substituted or unsubstituted C1-C6 alkoxy, substituted or unsubstituted C3-C6 cycloalkyl, and C2-C6 alkynyl optionally substituted with C3-C6 cycloalkyl, preferably hydrogen, halogen, substituted or unsubstituted C1-C3 alkyl, substituted or unsubstituted C1-C3 alkoxy, substituted or unsubstituted C3-C4 cycloalkyl, and C2-C4 alkynyl optionally substituted with C3-C4 cycloalkyl, and more preferably H, F, Cl, cyano, methoxy, cyclopropyl, ethynyl, and cyclopropylalkynyl.

In one embodiment of the compound represented by formula (IV), $R_{12}$ is selected from hydrogen, deuterium, halogen, substituted or unsubstituted C1-C6 alkyl, and substituted or unsubstituted C1-C6 alkoxy, preferably hydrogen, deuterium, halogen, substituted or unsubstituted C1-C3 alkyl, and substituted or unsubstituted C1-C3 alkoxy, and more preferably H, deuterium, F, Cl, methyl, and methoxy.

In one embodiment of the compound represented by formula (IV), $R_{13}$ and $R_{14}$ are each independently selected from hydrogen, deuterium, halogen, and substituted or unsubstituted C1-C6 alkyl, preferably hydrogen, deuterium, F, Cl,

Br, and substituted or unsubstituted C1-C3 alkyl, and more preferably hydrogen, deuterium, and F.

In one embodiment of the compound represented by formula (IV), $R_{15}$ is selected from the following groups optionally substituted with an $R_6$ substituent: 4- to 6-membered heterocyclyl and 5- to 6-membered heteroaryl, wherein the heterocyclyl contains 1-2 heteroatoms selected from N, and the heteroaryl contains 1-3 heteroatoms selected from N; preferably 4- to 5-membered heterocyclyl and 5-membered heteroaryl, wherein the heterocyclyl contains 1 heteroatom selected from N, and the heteroaryl contains 1-3 heteroatoms selected from N; more preferably 5-membered heteroaryl, wherein the heteroaryl contains 2 heteroatoms selected from N; particularly, $R_{15}$ is selected from the following groups:

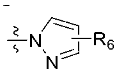, 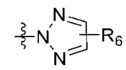, 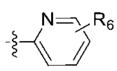, 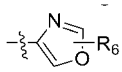;

the $R_6$ substituent is selected from hydrogen, deuterium, halogen, C1-C6 alkyl, C1-C6 alkoxy, and C1-C6 alkylcarbonyl; preferably, the $R_6$ substituent is selected from hydrogen, halogen, C1-C3 alkyl, C1-C3 alkoxy, and C1-C3 alkylcarbonyl; more preferably, the $R_6$ substituent is selected from hydrogen, fluoro, methyl, ethyl, methoxy, ethoxy, and acetyl; most preferably, the $R_6$ substituent is selected from hydrogen, fluoro, and methyl.

The present disclosure also provides a compound represented by formula (I), (II), (III), or (IV), or a stereoisomer, an isotopic derivative, a hydrate, a solvate, a prodrug, or a pharmaceutically acceptable salt thereof.

As a preferred embodiment, the compound represented by formula (I), (II), (III), or (IV), or a deuterated substance, the hydrate, the solvate, the stereoisomer, or the pharmaceutically acceptable salt thereof may be selected from the following compounds or stereoisomers, isotopic derivatives, hydrates, solvates, prodrugs, or pharmaceutically acceptable salts thereof:

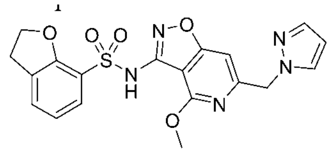, 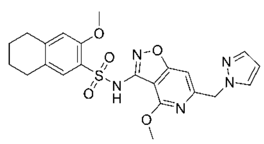, 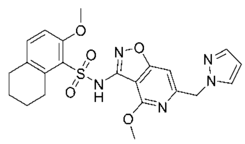, 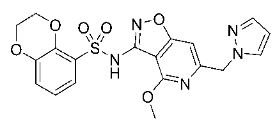,

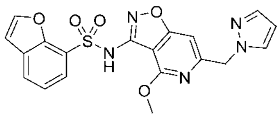, 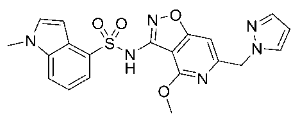, 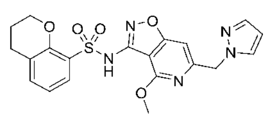, 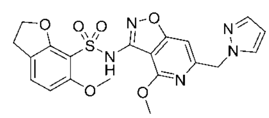,

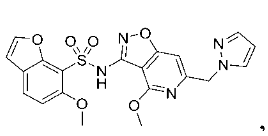, 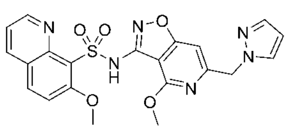, 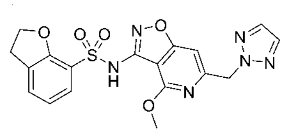, 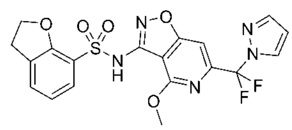

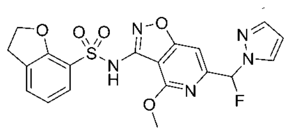, 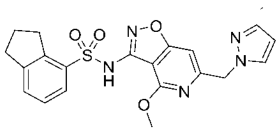, 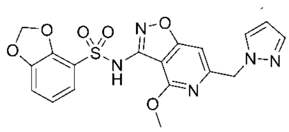, 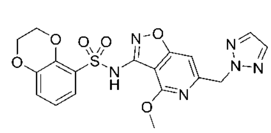

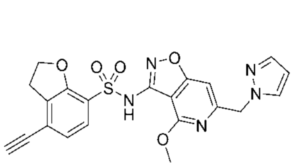, 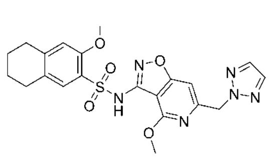, 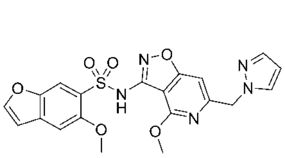, 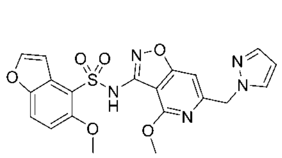

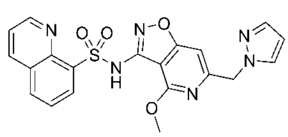, 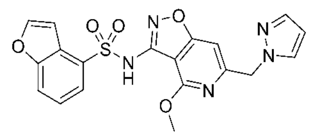, 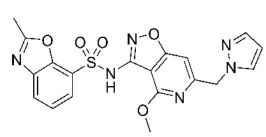, 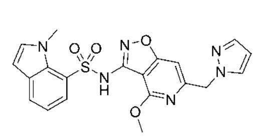

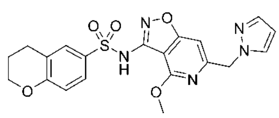, 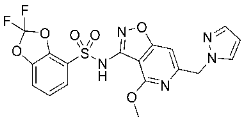, 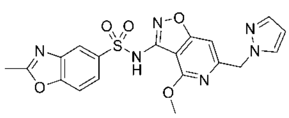, 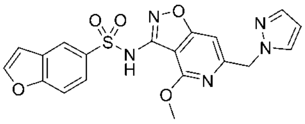,

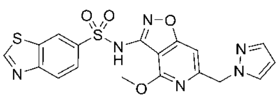, 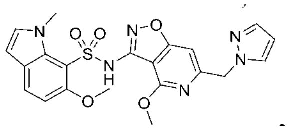, 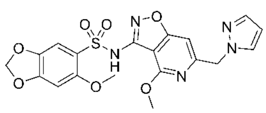, 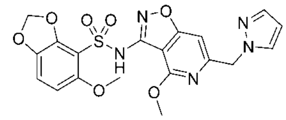,

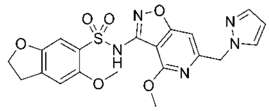, 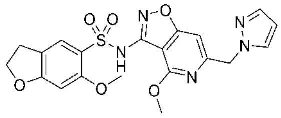, 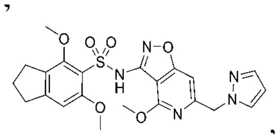, 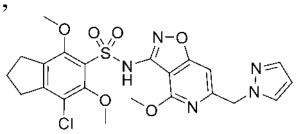,

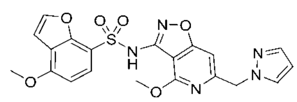, 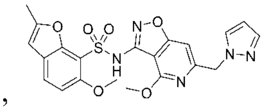, 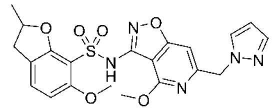, 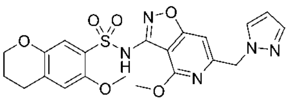,

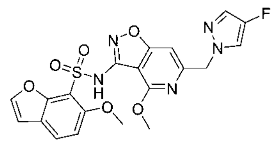, 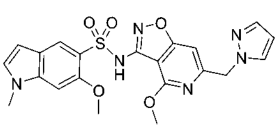, 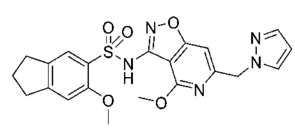, 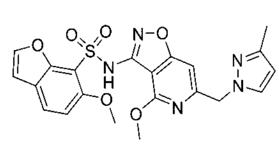,

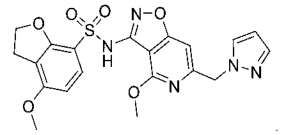, 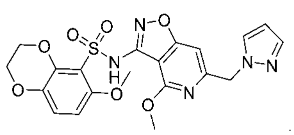, 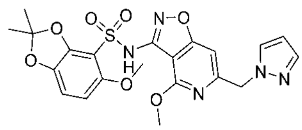, 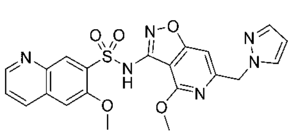,

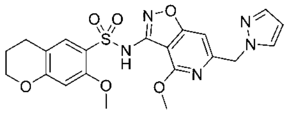, 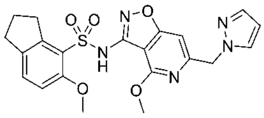, 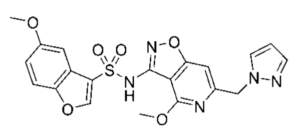, 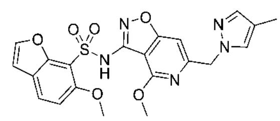,

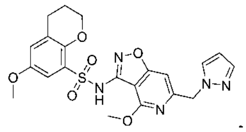, 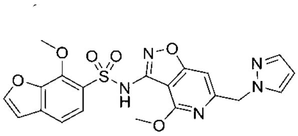, 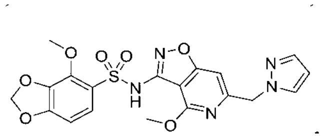, 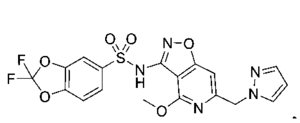,

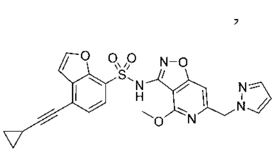, 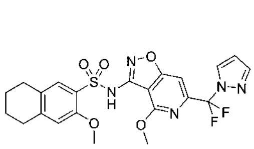, 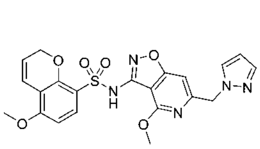, 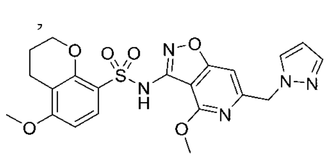,

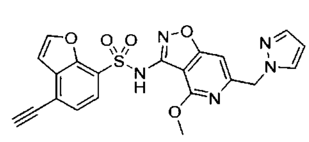, 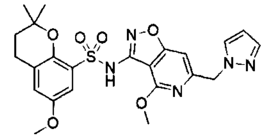, 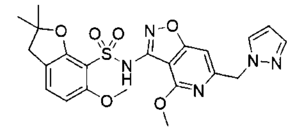, 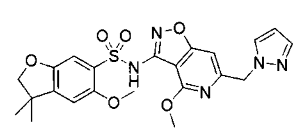

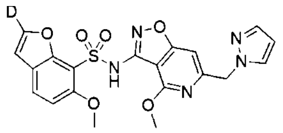, 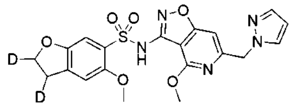, 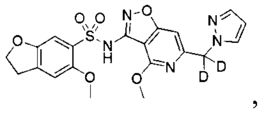, 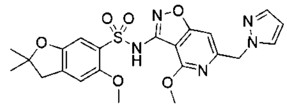,

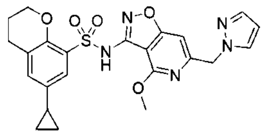, 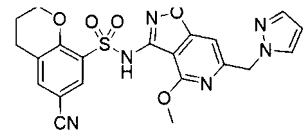, and 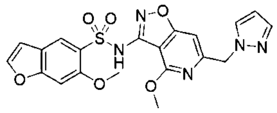.

The following compounds or stereoisomers, isotopic derivatives, hydrates, solvates, prodrugs, or pharmaceutically acceptable salts thereof are preferred in the present disclosure:

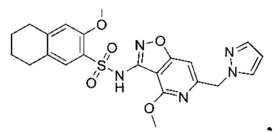, 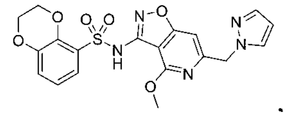, 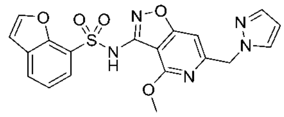, 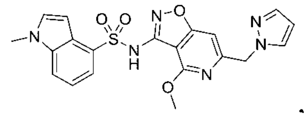,

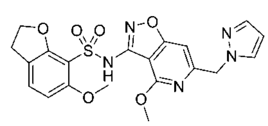, 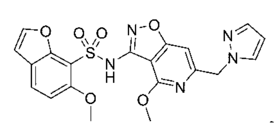, 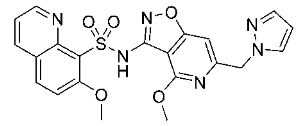, 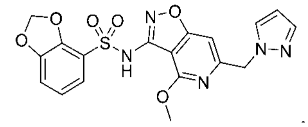,

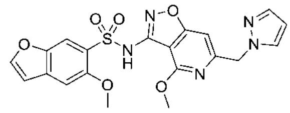, 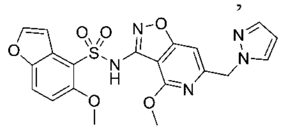, 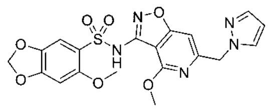, 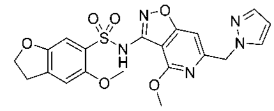,

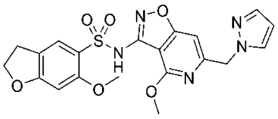, 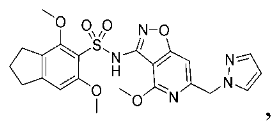, 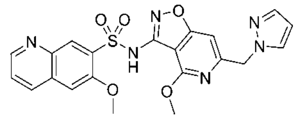, 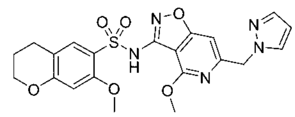,

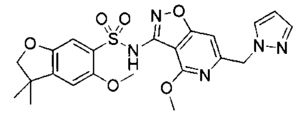, 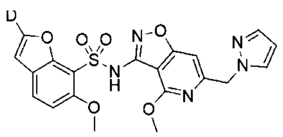, 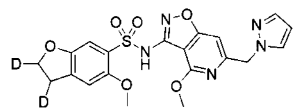, 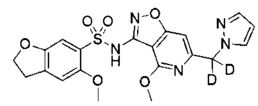,

and

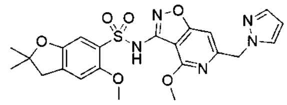.

The following compounds or stereoisomers, isotopic derivatives, hydrates, solvates, prodrugs, or pharmaceutically acceptable salts thereof are more preferred in the present disclosure:

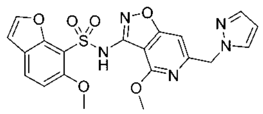 and 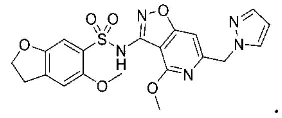.

According to an embodiment of the present disclosure, the isotopic derivative may be a deuterated substance of

the compound.

In the solutions described above, the sulfonamide compound of the present disclosure has a benzoheterocyclic structure and a pyridoisoxazole structure, wherein containing the benzoheterocyclic structure located at the side of the sulfonamido group can effectively improve the electron cloud density of the sulfonamido group moiety, enhance the ability of the two oxygen atoms in the sulfonamido group to act as hydrogen bond acceptors, and increase the binding affinity of the compound of the present disclosure for the pocket; after the phenyl ring is replaced by pyridine, the pyridoisoxazole structure effectively reduces the electron cloud density of N-O in the isoxazole, reduces the space and the degree of freedom of lone pair electrons of the oxygen, minimizes steric clashes, and makes the compound of the present disclosure bind to the pocket better. The binding of the two significantly improves the ability of the compound of the present disclosure to compete with the acetyl coenzyme for binding to KAT6A and KAT6B, which is significantly superior to those of similar compounds already reported.

In some embodiments, the sulfonamide compound of the present disclosure may also be selected from the compounds represented by formulas (I) to (IV) or the stereoisomers, the isotopic derivatives, the hydrates, the solvates, the prodrugs, or the pharmaceutically acceptable salts thereof, such as enantiomers, diastereoisomers, pharmaceutically acceptable salts, prodrugs, isotopic derivatives, and solvates thereof. It can be understood that the compounds represented by formulas (I) to (IV) of the present disclosure are used as active reaction materials, and conventional design may be performed based on these compounds, such that the designed compounds may still retain the pharmaceutical efficacy of the compounds represented by formulas (I) to (IV).

The present disclosure also provides a pharmaceutical composition comprising any one of the compounds represented by formulas (I) to (IV) or the stereoisomers, the isotopic derivatives, the hydrates, the solvates, the prodrugs, or the pharmaceutically acceptable salts thereof, such as any one of enantiomers, diastereoisomers, pharmaceutical salts, or solvates of the compounds, as an active ingredient, and a pharmaceutically acceptable carrier. The pharmaceutical composition of the present application may be used as a medicament for the treatment of a disease associated with amplification or overexpression of KAT6 (preferably KAT6A and/or KAT6B).

The present disclosure also relates to use of any one of the compounds represented by formulas (I) to (IV) or the stereoisomers, the isotopic derivatives, the hydrates, the solvates, the prodrugs, or the pharmaceutically acceptable salts thereof, such as the compounds represented by formulas (I) to (IV) or the pharmaceutically acceptable salts thereof, for manufacturing a medicament for the treatment and/or prevention of a disease associated with amplification or overexpression of KAT6.

The present disclosure also relates to a method for diagnosing, preventing, or treating a disease associated with amplification or overexpression of KAT6, which comprises administering to a patient in need thereof a therapeutically and/or prophylactically effective amount of any one of the compounds represented by formulas (I) to (IV) or the stereoisomers, the isotopic derivatives, the hydrates, the solvates, the prodrugs, or the pharmaceutically acceptable salts thereof, such as the compounds represented by formulas (I) to (IV) or the pharmaceutically acceptable salts, or the pharmaceutical composition thereof.

According to an embodiment of the present disclosure, the disease associated with amplification or overexpression of KAT6 is a cancer, including at least one of breast cancer, triple-negative breast cancer, acute myeloid leukemia, lung cancer, lung adenocarcinoma, hematological tumors, digestive system tumors, reproductive system tumors, prostate cancer, nervous system tumors, or head and neck cancer. Among them, the breast cancer is preferably ER+ breast cancer or ER+/HER2- breast cancer; the lung cancer is preferably non-small cell lung cancer; the prostate cancer is preferably castration-resistant prostate cancer.

Beneficial Effects:

Compared with the compounds in the prior art known to target KAT6, the compound of the present disclosure not only has relatively good target selectivity and stronger protein level inhibitory effect, but also has the advantages that the preferred compound of the present disclosure has an appropriate half-life, is more suitable for a clinical once-a-day administration route, and combined with relatively moderate exposure, has lower possibility of causing *in-vivo* accumulation, thereby effectively reducing the safety risk of such compounds caused by accumulation.

The present disclosure also discloses a preparation method for the sulfonyl compounds described above, which comprises the following reaction route and preparation steps:

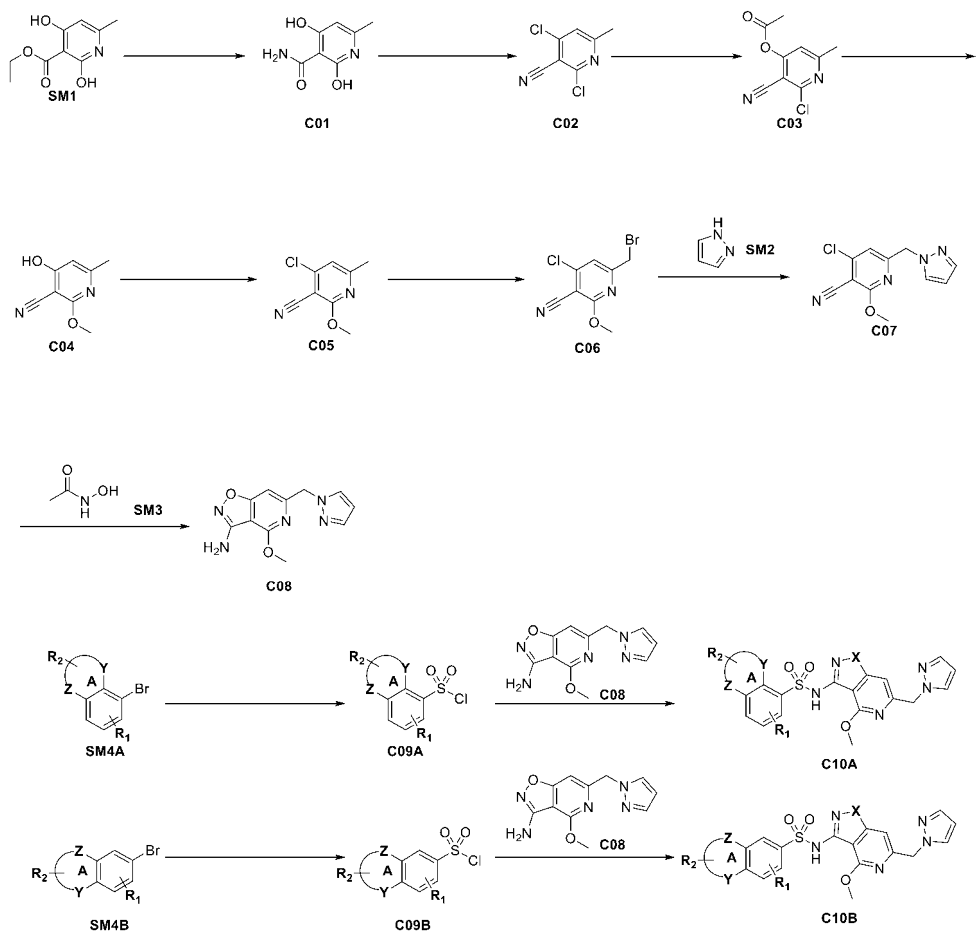


SM1 C01 C02 C03

C04 C05 C06 C07

C08

SM4A C09A C10A

SM4B C09B C10B

wherein A, Z, Y, $R_1$, $R_2$, and X are independently defined as described above; or C10B is selected from the compound represented by formula (IV) above.

Specifically, the preparation method comprises the following steps:

(1) performing an ammonolysis reaction on SM1 with aqueous ammonia at 40-80 °C to give compound C01;
(2) mixing the compound C01 with phosphorus oxychloride, and then adding N,N-dimethylformamide for reaction at 50-100 °C to give compound C02;
(3) dissolving the compound C02 in a first solvent and adding cesium acetate at 20-100 °C for reaction to give compound C03;
(4) dissolving the compound C03 in methanol, adding a first basic reagent, and reacting under the reaction condition of high temperature and high pressure to give compound C04;
(5) dissolving the compound C04, $PCl_5$, and $POCl_3$ in a second solvent, and reacting at 20-60 °C to give compound C05;
(6) dissolving the compound C05 and N-bromosuccinimide (NBS) in a third solvent, adding a free radical initiator, and reacting at 30-100 °C to give compound C06;
(7) dissolving the compound C06 and compound SM2 in a fourth solvent, adding a second basic reagent, and performing an alkylation reaction to give compound C07;
(8) dissolving the compound C07 and compound SM3 in a fifth solvent, adding a third basic reagent, and reacting at 20-60 °C to give compound C08;
(9) dissolving compound SM4A or compound SM4B in a sixth solvent, adding a fourth basic reagent at -80 °C to - 40 °C for reaction, then introducing $SO_2$ gas for reaction, and adding N-chlorosuccinimide (NCS) at -80 °C to -40 °C for reaction to give compound C09A or compound C09B, respectively; and
(10) dissolving the compound C08 and the compound C09A or the compound C09B in a seventh solvent, adding a fifth basic reagent, and reacting at a temperature ranging from 20 °C to 100 °C to give compound C10A or compound C10B.

In some embodiments, in step (3), the first solvent includes at least one selected from tetrahydrofuran,

dichloromethane, trichloromethane, dimethylsulfoxide (DMSO), dimethylformamide (DMF), and acetonitrile.

In some embodiments, in step (4), the first basic reagent includes at least one selected from sodium hydride, sodium methoxide, potassium tert-butoxide, and sodium tert-butoxide.

In some embodiments, in step (5), the second solvent includes at least one selected from tetrahydrofuran, dimethylsulfoxide (DMSO), and dimethylformamide (DMF).

In some embodiments, in step (6), the third solvent includes at least one of tetrachloromethane and benzene.

In some embodiments, in step (6), the free radical initiator includes at least one of dibenzoyl peroxide (BPO) and azobisisobutyronitrile (AIBN).

In some embodiments, in step (7), the fourth solvent includes at least one of tetrahydrofuran, dimethylsulfoxide (DMSO), dimethylformamide (DMF), dichloromethane, trichloromethane, N-methylpyrrolidone (NMP), and acetonitrile.

In some embodiments, in step (7), the second basic reagent includes at least one of cesium carbonate, potassium carbonate, sodium carbonate, potassium tert-butoxide, sodium tert-butoxide, triethylamine, pyridine, sodium hydride, and N,N-diisopropylethylamine.

In some embodiments, in step (8), the fifth solvent includes at least one of methanol, ethanol, tert-butanol, isopropanol, and water.

In some embodiments, in step (8), the third basic reagent includes at least one of cesium carbonate, potassium carbonate, sodium carbonate, potassium tert-butoxide, and sodium tert-butoxide.

In some embodiments, in step (9), the sixth solvent includes at least one of tetrahydrofuran, dichloromethane, and trichloromethane.

In some embodiments, in step (9), the fourth basic reagent includes at least one of n-butyllithium, lithium diisopropylamide (LDA), lithium bis(trimethylsilyl)amide (LiHMDS), sodium bis(trimethylsilyl)amide (NaHMDS), and potassium bis(trimethylsilyl)amide (KHMDS).

In some embodiments, in step (10), the seventh solvent includes at least one of tetrahydrofuran, dichloromethane, trichloromethane, N-methylpyrrolidone (NMP), dimethylsulfoxide (DMSO), dimethylformamide (DMF), and acetonitrile.

In some embodiments, in step (10), the fifth basic reagent includes at least one of sodium hydride, lithium diisopropylamide (LDA), lithium bis(trimethylsilyl)amide (LiHMDS), sodium bis(trimethylsilyl)amide (NaHMDS), potassium bis(trimethylsilyl)amide (KHMDS), cesium carbonate, potassium carbonate, sodium carbonate, potassium tert-butoxide, sodium tert-butoxide, triethylamine, and pyridine.

In some embodiments, the sulfonyl compound C10A is one of the compounds represented by formula I, and the sulfonyl compound C10B is one of the compounds represented by formula II.

In some embodiments, the compound C09A may be prepared from the compound SM4A, and the compound C09B may be prepared from the compound SM4B.

In other embodiments, the compound C09 may also be purchased directly, and correspondingly, the compound C10 may be prepared according to the following synthesis method:

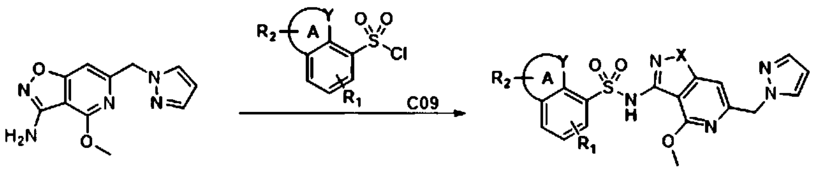


C08 C10

wherein A, Y, $R_1$, $R_2$, and X are independently defined as described above;
or C10 is selected from the compound represented by formula (IV) above.

Definitions

Unless otherwise defined, terms used herein have the same meaning as commonly understood by those of ordinary skill in the art to which the present disclosure pertains The terms used in this specification are used for the purpose of describing particular embodiments only and are not intended to be limiting of the present disclosure.

The compounds defined or disclosed in the present disclosure include all stereoisomers, optical isomers, and racemates thereof (if such isomers exist). Unless otherwise indicated, all chiral (enantiomeric and diastereoisomeric) and racemic forms are within the scope of the present disclosure. The compounds of the present disclosure may be separated in an optically active form or in a racemic form. The optically active form may be prepared by resolution of the racemic form or by synthesis from optically active starting materials. All methods for preparing the compounds of the present disclosure and intermediates prepared therein are considered to be part of the present disclosure. When enantiomeric or diastereoisomeric products are prepared, they may be separated by conventional methods, for example, by chromatography or fractional crystallization.

The compounds defined or disclosed in the present disclosure may also exist in a variety of geometric isomers of C=C double bonds, C=N double bonds, ring systems, and the like, and all such stable isomers are included in the present disclosure. Cis and trans (or E and Z) geometric isomers of the compounds of the present disclosure are described and may be separated into mixtures of isomers or separated isomeric forms.

The compounds defined or disclosed in the present disclosure may exist in a variety of tautomeric forms, wherein the hydrogen atom is transferred to other parts of a molecule and chemical bonds between atoms in the molecule are thereby rearranged. It should be understood that all tautomeric forms are included in the present disclosure as long as they can exist.

The compounds defined or disclosed in the present disclosure may exist in a free form or in the form of a pharmaceutically acceptable salt, and thus both free forms and salts are within the scope of the present disclosure. One form of the compound may be converted into another form as desired. A free base or acid may be converted into a salt; the salt may be converted into a free compound or another salt.

Isotopic derivatives described herein are compounds that can contain one or more isotopic substitutions or isotopically labeled compounds. For example, H may be in any isotopic form, including $^1H$, $^2H$ (D or deuterium), and $^3H$ (T or tritium); C may be in any isotopic form, including $^{12}C$, $^{13}C$, and $^{14}C$; O may be in any isotopic form, including $^{16}O$ and $^{18}O$, and the like.

The term "alkyl" should refer to, within its context, linear, branched, or cyclic, fully saturated alkyl that may optionally be substituted, preferably C1-C6 alkyl, and more preferably C1-C4 or C1-C3 alkyl. Examples of alkyl are methyl, ethyl, n-butyl, sec-butyl, n-hexyl, n-heptyl, n-octyl, n-nonyl, n-decyl, isopropyl, 2-methylpropyl, and the like. "Alkyl" in the terms "alkoxy", "alkylthio", "haloalkyl", and the like also has the same meaning.

The term "alkynyl" refers to linear or branched C2-C6 alkynyl containing at least one C≡C bond, preferably C2-C4 alkynyl.

The term "cycloalkyl" should refer to, within its context, C3-C10 monocyclic or polycyclic alkyl, preferably C3-C6 cycloalkyl, and more preferably C3-C4 cycloalkyl. Examples of cycloalkyl are cyclopropyl, cyclobutyl, cyclopentyl, cyclohexyl, or the like.

The term "heterocycloalkyl" or "heterocyclyl" should refer to, within its context, a 3- to 10-membered cyclic group containing at least one heteroatom (e.g., N, O, or S), preferably a 3- to 6-membered cyclic group. It may be, for example, a 3-, 4-, 5-, or 6-membered cyclic group, and it may be aromatic (heteroaryl) or non-aromatic. The heterocycloalkyl or heterocyclyl includes: azetidinyl, oxetanyl, 1,3-dioxolane, 1,3-dioxane, 1,4-dioxane, tetrahydrofuranyl, tetrahydropyranyl, pyranyl, furanyl, morpholinyl, pyrrolyl, piperidyl, imidazolyl, pyridinyl, and pyrimidinyl.

The term "aryl" should refer to, within its context, a substituted or unsubstituted C6-C10 aromatic group having a single ring or fused rings, including phenyl, naphthyl, and the like.

The term "heteroaryl" should refer to, within its context, a 5- to 10-membered aromatic group containing at least one heteroatom (e.g., N, O, or S), preferably a 5- to 6-membered aromatic group. It may be, for example, a 3-, 4-, 5-, or 6-membered aromatic group, including: optionally substituted imidazole, optionally substituted isoxazole, optionally substituted oxazole, optionally substituted diazole (pyrazolyl or imidazolyl), optionally substituted triazole (1,2,3-triazolyl or 1,2,4-triazolyl), optionally substituted thiophene, optionally substituted isothiazole, and optionally substituted pyridine (2-pyridine, 3-pyridine, or 4-pyridine).

The term "halogen" refers to F, Cl, Br, or I.

The term "substituted" or "optionally substituted" means that one or more substituents, preferably 1-5 substituents, more preferably 1-3 substituents, and most preferably 1-2 substituents, independently exist at any carbon (or nitrogen) position in a molecule. Each substituent is identical or different, and may be independently selected from: hydroxyl, thiol, carboxyl, cyano, nitro, halogen, C1-C6 alkyl, C1-C6 haloalkyl, C1-C6 alkoxy, C6-C10 aryl, 5- to 10-membered heteroaryl, C1-C6 thioether, C1-C6 acyl, C1-C6 alkylamine, C1-C6 dialkylamine, carboxyl, and the like.

The substitution position designation for each substituent is understood in accordance with the nomenclature of conventional compounds in the art. For example, the substitution position on a heterocyclic or heteroaromatic ring is understood in accordance with the conventional nomenclature of aryl in heterocyclic or heteroaromatic rings. For example, the substitution positions of benzofuran or 2,3-dihydrobenzofuran are numbered as follows:

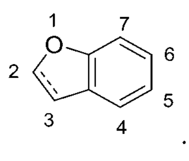

As used herein,

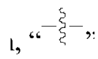

represents a chemical bond.

The term "pharmaceutically acceptable salt" is used to describe a salt form of one or more of the compounds described herein, which is provided to increase the solubility of the compound in the gastric juice of the gastrointestinal tract of a patient, thereby facilitating dissolution and bioavailability of the compound. Pharmaceutically acceptable salts, where applicable, include salts derived from pharmaceutically acceptable inorganic or organic bases and acids. The term "effective" may mean, but is not limited to, an amount/dose of an active pharmaceutical ingredient that, when used in the context of its intended use, achieves or is sufficient to prevent a disease symptom, a condition, or a disease state, inhibit the onset of the disease symptom, the condition, or the disease state, or ameliorate, delay, or treat the symptom (to some extent, preferably to completely alleviate the symptom) in a subject in need of or receiving such treatment.

The term "pharmaceutically acceptable carrier" may mean any and all solvents, dispersion media, coatings, or the like, compatible with the administration of drugs.

The examples of the present disclosure are further illustrated in the following examples. Among them, the examples of the present disclosure are not limited to the following specific examples. The present disclosure can be modified and implemented as appropriate within the scope of the main claims.

**Example 1**

Sulfonyl compound 1-1:

*N*-(6-((1H-pyrazol-1-yl)methyl)-4-methoxyisoxazolo[4,5-c]pyridin-3-yl)-2,3-dihydrobenzofuran-7-sulfonamide;

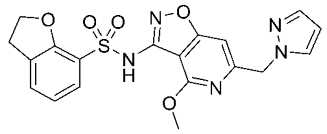

**1-1**

Synthetic route:

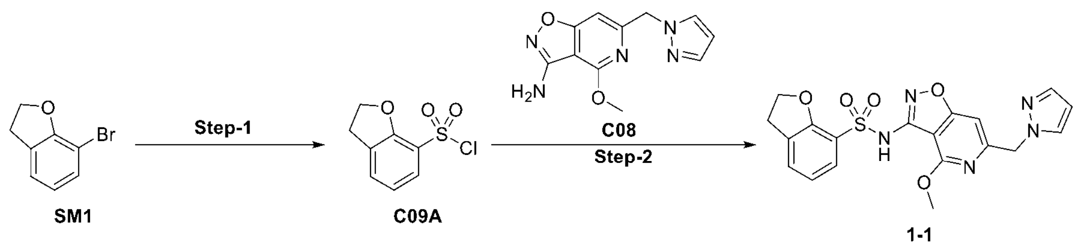


**SM1** **C09A** **C08** **1-1**

Step 1: The preparation of intermediate C08 comprises the following reaction route and preparation steps:

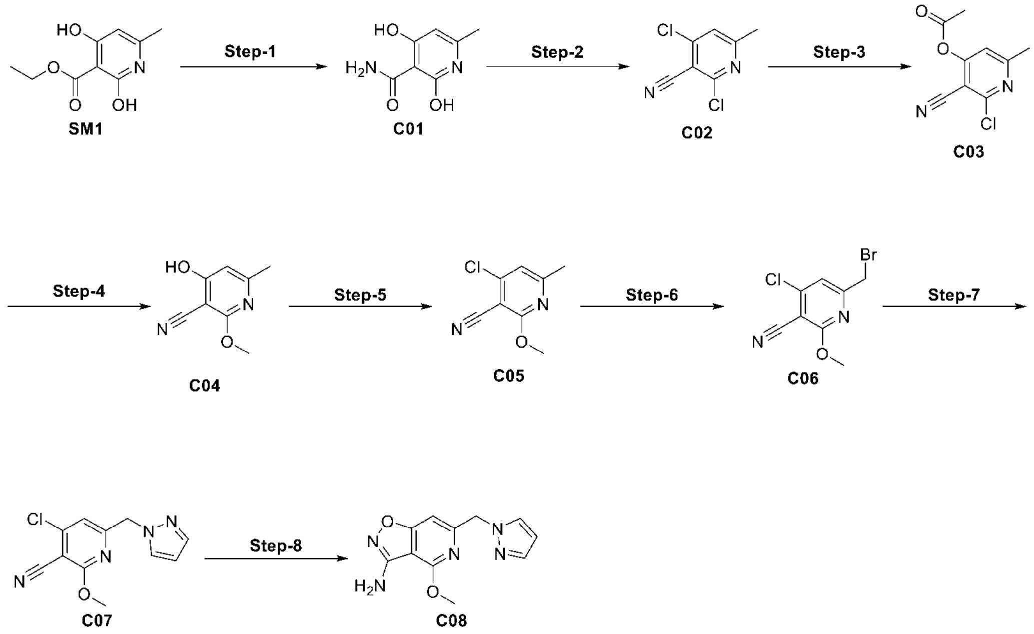


SM1 C01 C02 C03 C04 C05 C06 C07 C08

Step-1: Preparation of intermediate C01: Compound SM1 (86.0 g, 1 eq) was added to aqueous ammonia (100 mL), and the reaction system was heated to 50 °C under nitrogen atmosphere and stirred for 16 h. A solid was precipitated, and liquid chromatography-mass spectrometry (LCMS) showed that the reaction was completed. The mixture was filtered, and the filter cake was washed with water. The filtrate was concentrated under reduced pressure to one third, and then the precipitated solid was further filtered, combined, and dried to give a crude product of intermediate C01 (74.1 g, yield: 96.2%), which was directly used in the next step without treatment.

Step-2: Preparation of intermediate C02: Intermediate C01 (74.1 g, 1 eq) was mixed with $POCl_3$ (800 mL). Under nitrogen atmosphere, the mixture was heated to 70 °C and stirred for 30 min. Then, DMF (4.0 mL, 0.12 eq) was added when the mixture was heated to 90 °C, and the reaction mixture was stirred for 2 h. LCMS showed that the reaction was completed. Crushed ice was added to quench the reaction, and the mixture was adjusted to be alkaline with a saturated sodium carbonate solution. At this time, a solid was precipitated, and the mixture was filtered. The filter cake was collected and washed three times with a small amount of water, and the solid was dried. The mother liquor was extracted with ethyl acetate and washed with a saturated aqueous sodium chloride solution. The organic phase was dried over anhydrous sodium sulfate, subjected to suction filtration, and concentrated by rotary evaporation. All the resulting solids were combined to give a crude product of intermediate C02 (64.1 g, yield: 96.2%), which was directly used in the next step without treatment.

Step-3: Preparation of intermediate C03: Intermediate C02 (64.1 g, 1 eq) and CsOAc (192.8 g, 3 eq) were dissolved in DMF (800 mL). The mixture was heated to 70 °C under nitrogen atmosphere and stirred for 16 h. LCMS showed that the reaction was completed. The reaction system was added to an ice water mixture to quench the reaction, and the mixture was extracted with ethyl acetate and washed with a saturated aqueous sodium chloride solution. The organic phase was dried over anhydrous sodium sulfate, subjected to suction filtration, and concentrated by rotary evaporation. The resulting crude product was slurried with a mixed solution of ethyl acetate and n-hexane. The mixture was filtered, and the resulting filter cake was dried to give a crude product of intermediate C03 (58.1 g, yield: 80.5%), which was directly used in the next step without further treatment.

Step-4: Preparation of intermediate C04: C03 (58.0 g, 1 eq), sodium methoxide (149.1 g, 10 eq), and methanol (2 L) were added to a 5 L high-pressure reaction kettle. After nitrogen purging, the mixture was heated to 150 °C and reacted for 16 h under a pressure not exceeding 10 standard atmospheres. The mixture was cooled, and the reaction was monitored. LCMS showed that the reaction was completed, and the reaction system was cooled to room temperature, added to a small amount of water, and concentrated by rotary evaporation under reduced pressure to remove most of methanol. Then, the aqueous phase was adjusted to be acidic with hydrochloric acid, and a solid was precipitated. The mixture was filtered, and the filter cake was collected and washed with a small amount of water. The solid was dried. The mother liquor was extracted with ethyl acetate and washed with a saturated aqueous sodium chloride solution. The organic phase was dried over anhydrous sodium sulfate, subjected to suction filtration, and concentrated by rotary evaporation. The two obtained solids were combined to give a crude product of intermediate C04 (14.9 g, yield: 80.5%), which was directly used in the next step without further treatment.

Step-5: Preparation of intermediate C05: DMF (6.7 g, 1 eq) was slowly added dropwise to a mixture containing

intermediate C04 (14.9 g, 1 eq), $POCl_3$ (13.8 g, 1 eq), $PCl_5$ (18.7 g, 1 eq), and DCM (1.2 L) at room temperature. Under nitrogen atmosphere, the mixture was heated to 40 °C and stirred for 30 min. LCMS showed that there were about 50% products, some starting materials remaining, and some dichloro-substituted by-products. After cooling to room temperature, the reaction system was added to an ice water mixture to quench the reaction, and the mixture was adjusted to be alkaline with a saturated sodium carbonate solution. A solid was precipitated. The mixture was filtered, and the filter cake was collected and washed with a small amount of water. The solid was dried. The mother liquor was extracted with ethyl acetate and washed with a saturated aqueous sodium chloride solution. The organic phase was dried over anhydrous sodium sulfate, subjected to suction filtration, and concentrated by rotary evaporation. The two obtained solids were combined and purified by normal phase column chromatography to give intermediate C05 (5.21 g, yield: 31.5%).

Step-6: Preparation of intermediate C06: Intermediate C05 (5.21 g, 1 eq), N-bromosuccinimide (NBS, 5.12 g, 1 eq), and dibenzoyl peroxide (BPO, 1.38 g, 0.2 eq) were dissolved in tetrachloromethane (400 mL). Under $N_2$ atmosphere, the mixture was heated to 90 °C and stirred for 3 h. LCMS showed that there were 57% products, 20% by-products, and 14% starting materials remaining. The reaction was stopped, and the mixture was cooled to room temperature. A saturated sodium bisulfite solution was added to quench the reaction, and the mixture was extracted 3 times with a large amount of ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give intermediate C06 (2.83 g, yield: 37.9%).

Step-7: Preparation of intermediate C07: Intermediate C06 (2.83 g, 1 eq), pyrazole (1.11 g, 1.5 eq), and DIEA (2.79 g, 2 eq) were dissolved in acetonitrile. Under $N_2$ atmosphere, the mixed solution was heated to 90 °C, refluxed, and stirred overnight. LCMS showed that the reaction was completed. The reaction was stopped, and the mixture was cooled to room temperature, concentrated by rotary evaporation, and purified by normal phase column chromatography to give intermediate C07 (1.76 g, yield: 65.6%).

Step-8: Preparation of key intermediate C08: Intermediate C07 (1.76 g, 1 eq) was dissolved in tert-butanol (200 mL), and acetohydroxamic acid (1.07 g, 2 eq) and potassium carbonate (1.85 g, 2 eq) were added to the solution. Under $N_2$ atmosphere, the mixed solution was slowly heated to 30 °C and stirred overnight. LCMS showed that the reaction was completed. The reaction was stopped, and the mixture was cooled to room temperature. Ethyl acetate was added for dilution, and the carbonate was filtered off. The filter cake was washed 3 times with ethyl acetate. The filtrates were combined and concentrated by rotary evaporation. The resulting residue was purified by normal phase column chromatography to give key intermediate C08 (1.22 g, yield: 69.5%) as a light yellow solid. MS: $[M+1]^+$: 246.1.

$^1H$ NMR (400 MHz, DMSO- $d_6$) δ 7.90 (d, $J$ = 2.4 Hz, 1H), 7.52 (d, $J$ = 1.6 Hz, 1H), 6.54 (s, 1H), 6.33(t, $J$ = 2.0 Hz, 1H), 6.21 (s, 2H), 5.42 (s, 2H), 3.98 (s, 3H).

Step II: Preparation of 2,3-dihydrobenzofuran-7-sulfonyl chloride (C09A):

Compound SM1 (800 mg, 1 eq) was dissolved in freshly distilled tetrahydrofuran (20 mL). After the compound was completely dissolved, n-butyllithium (2.5 M in n-hexane, 1.9 mL, 1.2 eq) was slowly added dropwise at -78 °C. The mixture was reacted at -78 °C for 10 min. $SO_2$ gas was introduced at -78 °C for 10 min. After the mixture was naturally warmed to room temperature, N-chlorosuccinimide (NCS, 534 mg, 1 eq) was added. The mixture was reacted at room temperature for 1 h. The reaction system was added to a saturated ammonium chloride solution to quench the reaction, and the mixture was extracted 3 times with ethyl acetate. The organic phases were combined, washed with a saturated aqueous sodium chloride solution, dried over anhydrous sodium sulfate, subjected to suction filtration, and concentrated by rotary evaporation. The resulting solid was purified by normal phase column chromatography to give intermediate C09A (397.2 mg, yield: 45.6%).

$^1H$ NMR (400 MHz, $CDCl_3$-$d$) δ (ppm): 7.68 (d, $J$ = 8.4 Hz, 1H), 7.51 (d, $J$ = 8.4 Hz, 1H), 6.89 (t, $J$ = 8.4 Hz, 1H), 4.91-4.85 (m, 2H), 3.42-3.31 (m, 2H).

Step III: Preparation of sulfonyl compound 1-1:

Intermediate C08 (25.1 mg, 1 eq) was dissolved in freshly distilled tetrahydrofuran (3 mL). After the compound was completely dissolved, sodium hydride (7.3 mg, 3 eq) was added at 0 °C. Under $N_2$ atmosphere, the mixture was stirred at 0 °C for 20 min, and then 2,3-dihydrobenzofuran-7-sulfonyl chloride (C09A, 44.6 mg, 2 eq) was added to the reaction system. The reaction system was stirred at room temperature overnight, and LCMS showed that the reaction was completed. The reaction system was added to a saturated ammonium chloride solution to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by high-pressure preparative liquid chromatograph to give sulfonyl compound 1-1 (10.6 mg, yield: 24.2%) as a white solid. MS: $[M+1]^+$: 427.95.

Sulfonyl compound 1-1: $^1H$ NMR (400 MHz, DMSO-$d_6$) δ (ppm): 11.05 (s, 1H), 7.89(s, 1H), 7.78 (s, 1H), 7.71 (d, $J$

= 8.4 Hz, 1H), 7.52 (s, 1H), 6.96-6.86 (m, 1H), 6.62 (s, 1H), 6.33 (s, 1H), 5.43 (s, 2H), 4.61 (t, *J* = 8.4 Hz, 2H), 3.98 (s, 3H), 3.23-3.11 (m, 2H).

II. A preparation method for a sulfonyl compound was different from Example 1 in that the following sulfonyl chloride compounds (C09) D-1 to D-13 were prepared according to a preparation method similar to Step-1 in Example 1, as shown in Table 1 below.

Table 1. Sulfonyl chloride compounds (C09) D-1 to D-13 prepared in examples

| No. | Structure/Sulfonyl chloride compound number | Analysis data |
|---|---|---|
| **D-1** | 3,4-Dihydro-1H-benzopyran-8-sulfonyl chloride<br>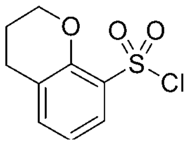 | $^1$H NMR (400 MHz, $CDCl_3$-*d*) δ (ppm): 7.77 (d, *J* = 8.4 Hz, 1H), 7.39 (d, *J* = 8.4 Hz, 1H), 6.96 (t, *J* = 8.4 Hz, 1H), 4.47 (t, *J* = 4.4 Hz, 2H), 2.90 (t, *J* = 4.4 Hz, 2H), 2.17-2.11 (m, 2H). |
| **D-2** | 2,3-Dihydrobenzo[b][1,4]dioxane-5-sulfonyl chloride<br>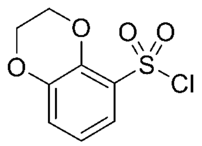 | $^1$H NMR (400 MHz, $CDCl_3$-*d*) δ (ppm): 7.51 (d, *J* = 8.4 Hz, 1H), 7.24 (d, *J* = 8.4 Hz, 1H), 6.91 (t, *J* = 8.4 Hz, 1H), 4.51-4.47 (M, 2H), 4.37-4.31 (m, 2H). |
| **D-3** | 5,6,7,8-Tetrahydronaphthalene-1-sulfonyl chloride<br>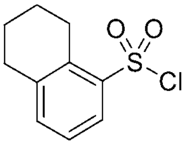 | H NMR (400 MHz, $CDCl_3$-*d*) δ (ppm): 7.76 (d, *J* = 8.4 Hz, 1H), 7.39 (d, *J* = 8.4 Hz, 1H), 7.05 (t, *J*= 8.4 Hz, 1H), 2.99-2.89 (m, 2H), 2.81-2.75 (m, 2H), 1.88-1.81 (m, 4H). |
| **D-4** | 2,3-Benzofuran-4-sulfonyl chloride<br>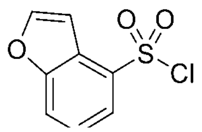 | $^1$H NMR (400 MHz, $CDCl_3$-*d* δ (ppm): 8.01 (s, 1H), 7.79 (d, *J* = 8.4 Hz, 1H),7.51 (d, *J* = 8.4 Hz, 1H), 7.01 (t, *J* = 8.4 Hz, 1H), 6.84 (s, 1H). |
| **D-5** | 1-Methyl-1H-indole-4-sulfonyl chloride<br>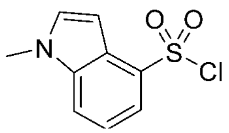 | $^1$H NMR (400 MHz, $CDCl_3$-*d*) δ (ppm): 7.98 (s, 1H), 7.87(d, *J* = 8.4 Hz, 1H), 7.38-7.31 (m, 2H), 7.05(s, 1H), 3.94 (s, 3H). |
| **D-6** | 2-Methylbenzo[d]oxazole-7-sulfonyl chloride<br>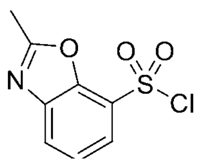 | $^1$H NMR (400 MHz, $CDCl_3$-*d*) δ (ppm): 7.77 (d,*J* = 8.4 Hz, 1H), 7.46 (d, *J* = 8.4 Hz, 1H), 7.24 (t, *J* = 8.4 Hz, 1H), 2.69 (s, 3H). |

(continued)

| No. | Structure/Sulfonyl chloride compound number | Analysis data |
|---|---|---|
| **D-7** | 1,2-Methylenedioxybenzene-4-sulfonyl chloride<br>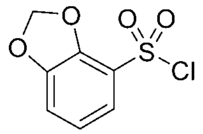 | $^1$H NMR (400 MHz, $CDCl_3$-*d*) δ (ppm): 7.36 (d, *J* = 8.4 Hz, 1H), 7.14 (d, *J* = 8.4 Hz, 1H), 7.01 (t, *J* = 8.4 Hz, 1H), 6.27 (s, 2H). |
| **D-8** | 2,2-Difluoro-1,3-benzodioxole-4-sulfonyl chloride<br>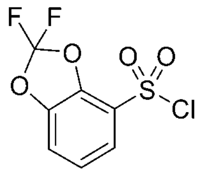 | $^1$H NMR (400 MHz, $CDCl_3$-*d*) δ (ppm): 7.67 (d, *J* = 8.4 Hz, 1H), 7.48 (d, *J* = 8.4 Hz, 1H), 7.36 (t, *J* = 8.4 Hz, 1H). |
| **D-9** | 1-Methyl-1H-indole-7-sulfonyl chloride<br>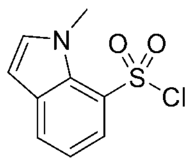 | $^1$H NMR (400 MHz, $CDCl_3$-*d*) δ (ppm): 8.04-7.88 (m, 2H), 7.22-7.18 (m, 2H), 6.71 (s, 1H), 4.33 (s, 3H). |
| **D-10** | 3,4-Dihydro-1H-benzopyran-6-sulfonyl chloride<br>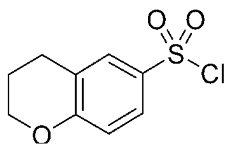 | $^1$H NMR (400 MHz, $CDCl_3$-*d*) δ (ppm): 7.77-7.71 (m, 2H), 6.94 (d, *J* = 8.0 Hz, 1H), 4.35-4.29 m, 2H), 2.91-2.87 (m, 2H), 2.12-2.05(m, 2H) |
| **D-11** | 4-Ethynyl-2,3-dihydrobenzofuran-7-sulfonyl chloride<br>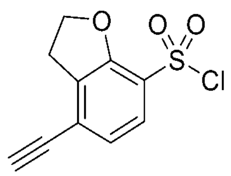 | $^1$H NMR (400 MHz, $CDCl_3$-*d*) δ (ppm):7.37 (d, *J* = 8.4 Hz, 1H), 6.98 (d, *J* = 8.4 Hz, 1H), 4.31-4.25 (m, 2H), 3.23(s, 1H), 3.12-3.01 (m, 2H). |
| **D-12** | Benzofuran-5-sulfonyl chloride<br>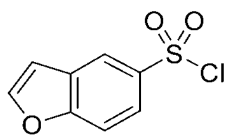 | H NMR (400 MHz, $CDCl_3$-*d*) δ (ppm): 8.38-8.31 (m, 1H), 8.08-7.98 (m, 1H), 7.84 (s, 1H),7.74-7.69 (m, 1H), 7.01 (s, 1H). |
| **D-13** | 2-Methylbenzoxazole-5-sulfonyl chloride<br>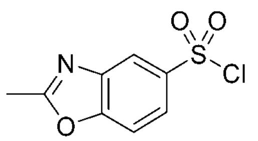 | $^1$H NMR (400 MHz, $CDCl_3$-*d*) δ (ppm): 7.88 (d, *J* = 8.8 Hz, 1H), 7.14 (s, 1H), 6.89-6.87 (m 1H), 2.61 (s, 3H). |

The sulfonyl chloride compound C09A in Example 1 was replaced by the compounds D-1 and D-13 described above to give thirteen novel sulfonamide compounds.

**III.** A preparation method for a sulfonyl compound was different from Example 1 in that the sulfonyl chloride compound C09A was replaced by structures represented by D-14A and D-14B:

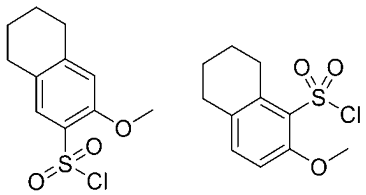

**D-14A** **D-14B**

Step I: The synthetic route of the preparation of the sulfonyl chloride compounds D-14A and D-14B was as follows:

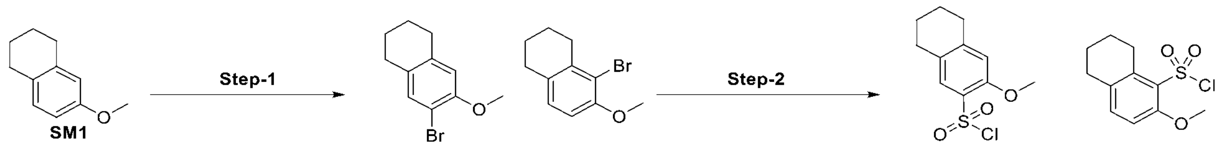


Step-1: Preparation of compound D-14A-1 and compound D-14B-1:

Under nitrogen atmosphere, compound SM1 (19.7 g, 1 eq) was dissolved in a mixed solvent of dichloromethane (500 mL) and methanol (300 mL). A solution of tetrabutylammonium tribromide (78.1 g, 1.5 eq) in dichloromethane (500 mL) was added dropwise at 0 °C, and the mixture was warmed to room temperature and stirred for 1.5 h. GCMS showed that the reaction was completed. The reaction system was added to 1 L of water to quench the reaction, and the mixture was extracted 3 times with ethyl acetate and washed with a saturated aqueous sodium chloride solution. The organic phase was dried over anhydrous sodium sulfate, subjected to suction filtration, and concentrated by rotary evaporation. The crude product was purified by normal phase liquid chromatography to give a mixture of D-14A-1 and D-14B-1 (16.7 g, yield: 57.1%). GCMS (ES, m/z): 240/242 [M].

Step-2: Preparation of compound D-14A and compound D-14B:

A mixture of D-14A-1 and D-14B-1 (2.0 g, 1 eq) was dissolved in freshly distilled tetrahydrofuran (20 mL). Under $N_2$ atmosphere, n-butyllithium (2.5 M in hexanes, 4.0 mL, 1.2 eq) was added dropwise under stirring at -78 °C, and the temperature was maintained for 10 min for reaction. $SO_2$ gas was introduced at -78 °C for 10 min. After the mixture was slowly warmed to room temperature, NCS (1.1 g, 1 eq) was added, and the mixture was reacted at room temperature for 1 h. Water was added to the reaction system to quench the reaction, and the reaction system was extracted three times with ethyl acetate. The organic phases were combined, dried over anhydrous sodium sulfate, filtered, and concentrated by rotary evaporation. The crude product was purified by normal phase chromatographic column to give a mixture of D-14A and D-14B (0.85 g, yield: 39.2%, D-14A:D-14B = 2:1 in $^1$H NMR).

Sulfonyl chloride compound D-14A: $^1$H NMR (400 MHz, $CDCl_3$-*d*) δ (ppm): 7.66 (s, 1H), 6.80 (s, 1H), 4.02 (s, 3H), 2.85- 2.77 (m, 4H), 1.84-1.81 (m, 4H).

Sulfonyl chloride compound D-14B: $^1$H NMR (400 MHz, $CDCl_3$-*d*) δ (ppm): 7.36 (d, *J* = 8.0 Hz, 1H), 6.93 (d, *J* = 8.4 Hz, 1H), 4.02 (s, 3H), 3.27-3.25 (m, 2), 2.81-2.79 (m, 2H), 1.80-1.77 (m, 4H).

**IV.** A preparation method for a sulfonyl compound was different from Example 1 in that C09A was replaced by a structure represented by D-15:

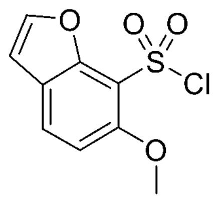

D-15

Step I: The synthetic route of the preparation of the sulfonyl chloride compound D-15 was as follows:

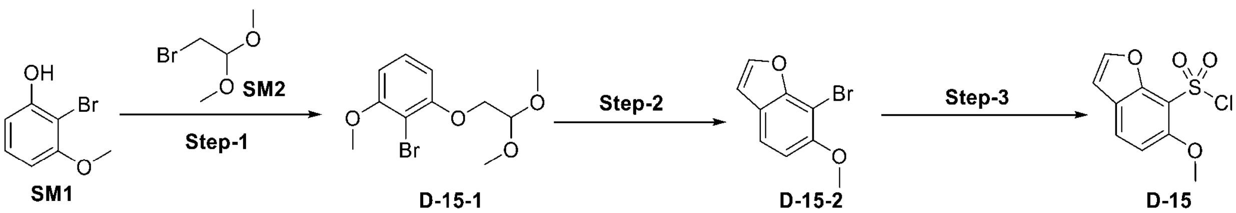


Step-1: Preparation of compound D-15-1:

Under nitrogen atmosphere, SM1 (5.1 g, 1 eq) was added to a 250 mL three-necked flask, andN,N-diethylformamide (50 mL) was added to the three-necked flask for complete dissolution. After the mixture was completely dissolved, SM2 (5.7 g, 1.5 eq), potassium iodide (0.41 g, 0.1 eq), and potassium carbonate (8.5 g, 2.5 eq) were sequentially added to the system. Under nitrogen atmosphere, the mixture was reacted at 120 °C overnight. The reaction mixture was quenched with water and extracted three times with ethyl acetate. The organic phases were combined and washed 3 times with a saturated sodium chloride solution. The resulting residue was concentrated under reduced pressure, and the crude product was purified by normal phase column chromatography to give D-15-1 (6.1 g, yield: 85.1%) as a white solid.

Step-2: Preparation of compound D-15-2:

D-15-1 (6.1 g, 1 eq) was dissolved in toluene (60 mL), and polyphosphoric acid (4.11 g, 2 eq) was added to the system. Under nitrogen atmosphere, the mixture was heated to 120 °C and reacted overnight. After the reaction was completed, the reaction mixture was quenched with crushed ice and extracted three times with ethyl acetate. The organic phases were combined and washed 3 times with a saturated sodium chloride solution. The resulting residue was concentrated under reduced pressure, and the crude product was purified by normal phase column chromatography to give D-15-2 (4.1 g, yield: 84.1%) as a white solid.

Step-3: Preparation of compound D-15:

Under nitrogen atmosphere, D-15-2 (800 mg, 1 eq) was dissolved in freshly distilled tetrahydrofuran (20.0 mL), and n-butyllithium (2.5 M in n-hexane, 0.34 mL, 1 eq) was slowly added dropwise at -78 °C for reaction for 10 min. Subsequently, sulfur dioxide gas was introduced for 10 min. The mixture was slowly warmed to room temperature, and NCS (470 mg, 1 eq) was added. The mixture was reacted at room temperature for 1 h. The reaction system was poured into a saturated aqueous ammonium chloride solution to quench the reaction, and the mixture was extracted three times with ethyl acetate. The organic phases were combined, dried over anhydrous sodium sulfate, filtered, and concentrated by rotary evaporation. The crude product was purified by normal phase chromatographic column to give sulfonyl compound D-15 (300 mg, yield: 34.5%) as a light yellow solid.

Sulfonyl compound D-15: $^1$H NMR (400 MHz, $CDCl_3$-*d*) δ (ppm): 7.87 (s, 1H), 7.56 (d, *J* = 8.4 Hz, 1H), 6.99 (d, *J* = 8.4 Hz, 1H), 6.84 (s, 1H), 3.80 (s, 3H).

**V.** A preparation method for a sulfonyl compound was different from Example 1 in that C09A was replaced by a structure represented by D-16:

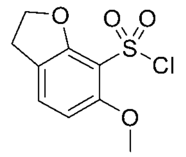

**D-16**

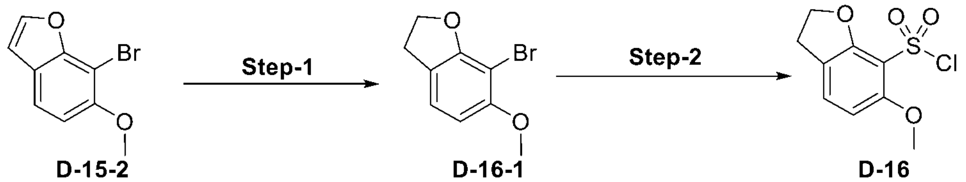


**D-15-2** **D-16-1** **D-16**

Step-1: Preparation of compound D-16-1: D-15-2 (3.1 g, 1 eq) was dissolved in ethanol, and rhodium on carbon (100 mg) was added. The mixture was reacted at room temperature for 4 h under hydrogen atmosphere (1 atm). GCMS showed that the reaction was completed. The mixture was pressurized and filtered, and the filter cake was washed with ethanol and concentrated by rotary evaporation to give a crude product of D-16-1 (2.6 g). The crude product was directly used in the next step without further purification.

Step-2: Preparation of compound D-16: The sulfonyl chloride compound D-16 was prepared according to the procedure of Step-3 for compound D-15.

Sulfonyl chloride compound D-16: $^1$H NMR (400 MHz, $CDCl_3$-*d*) δ (ppm): 7.38 (d, *J* = 8.4 Hz, 1H), 6.49 (d, *J* = 8.4 Hz, 1H), 4.83 (t, *J* = 8.4 Hz, 2H), 3.99 (s, 3H), 3.22 (t, *J* = 8.4 Hz, 2H).

**VI.** A preparation method for a sulfonyl chloride compound was different from Example 1 in that the following sulfonyl chloride compound D-17 (5-methoxy-2,3-benzofuran-4-sulfonyl chloride) was prepared according to a preparation method similar to that for D-15, and the sulfonyl chloride compound D-17 has a structure shown as follows:

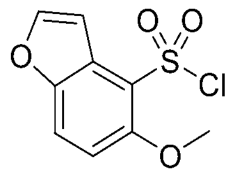
(D-17)

Sulfonyl chloride compound D-17: $^1$H NMR (400 MHz, $CDCl_3$-*d*) δ (ppm): 8.01 (s, 1H), 7.66 (d, *J* = 8.4 Hz, 1H), 7.01 (d, *J* = 8.4 Hz, 1H), 6.88 (s, 1H), 3.84 (s, 3H).

**VII.** A preparation method for a sulfonyl chloride compound was different from Example 1 in that the following sulfonyl chloride compound D-18 (5-methoxy-2,3-benzofuran-6-sulfonyl chloride) was prepared according to a preparation method similar to that for D-15, and the sulfonyl chloride compound D-18 has a structure shown as follows:

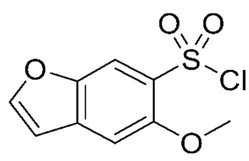
(D-18)

Sulfonyl chloride compound D-18: $^1$H NMR (400 MHz, $CDCl_3$-*d*) δ (ppm): 7.89 (s, 1H), 7.69 (d, *J* = 8.4 Hz, 1H), 6.88 (d, *J* = 8.4 Hz, 1H), 6.71 (s, 1H), 3.80 (s, 3H).

**VIII.** A preparation method for a sulfonyl compound was different from Example 1 in that C09A was replaced by a structure represented by D-19:

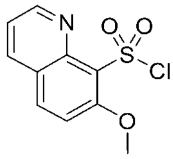

D-19

The synthetic route of D-19 is as follows:

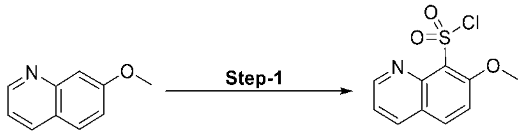


Step-1: Preparation of compound D-19: Under nitrogen atmosphere, a mixture of SM1 (200 mg, 1 eq) and chlorosulfonic acid (5 mL) was heated to 100 °C, and the mixture was stirred for 1 h. LCMS showed that the reaction was completed. The reaction system was added to ice water to quench the reaction, and the mixture was adjusted to pH 7 to 8 with a saturated aqueous sodium bicarbonate solution and extracted three times with ethyl acetate. The organic phases were combined, washed 3 times with a saturated sodium chloride solution, dried over anhydrous sodium sulfate, subjected to suction filtration, and concentrated by rotary evaporation. The crude product was purified by normal phase column chromatography to give sulfonyl chloride compound D-19 (67 mg, yield: 22.1%) as a yellow solid.

Sulfonyl chloride compound D-19: $^1$H NMR (400 MHz, $CDCl_3$-*d*) δ (ppm): 9.34 (s, 1H), 8.37 (d, *J* = 8.0 Hz, 1H), 8.29 (d, *J* = 8.0 Hz, 1H), 7.58-7.54 (m, 2H), 4.23 (s, 3H).

**IX.** A preparation method for a sulfonyl chloride compound was different from Example 1 in that the following sulfonyl chloride compound D-20 (quinoline-8-sulfonyl chloride) was prepared according to a preparation method similar to that for D-19, and the sulfonyl chloride compound D-20 has a structure shown as follows:

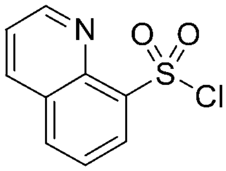

D-20

Sulfonyl chloride compound D-20: $^1$H NMR (400 MHz, DMSO-$d_6$) δ (ppm): 9.42-9.36(m, 2H), 8.48-8.45(m, 2H), 8.23-8.20(m, 1H), 8.04-8.00(m, 1H).

**X.** A preparation method for a sulfonyl compound was different from Example 1 in that C09A was replaced by a structure represented by D-21:

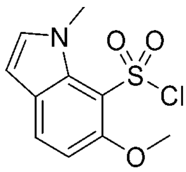

(D-21)

The synthetic route of D-21 is as follows:

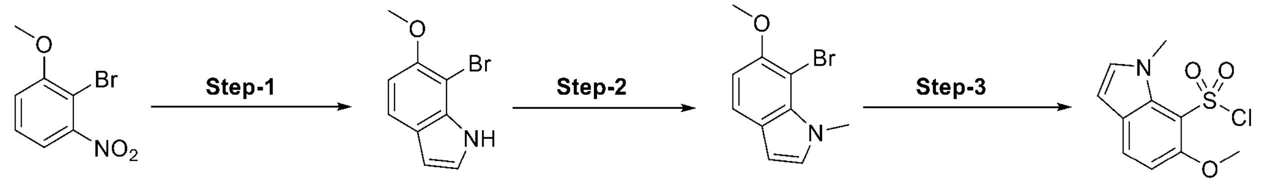


**SM1** **D-21-1** **D-21-2** **D-21**

Step-1: Preparation of compound D-21-1: SM1 (10.1 g, 1 eq) was dissolved in tetrahydrofuran (800 mL). Under nitrogen atmosphere, vinylmagnesium bromide (1 M in THF, 120 mL, 3 eq) was added dropwise at -78 °C. The mixture was stirred at -78 °C for 3 h, and TLC showed that the reaction was completed. The reaction system was added to a saturated aqueous ammonium chloride solution to quench the reaction, and the mixture was extracted three times with ethyl acetate. The organic phases were combined, washed 3 times with a saturated sodium chloride solution, dried over anhydrous sodium sulfate, subjected to suction filtration, and concentrated by rotary evaporation. The crude product was purified by normal phase column chromatography to give compound D-21-1 (3.79 g, yield: 38.9%). $[M+1]^+$: 226.05.

Step-2: Preparation of compound D-21-2: D-21-1 (3.7 g, 1 eq) was dissolved in tetrahydrofuran (100 mL). Under nitrogen atmosphere, sodium hydride (0.98 g, 60% content, 1.5 eq) was added at 0 °C. Under $N_2$ atmosphere, the mixture was stirred at 0 °C for 20 min, and then iodomethane (3.48 g, 1.5 equiv) was added to the reaction system. The mixture was stirred at 0 °C for 1.5 h, and LCMS showed that the reaction was completed. The reaction system was added to a saturated ammonium chloride solution to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give compound D-21-2 (3.4 g, yield: 86.5%). $[M+1]^+$: 240.05. $^1H$ NMR (400 MHz, DMSO-$d_6$) δ (ppm): 7.48 (d, *J* = 8.4 Hz, 1H), 7.22 (d, *J* = 2.8 Hz, 1H), 6.91 (d, *J* = 8.8 Hz, 1H), 6.39 (d, *J* = 3.2 Hz, 1H), 4..9 (s, 3H), 3.85 (s, 3H).

Step-3: Preparation of compound D-21: The sulfonyl chloride compound D-21 was prepared according to the procedure of Step-3 for compound D-15. $[M+1]^+$: 260.10.

**XI.** A preparation method for a sulfonyl compound was different from Example 1 in that C09A was replaced by structures represented by D-22A and D-22B:

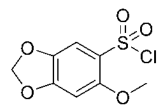

**D-22A**

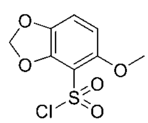

**D-22B**

The synthetic routes of D-22A and D-22B are as follows:

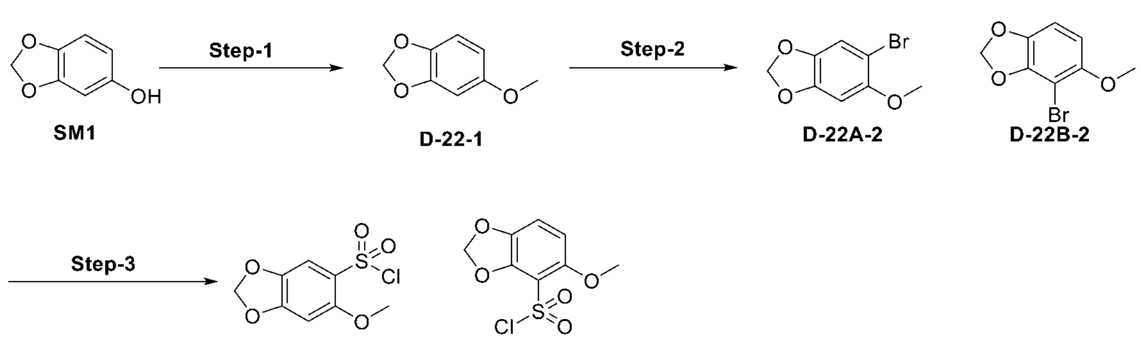


**SM1** **D-22-1** **D-22A-2** **D-22B-2** **D-22A** **D-22B**

Step-1: Preparation of compound D-22-1: SM1 (15.1 g, 1 eq) and potassium carbonate (16.5 g, 1 eq) was added to acetone (500 mL). Under nitrogen atmosphere, the mixture was stirred at 80 °C for 1 h and cooled to 0 °C. Iodomethane (84.8 g, 5.5 equiv) was added, and the mixture was heated to 80 °C and stirred for 16 h. GCMS showed that the reaction was completed. The reaction system was added to water to quench the reaction, and the mixture was extracted three times with ethyl acetate. The organic phases were combined, washed 3 times with a saturated sodium chloride solution, dried

over anhydrous sodium sulfate, subjected to suction filtration, and concentrated by rotary evaporation. The crude product was purified by normal phase column chromatography to give compound D-22-1 (15.1 g, yield: 90.1%). GCMS (ES, m/z): $[M]^+$:152.

Step-2: Preparation of compounds D-22A-2 and D-22B-2: D-22-1 (5 g, 1 eq) was dissolved in tetrahydrofuran (250 mL). Under nitrogen atmosphere, bromine (5.25 g, 1.0 eq) was added dropwise at 0 °C. Under $N_2$ atmosphere, the mixture was stirred at 0 °C for 20 min. The reaction system was added to a saturated aqueous sodium carbonate solution to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give a mixture of compounds D-22A-2 and D-22B-2 (7.1 g, yield: 92.2%). GCMS (ES, m/z): $[M]^+$:230/232. Step-3: Preparation of compounds D-22A and D-22B: The sulfonyl chloride compounds D-22A and D-22B were prepared according to the procedure of Step-3 for compound D-15.

Sulfonyl chloride compound D-22A: $^1H$ NMR (400 MHz, $CDCl_3$-*d*) δ (ppm): 7.40 (s, 1H), 6.67 (s, 1H), 6.11 (s, 2H), 4.03 (s, 3H).

Sulfonyl chloride compound D-22B: $^1H$ NMR (400 MHz, $CDCl_3$-*d*) δ (ppm): 7.11 (d, *J* = 8.4 Hz, 1H), 6.45 (d, *J* = 8.4 Hz, 1H), 6.34 (s, 2H), 3.98 (s, 3H).

**XII.** A preparation method for a sulfonyl compound was different from Example 1 in that C09A was replaced by a structure represented by D-23:

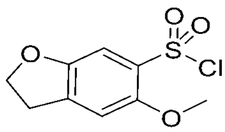
(D-23)

The synthetic route of D-23 is as follows:

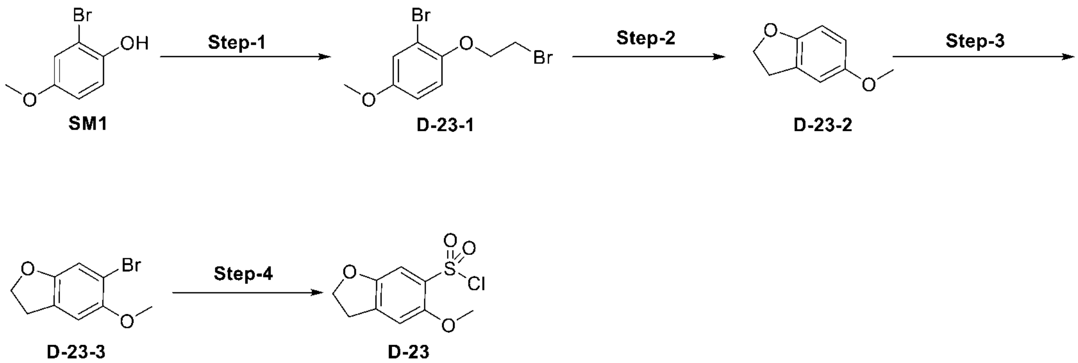


SM1 D-23-1 D-23-2

D-23-3 D-23

Step-1: Preparation of compound D-23-1: Sodium hydroxide (14.2 g, 2.4 eq) was dissolved in 59 mL of water at 0 °C, and then SM1 (30.1 g, 1 eq) and 1,2-dibromoethane (111.1 g, 4 eq) were added to the aqueous sodium hydroxide solution described above. The mixture was heated to 100 °C and stirred for 16 h. TLC showed that the reaction was completed. The reaction system was added to water for dilution and extracted three times with dichloromethane. The organic phases were combined, washed 3 times with a saturated sodium chloride solution, dried over anhydrous sodium sulfate, subjected to suction filtration, and concentrated by rotary evaporation. The crude product was purified by normal phase column chromatography to give compound D-23-1 (28.3 g, yield: 56.2%).

Step-2: Preparation of compound D-23-2: D-23-1 (20.1 g, 1 eq) was dissolved in tetrahydrofuran (250 mL). Under nitrogen atmosphere, n-butyllithium (26 mL, 2.5 M in hexane, 1.0 eq) was added dropwise at -78 °C, and the mixture was stirred at -78 °C for 2 h. TLC showed that the reaction was completed. The reaction system was added to a saturated aqueous ammonium chloride solution to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give a mixture of compound D-23-2 (8.2 g, yield: 84.2%). $^1H$ NMR (400 MHz, $CDCl_3$-*d*) δ (ppm): 6.79 (t, *J* = 1.2 Hz, 1H), 6.70-6.63 (m, 2H), 4.53 (t, *J* = 8.8 Hz, 2H), 3.75 (s, 3H), 3.18 (t, *J* = 8.4 Hz, 2H).

Step-3: Preparation of compound D-23-3: The mixture of compound D-23-2 (8.2 g, 1 eq) was dissolved in 240 mL

of dichloromethane, and dibromohydantoin (11.4 g, 0.5 eq) was added at 0 °C. The mixture was stirred at 0 °C for 2 h, and TLC showed that the reaction was completed. The reaction system was added to water to quench the reaction, and the mixture was extracted three times with dichloromethane. The organic phases were combined, washed 3 times with a saturated sodium chloride solution, dried over anhydrous sodium sulfate, subjected to suction filtration, and concentrated by rotary evaporation. The crude product was purified by normal phase column chromatography to give compound D-23-3 (9.4 g, yield: 75.4%). $^1$H NMR (400 MHz, $CDCl_3$-*d*) δ (ppm): 7.06 (s, 1H), 6.98 (s, 1H), 4.51 (t, *J* = 8.8 Hz, 2H), 3.76 (s, 3H), 3.14 (t, *J* = 8.8 Hz, 2H).

Step-4: Preparation of compound D-23: The sulfonyl chloride compound D-23 was prepared according to the procedure of Step-3 for compound D-15.

Sulfonyl chloride compound D-23: $^1$H NMR (400 MHz, $CDCl_3$-*d*) δ (ppm): 7.35 (s, 1H), 7.03 (s, 1H), 4.66 (t, *J* = 8.8 Hz, 2H), 4.02 (s, 3H), 3.33 (t, *J* = 8.8 Hz, 2H).

**XIII.** A preparation method for a sulfonyl compound was different from Example 1 in that C09A was replaced by a structure represented by D-24:

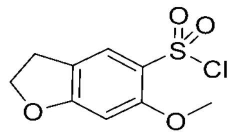

(D-24)

The synthetic route of D-24 is as follows:

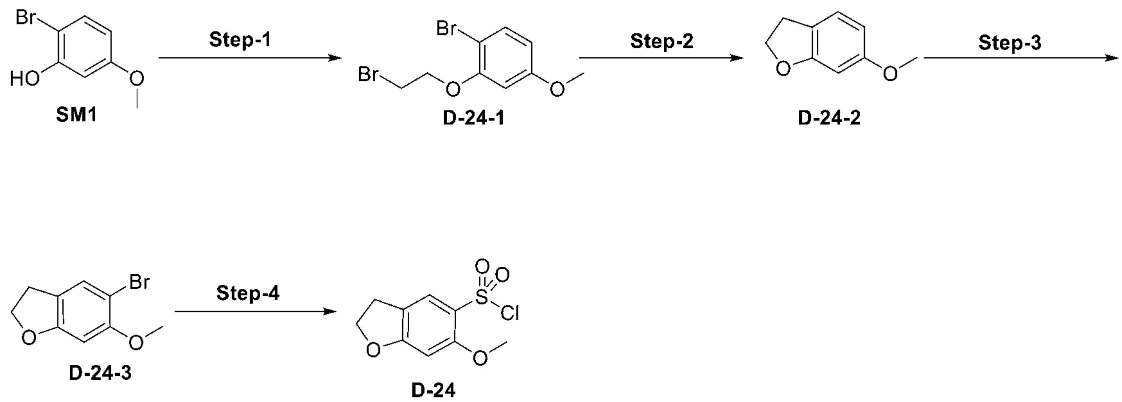


Synthesis of sulfonyl chloride compound D-24: Compound D-24 was synthesized by procedures similar to Step-1 to Step-4 for D-23 according to a preparation method similar to that for D-23, with SM1 as a starting material. Sulfonyl chloride compound D-24: $^1$H NMR (400 MHz, $CDCl_3$-*d*) δ (ppm): 7.78 (s, 1H), 6.52 (s, 1H), 4.75 (t, *J* = 8.8 Hz, 2H), 4.06 (s, 3H), 3.24 (t, *J* = 8.8 Hz, 2H).

**XIV.** A preparation method for a sulfonyl compound was different from Example 1 in that C09A was replaced by a structure represented by D-25:

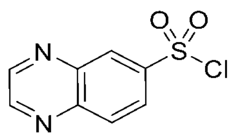

(D-25)

The synthetic route of D-25 is as follows:

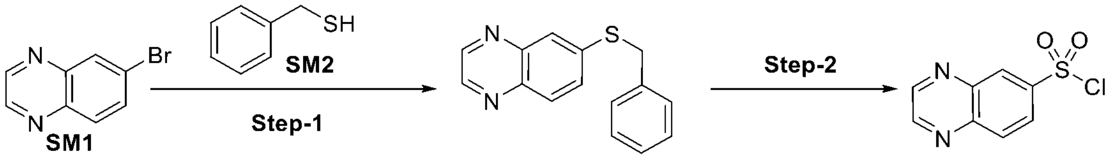

SM1 D-25-1 D-25

Step-1: Preparation of compound D-25-1: SM1 (1.5 g, 1.0 equiv), SM2 (1.34 g, 1.5 equiv), $Pd_2(dba)_3$ (657.1 mg, 0.1 equiv), XantPhos (830.4 mg, 0.2 equiv), and triethylamine (2.18 g, 3 equiv) were dissolved in 1,4-dioxane (20 mL). Under nitrogen atmosphere, the mixture was stirred at 100 °C overnight. LCMS showed that the reaction was completed. The reaction system was added to water to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give compound D-25-1 (1.8 g, yield: 99.2%). $[M+H]^+$: 253.15. Step-2: Preparation of compound D-25: Compound D-25-1 (1.5 g, 1.0 equiv) was dissolved in dichloromethane (30 mL), and acetic acid (6 mL) was added. The reaction system was cooled to 0 °C, and $SO_2Cl_2$ (6.1 g, 7.5 eq) was added dropwise under stirring. After the dropwise addition, the mixture was successively stirred at 0 °C for 10 min, and 0.12 mL of water was added at 0 °C. The reaction mixture was warmed to room temperature and successively stirred for 1 h. LCMS showed that the reaction was completed. The reaction mixture was added to ice water (5 mL) to quench the reaction, and the organic phase was separated. The reaction mixture was extracted 3 times with dichloromethane. The organic phases were combined, washed with a saturated aqueous sodium chloride solution, dried over anhydrous sodium sulfate, subjected to suction filtration, concentrated by rotary evaporation, and purified by normal phase column chromatography to give compound D-25 (680 mg, yield: 50.2%). $[M+H]^+$: 229.05. $^1H$ NMR (400 MHz, $CDCl_3$-*d*) $\delta$ (ppm): 9.08 (s, 2H), 8.90 (d, *J* = 1.6 Hz, 1H), 8.42-8.32 (m, 2H).

**XV.** A preparation method for a sulfonyl compound was different from Example 1 in that C09A was replaced by a structure represented by D-26:

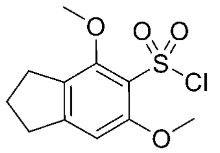
(D-26)

The synthetic route of D-26 is as follows:

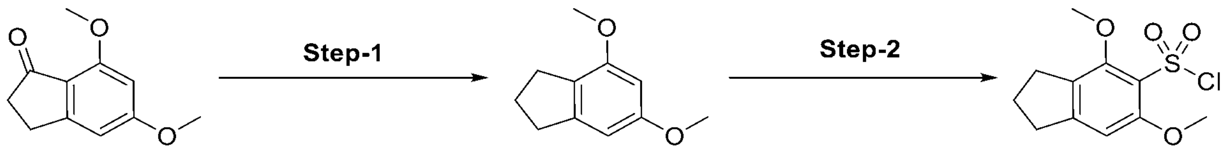

SM1 D-26-1 D-26

Step-1: Preparation of compound D-26-1: SM1 (3.9 g, 1.0 equiv) was dissolved in trifluoroacetic acid (26 mL), and triethylsilane (9.4 g, 4 eq) was added at room temperature. The mixture was stirred at room temperature overnight under nitrogen atmosphere. TLC showed that the reaction was completed. The reaction system was directly distilled under reduced pressure, and the crude product was purified by normal phase column chromatography to give compound D-26-1 (3.4 g, yield: 92.7%). $^1H$ NMR (400 MHz, $CDCl_3$-*d*) $\delta$ (ppm): 6.44 (d, *J* = 1.6 Hz, 1H), 6.30 (d, *J* = 1.6 Hz, 1H), 3.86-3.81 (m, 6H), 2.90 (t, *J* = 8.0 Hz, 2H), 2.82 (t, *J* = 8.0 Hz, 2H), 2.12-2.05 (m, 2H).

Step-2: Preparation of compound D-26: Compound D-26-1 (0.5 g, 1.0 equiv), tetramethylethylenediamine (358.6 mg, 1.1 eq) was dissolved in n-hexane (10 mL). Under nitrogen atmosphere, the mixture was cooled to -78 °C, and n-butyllithium (1.23 mL, 2.5 M in hexane, 1.1 eq) was added dropwise. The mixture was stirred at 0 °C for 2 h. Then, the reaction system was cooled to -65 °C. Sulfur dioxide gas was introduced for 10 min, and the mixture was naturally heated to 10 °C under stirring. The resulting precipitate was collected by filtration and then washed with diethyl ether (the filtration was performed under nitrogen atmosphere). The resulting solid was slurried with 20 mL of n-hexane again to form a homogeneous slurry. Under nitrogen atmosphere, the mixture was cooled to 0 °C, and sulfuryl chloride (208.2 mg, 0.55 eq)

was added dropwise. After the dropwise addition, the reaction mixture was successively stirred at 0 °C for 1 h, diluted with ethyl acetate (40 mL), backwashed 2 times with a saturated sodium chloride solution, and dried over sodium sulfate. After the resulting mixture was filtered, the filtrate was concentrated under reduced pressure and purified by normal phase column chromatography to give compound D-26 (270 mg, yield: 34.6%). $^1H$ NMR (400 MHz, $CDCl_3$-*d*) δ (ppm): 6.75 (s, 1H), 3.97 (s, 6H), 3.01-2.96 (m, 4H), 2.19-2.11(m, 2H).

**XVI.** A preparation method for a sulfonyl compound was different from Example 1 in that C09A was replaced by a structure represented by D-27:

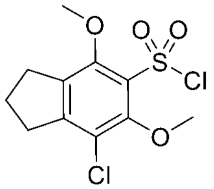

(D-27)

The synthetic route of D-27 is as follows:

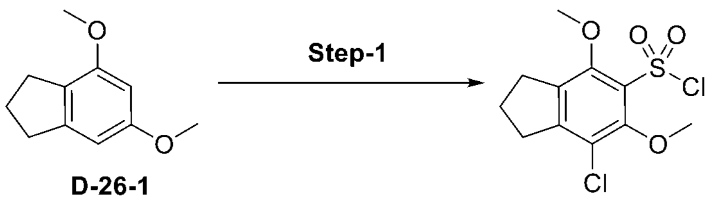


D-26-1

D-27

Step-1: Preparation of compound D-27: Compound D-26-1 (0.6 g, 1.0 equiv), tetramethylethylenediamine (430 mg, 1.1 eq) was dissolved in n-hexane (15 mL). Under nitrogen atmosphere, the mixture was cooled to -78 °C, and n-butyllithium (1.48 mL, 2.5 M in hexane, 1.1 eq) was added dropwise. The mixture was stirred at 0 °C for 2 h. Then, the reaction system was cooled to -65 °C. Sulfur dioxide gas was introduced for 10 min, and the mixture was naturally heated to 10 °C under stirring. The resulting precipitate was collected by filtration and then washed with diethyl ether (the filtration was performed under nitrogen atmosphere). The resulting solid was slurried with 20 mL of n-hexane again to form a homogeneous slurry. Under nitrogen atmosphere, the mixture was cooled to 0 °C, and sulfuryl chloride (500.6 mg, 1.1 eq) was added dropwise. After the dropwise addition, the reaction mixture was successively stirred at 0 °C for 1 h, diluted with ethyl acetate (40 mL), backwashed 2 times with a saturated sodium chloride solution, and dried over sodium sulfate. After the resulting mixture was filtered, the filtrate was concentrated under reduced pressure and purified by normal phase column chromatography to give compound D-27 (100 mg, yield: 9.6%). $^1H$ NMR (400 MHz, $CDCl_3$-*d*) δ (ppm): 4.03 (s, 3H), 3.97 (s, 3H), 3.14-3.05 (m, 4H), 2.23-2.20(m, 2H).

**XVII.** A preparation method for a sulfonyl compound was different from Example 1 in that C09A was replaced by a structure represented by D-28:

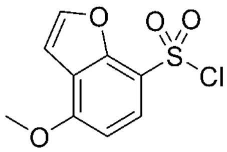

(D-28)

The synthetic route of D-28 is as follows:

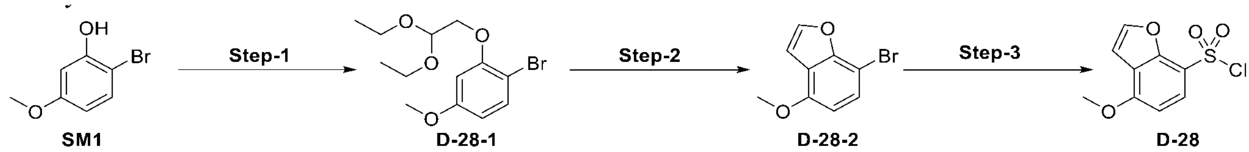


Synthesis of sulfonyl chloride compound D-28: Compound D-28 was synthesized by procedures similar to Step-1 to Step-3 for D-15 according to a preparation method similar to that for D-15, with SM1 as a starting material. $^1H$ NMR (400 MHz, $CDCl_3$-*d*) δ (ppm): 7.59 (d, *J* = 2.0 Hz, 1H), 7.35 (d, *J* = 8.4 Hz, 1H), 6.92 (d, *J* = 2.4 Hz, 1H), 6.56 (d, *J* = 8.4 Hz, 1H), 3.92 (s, 3H).

**XVIII.** A preparation method for a sulfonyl compound was different from Example 1 in that C09A was replaced by a structure represented by D-29:

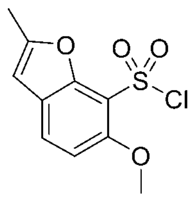

(D-29)

The synthetic route of D-29 is as follows:

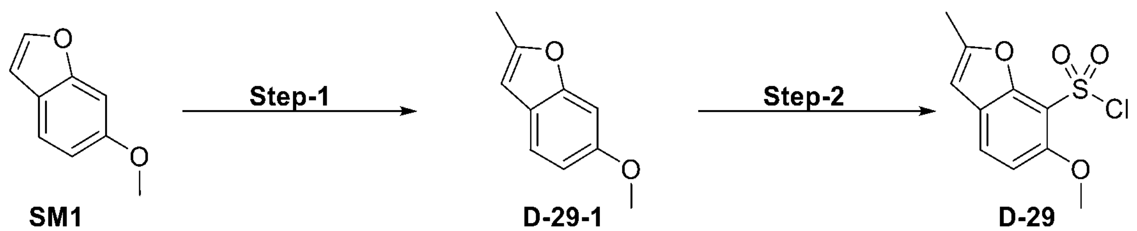


Step-1: Preparation of compound D-29-1: SM1 (4.6 g, 1.0 equiv) was dissolved in tetrahydrofuran (92 mL). Under nitrogen atmosphere, the mixture was cooled to -78 °C, and n-butyllithium (15 mL, 2.5 M in hexane, 1.2 eq) was added dropwise. The temperature was maintained, and the mixture was stirred for 30 min. Then, a solution of iodomethane (8.8 g, 2 eq) in tetrahydrofuran was added dropwise. The mixture was successively stirred at 0 °C for 2 h, and TLC showed that the reaction was completed. The reaction system was added to a saturated aqueous ammonium chloride solution to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give compound D-29-1 (3.7 g, yield: 64.2%). $^1H$ NMR (400 MHz, DMSO-$d_6$) δ (ppm): 7.37 (d, *J* = 8.4 Hz, 1H), 7.10 (d, *J* = 2.0 Hz, 1H), 6.83-6.80 (m, 1H), 6.47-6.46 (m, 1H), 3.78 (s, 3HHJH JH), 2.40 (s, 3H).

Step-2: Preparation of compound D-29: Compound D-29 was synthesized according to a procedure similar to Step-2 in the preparation method for D-26, with D-29-1 as a starting material.

Sulfonyl chloride compound D-29: $^1H$ NMR (400 MHz, $CDCl_3$-*d*) δ (ppm): 7.70 (d, *J* = 8.4 Hz, 1H), 6.96 (d, *J* = 8.4 Hz, 1H), 6.39 (s, 1H), 4.07 (s, 3HHJH JH), 2.51 (s, 3H).

**XIX.** A preparation method for a sulfonyl compound was different from Example 1 in that C09A was replaced by a structure represented by D-30:

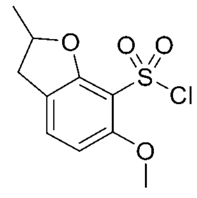

(D-30)

The synthetic route of D-30 is as follows:

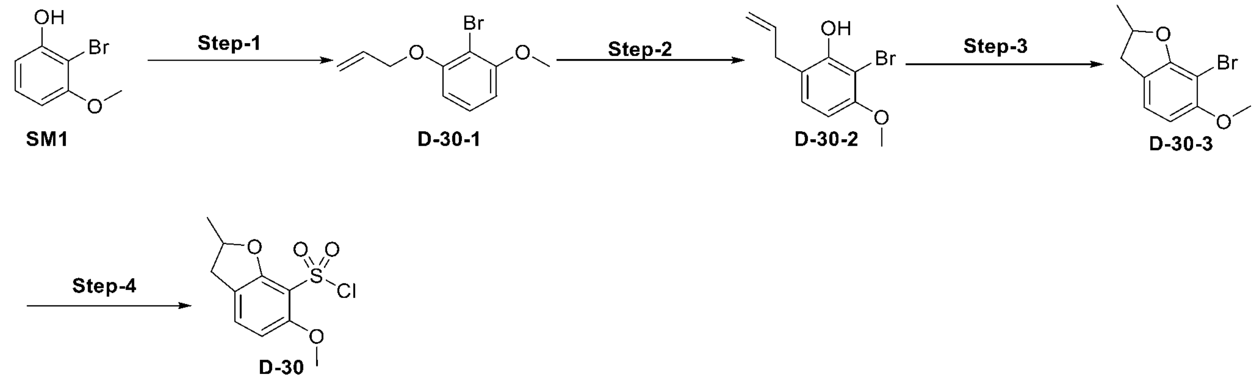


Step-1: Preparation of compound D-30-1: SM1 (5.1 g, 1.0 eq), 3-bromopropene (4.5 g, 1.5 eq), and potassium carbonate (8.5 g, 2.5 eq) were dissolved in *N,N*-dimethylformamide (100 mL). Under nitrogen atmosphere, the mixture was heated to 120 °C and stirred for 16 h. TLC showed that the reaction was completed. The reaction system was added to water to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give compound D-30-1 (5.8 g, yield: 91.5%). $^1$H NMR (400 MHz, DMSO-$d_6$) δ (ppm): 7.28 (t, *J* = 8.4 Hz, 1H), 6.74-6.72 (m, 2H), 6.08-6.03 (m, 1H), 5.48-5.43 (m, 1H), 5.29-5.26 (m, 1H), 4.65-4.63 (m, 2H), 3.84 (s, 3H).

Step-2: Preparation of compound D-30-2: D-30-1 (3.0 g, 1.0 equiv) was dissolved in n-hexane (30 mL). Under nitrogen atmosphere, the mixture was cooled to 0 °C, and diethylaluminium chloride (12.4 mL, 1 M in hexane, 1 eq) was added. The resulting mixture was slowly warmed to room temperature and stirred for 1 h. TLC showed that the reaction was completed. The reaction system was added to water to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give compound D-30-2 (1.9 g, yield: 51.8%). $^1$H NMR (400 MHz, $CDCl_3$-*d*) δ (ppm): 7.01 (d, *J* = 8.4 Hz, 1H), 6.44 (d, *J* = 8.4 Hz, 1H), 6.03-5.93 (m, 1H), 5.72 (s, 1H), 5.09-5.04 (m, 2H), 3.87 (s, 3H), 3.40-3.38 (m, 2H).

Step-3: Preparation of compound D-30-3: D-30-2 (1.6 g, 1.0 eq) was dissolved in trichloromethane (25 mL). Under nitrogen atmosphere, the mixture was cooled to 0 °C, and copper trifluoromethanesulfonate (0.9 g, 0.38 eq) was added. The resulting mixture was heated to 65 °C and stirred for 16 h. TLC showed that the reaction was completed. The reaction system was added to water to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give compound D-30-3 (480 mg, yield: 30.2%). $^1$H NMR (400 MHz, $CDCl_3$-*d*) δ (ppm): 7.01 (d, *J* = 8.4 Hz, 1H), 6.40 (d, *J* = 8.4 Hz, 1H), 5.09-5.05 (m, 1H), 3.88 (s, 3H), 3.42-3.34 (m, 1H), 2.91-2.85 (m, 1H), 1.54-1.52 (m, 3H).

Step-4: Preparation of compound D-30: The sulfonyl chloride compound D-30 was prepared according to the procedure of Step-3 for compound D-15.

**XX.** A preparation method for a sulfonyl compound was different from Example 1 in that C09A was replaced by a structure represented by D-31:

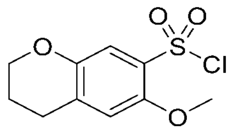

(D-31)

The synthetic route of D-31 is as follows:

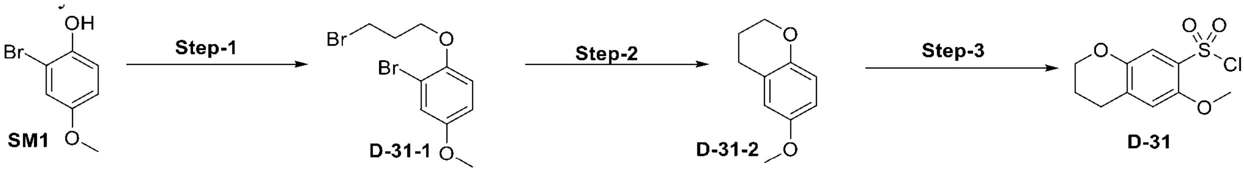


Step-1 and Step-2: Preparation of compound D-31-2: Compound D-31-2 was synthesized by Step-1 and Step-2 for compound D-23-2 according to a preparation method similar to that for D-23, with SM1 and 1,3-dibromopropane as starting materials. $^1$H NMR (400 MHz, $CDCl_3$-*d*) δ (ppm): 6.76-6.68 (m, 2H), 6.61 (d, *J* = 2.0 Hz, 1H), 4.19-4.17 (m, 2H), 3.77 (s, 3H), 2.82-2.78 (m, 2H), 2.04-1.98 (m, 2H).

Step-3: Preparation of compound D-31: D-31-2 (3.1 g, 1.0 eq) was dissolved in dichloromethane (50 mL). Under nitrogen atmosphere, the mixture was cooled to 0 °C, and chlorosulfonic acid (6.4 g, 3 eq) was added dropwise. The resulting mixture was stirred for 1 h. TLC showed that the reaction was completed. The reaction system was added to a saturated aqueous sodium bicarbonate solution to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give compound D-31 (800 mg, yield: 16.2%).

Sulfonyl chloride compound D-31: $^1$H NMR (400 MHz, $CDCl_3$-*d*) δ (ppm): 7.40 (s, 1H), 6.77 (s, 1H), 4.19(t, *J* = 8.8 Hz, 2H), 3.97 (s, 3H), 2.88-2.85 (m, 2H), 2.06-2.00 (m, 2H).

**XXI.** A preparation method for a sulfonyl compound was different from Example 1 in that C09A was replaced by a structure represented by D-32:

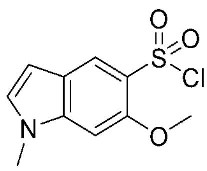

(D-32)

The synthetic route of D-32 is as follows:

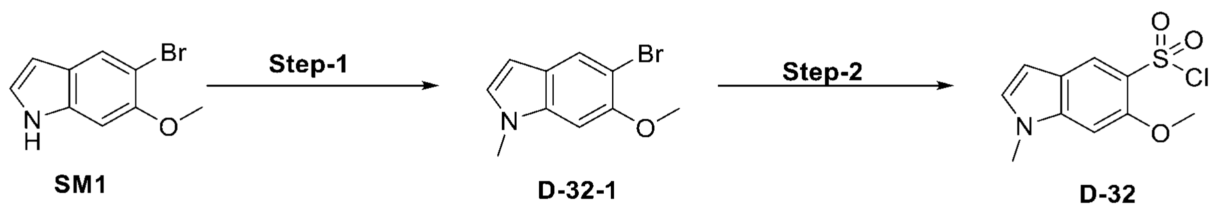


Preparation of compound D-32: Compound D-32 was synthesized according to procedures similar to Step-2 and Step-3 for compound D-21, with SM1 as a starting material. [M+1]$^+$: 260.15.

Sulfonyl chloride compound D-32: $^1$H NMR (400 MHz, $CDCl_3$-*d*) δ (ppm): 7.72 (s, 1H), 7.22 (d, *J* = 3.2 Hz, 1H), 7.15 (s, 1H), 3.88 (s, 3H), 3.48 (s, 3H).

**XXII.** A preparation method for a sulfonyl compound was different from Example 1 in that C09A was replaced by a structure represented by D-33:

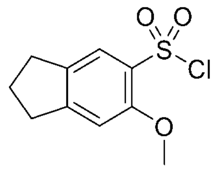

(D-33)

The synthetic route of D-33 is as follows:

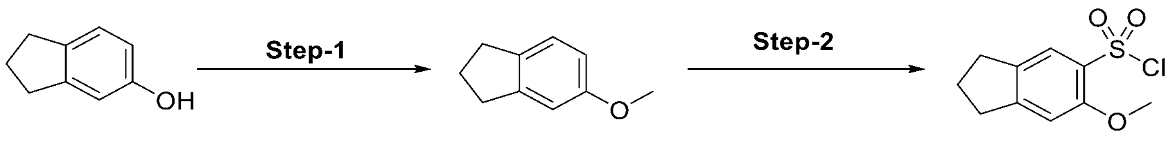


SM1 D-33-1 D-33

Step-1: Preparation of compound D-33-1: SM1 (20.1 g, 1 eq), potassium carbonate (32.9 g, 1.6 eq), and iodomethane (31.8 g, 1.5 eq) were mixed in N,N-dimethylformamide (DMF, 120 mL). The mixture was heated to 60 °C under stirring and stirred for 16 h. TLC showed that the reaction was completed. The reaction system was added to water to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give compound D-33-1 (18.2 g, yield: 81.2%). GCMS (ES, m/z), $[M]^+$: 184. $^1H$ NMR (400 MHz, $CDCl_3$-*d*) δ (ppm): 7.16 (d, *J* = 8.0 Hz, 1H), 6.84 (d, *J* = 2.0 Hz, 1H), 6.75-6.72 (m, 1H), 3.85 (s, 3H), 2.95-2.87 (m, 4H), 2.16-2.08 (m, 2H). Step-2: Preparation of compound D-33: Compound D-33 was synthesized according to a procedure similar to Step-3 for compound D-31, with D-33-1 as a starting material.

Sulfonyl chloride compound D-33: $^1H$ NMR (400 MHz, $CDCl_3$-*d*) δ (ppm): 7.79 (s, 1H), 7.01 (s, 1H), 4.04 (s, 3H), 3.03-2.99 (m, 2H), 2.95-2.89 (m, 2H), 2.21-2.12 (m, 2H).

**XXIII.** A preparation method for a sulfonyl compound was different from Example 1 in that C09A was replaced by a structure represented by D-34:

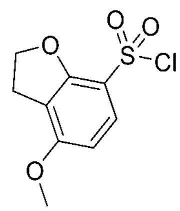

(D-34)

The synthetic route of D-34 is as follows:

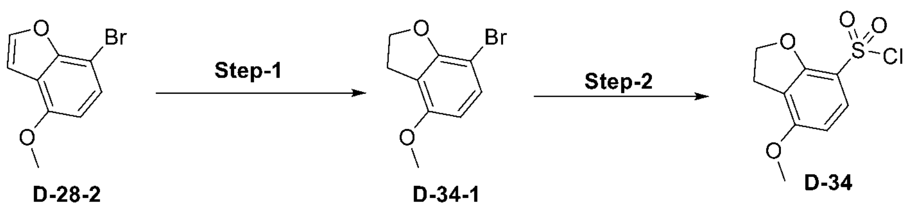


D-28-2 D-34-1 D-34

Preparation of compound D-34: Compound D-34 was synthesized by Step-1 and Step-2 for D-16 according to a procedure similar to that for D-16, with compound D-28-2 as a starting material.

Sulfonyl chloride compound D-34: $^1H$ NMR (400 MHz, $CDCl_3$-*d*) δ (ppm): 7.71 (d, *J* = 9.2 Hz, 1H), 6.54 (d, *J* = 9.2 Hz, 1H), 4.92 (t, *J* = 8.8 Hz, 2H), 3.96 (s, 3H), 3.27 (t, *J* = 8.8 Hz, 2H).

**XXIV.** A preparation method for a sulfonyl compound was different from Example 1 in that C09A was replaced by a structure represented by D-35:

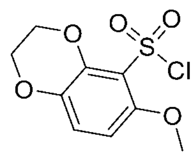

(D-35)

The synthetic route of D-35 is as follows:

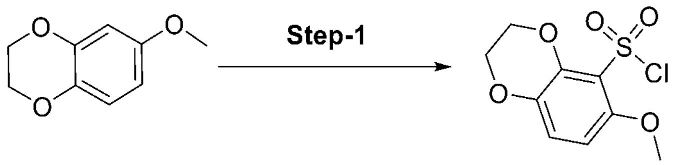


Preparation of compound D-35: Compound D-35 was synthesized according to a procedure similar to Step-2 in the preparation method for D-26, with SM1 as a starting material.

Sulfonyl chloride compound D-35: $^1H$ NMR (400 MHz, $CDCl_3$-*d*) δ (ppm): 7.15 (d, *J* = 9.2 Hz, 1H), 7.58 (d, *J* = 9.2 Hz, 1H), 4.47-4.45 (m, 2H), 4.31-4.29 (m, 2H), 3.96 (s, 3H).

**XXV.** A preparation method for a sulfonyl compound was different from Example 1 in that C09A was replaced by a structure represented by D-36:

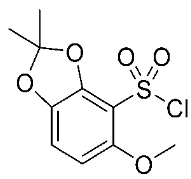

(D-36)

The synthetic route of D-36 is as follows:

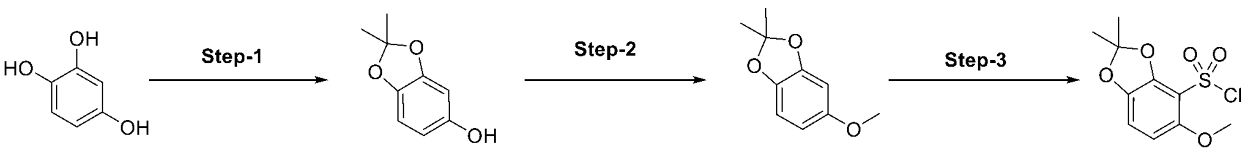


Step-1: Preparation of compound D-36-1: Under nitrogen atmosphere, a mixture of SM1 (5.1 g, 1 eq), pyridinium p-toluenesulfonate (PPTS, 80 mg, 0.01 eq), and toluene (400 mL) was heated to 130 °C and refluxed using a water separator. 2,2-Dimethoxypropane (6.2 g, 1.5 equiv) was added in batches every 15 min, and the system was supplemented with toluene (50 mL) at the same time to make up for solvent loss. The mixture was reacted for 2 h, and TLC showed that the reaction was completed. The mixture was directly mixed with silica gel and purified by normal phase column chromatography to give compound D-36-1 (3.7 g, yield: 56.2%). $^1H$ NMR (400 MHz, DMSO-$d_6$) δ (ppm): 8.95 (s, 1H), 6.55 (d, *J* = 8.4 Hz, 1H), 6.28 (d, *J* = 2.4 Hz, 1H), 6.14-6.11 (m, 1H), 1.58 (s, 6H).

Preparation of compound D-36: Compound D-36 was synthesized according to procedures similar to Step-1 and

Step-2 for compound D-33, with D-36-1 as a starting material.

Sulfonyl chloride compound D-36: $^{1}$H NMR (400 MHz, $CDCl_3$-*d*) δ (ppm): 6.90 (d, *J* = 8.8 Hz, 1H), 6.37 (d, *J* = 8.4 Hz, 1H), 3.96 (s, 3H), 1.75 (s, 6H).

**XXVI.** A preparation method for a sulfonyl compound was different from Example 1 in that C09A was replaced by a structure represented by D-37:

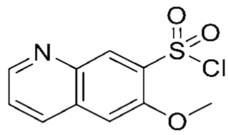

(D-37)

The synthetic route of D-37 is as follows:

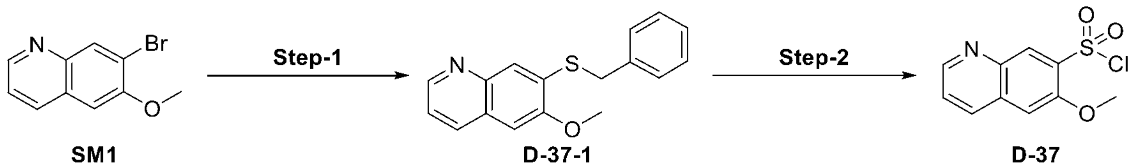


Preparation of compound D-37: Compound D-37 was synthesized according to procedures similar to Step-1 and Step-2 for compound D-25, with SM1 as a starting material.

Sulfonyl chloride compound D-37: $^{1}$H NMR (400 MHz, $CDCl_3$-*d*) δ (ppm): 9.46 (s, 1H), 9.08 (s, 1H), 8.73(d, *J* = 8.8 Hz, 1H), 8.01 (s, 1H), 7.58 (s, 1H), 4.27 (s, 3H).

**XXVII.** A preparation method for a sulfonyl compound was different from Example 1 in that C09A was replaced by a structure represented by D-38:

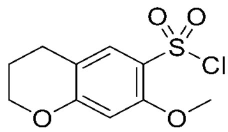

(D-38)

The synthetic route of D-38 is as follows:

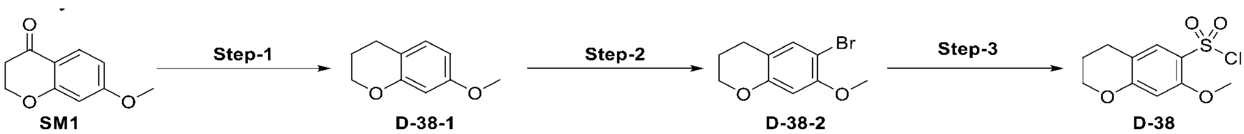


Step-1: Preparation of compound D-38-1: Compound SM1 (500 mg, 1 eq) was dissolved in acetic acid (3 mL) at room temperature. After complete dissolution, the mixture was added to a suspension containing zinc powder (10 g) in acetic acid (30 mL). The mixture was reacted at 100 °C overnight. TLC showed that the reaction was completed. The reaction solution was filtered, washed several times with ethyl acetate, concentrated under reduced pressure, and purified by normal phase column chromatography to give compound D-36-1 (0.42 g, yield: 90.2%).

Step-2: Preparation of compound D-38-2: Compound D-38-1 (420 mg, 1 eq) was dissolved in dichloromethane (10 mL) at room temperature. After complete dissolution, 1,3-dibromo-5,5-dimethylhydantoin (362 mg, 0.5 eq) was added, and the mixture was reacted at room temperature for 1 h. TLC showed that the reaction was completed. The reaction system was added to water to quench the reaction, and the reaction mixture was extracted three times with dichloromethane. The organic phases were combined, washed 3 times with a saturated sodium chloride solution, dried over anhydrous sodium sulfate, subjected to suction filtration, and concentrated by rotary evaporation. The crude product was purified by normal phase column chromatography to give D-38-2 (530 mg, yield: 86.21%). Step-3: Preparation of

compound D-38: The sulfonyl chloride compound D-38 was prepared according to the procedure of Step-3 for compound D-15.

Sulfonyl chloride compound D-38: $^1$H NMR (400 MHz, $CDCl_3$-*d*) δ (ppm): 7.70 (s, 1H), 6.45 (s, 1H), 4.26 (t, *J* = 5.2 Hz, 2H), 3.97 (s, 3H), 2.76 (t, *J* = 5.2 Hz, 2H), 2.06-1.99 (m, 2H).

**XXVIII.** A preparation method for a sulfonyl compound was different from Example 1 in that C09A was replaced by a structure represented by D-39:

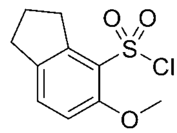

(D-39)

The synthetic route of D-39 is as follows:

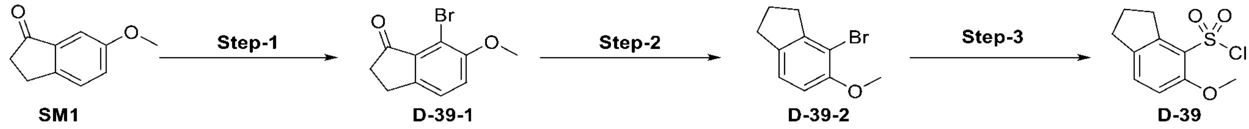


Step-1: Preparation of compound D-39-1: Compound SM1 (0.5 g, 1 eq) was dissolved in acetonitrile (50 mL) at room temperature. After complete dissolution, N-bromosuccinimide (NBS, 547.2 mg, 1 eq) and ferric chloride (0.5 g) were sequentially added. The mixture was stirred at room temperature for 1 h. TLC showed that the reaction was completed. The reaction system was added to water to quench the reaction, and the reaction mixture was extracted three times with ethyl acetate. The organic phases were combined, washed 3 times with a saturated sodium chloride solution, dried over anhydrous sodium sulfate, subjected to suction filtration, and concentrated by rotary evaporation. The crude product was purified by normal phase column chromatography to give compound D-39-1 (650 mg, yield: 87.84%).

Step-2: Preparation of compound D-39-2: Compound D-39-1 (650 mg, 1 eq) and triethylsilane (943 mg, 3 eq) were dissolved in TFA (10 mL). After complete dissolution, the mixture was stirred at room temperature for 1 h. TLC showed that the reaction was completed. The reaction system was added to a saturated aqueous sodium carbonate solution to quench the reaction, and the reaction mixture was extracted three times with ethyl acetate. The organic phases were combined, washed 3 times with a saturated sodium chloride solution, dried over anhydrous sodium sulfate, subjected to suction filtration, and concentrated by rotary evaporation. The crude product was purified by normal phase column chromatography to give D-39-2 (500 mg, yield: 81.20%).

Step-3: Preparation of compound D-39: The sulfonyl chloride compound D-39 was prepared according to the procedure of Step-3 for compound D-15.

Sulfonyl chloride compound D-39: $^1$H NMR (400 MHz, $CDCl_3$-*d*) δ (ppm): 7.49 (d, *J* = 8.8 Hz, 1H), 6.91 (d, *J* = 8.8 Hz, 1H), 4.04 (s, 3H), 3.45-3.44 (m, 2H), 2.91-2.85 (m, 2H), 2.18-2.08 (m, 2H).

**XXIX.** A preparation method for a sulfonyl compound was different from Example 1 in that C09A was replaced by a structure represented by D-40:

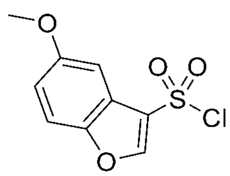

(D-40)

The synthetic route of D-40 is as follows:

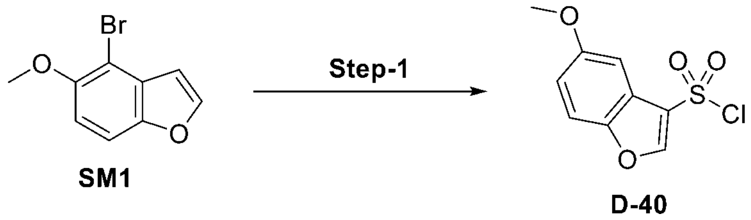

SM1
D-40

Step-1: Preparation of compound D-40: Under nitrogen atmosphere, D-SM1 (450 mg, 1 eq) was dissolved in freshly distilled tetrahydrofuran (20.0 mL), and n-butyllithium (2.5 M in n-hexane, 1.0 mL, 1.25 eq) was slowly added dropwise at -78 °C for reaction for 10 min. Subsequently, sulfur dioxide gas was introduced for 15 min. The mixture was slowly warmed to room temperature, and a solution of NCS (400 mg, 1.5 eq) in dichloromethane was added. The mixture was reacted at room temperature for 1 h. The reaction system was poured into a saturated aqueous ammonium chloride solution to quench the reaction, and the mixture was extracted three times with ethyl acetate. The organic phases were combined, dried over anhydrous sodium sulfate, filtered, and concentrated by rotary evaporation. The crude product was purified by normal phase chromatographic column to give sulfonyl compound D-40 (84 mg, yield: 17.2%) as a light yellow solid.

Sulfonyl compound D-40: $^1$H NMR (400 MHz, $CDCl_3$-*d*) δ (ppm): 7.60 (s, 1H), 7.57 (d, *J* = 8.4 Hz, 1H), 7.24 (dd, *J*=9.2, 2.4Hz, 1H), 7.15 (d, *J* = 2.0 Hz, 1H).

**XXX.** A preparation method for a sulfonyl compound was different from Example 1 in that C09A was replaced by a structure represented by D-41:

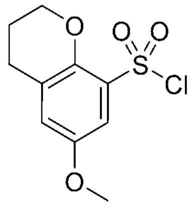
(D-41)

The synthetic route of D-41 is as follows:

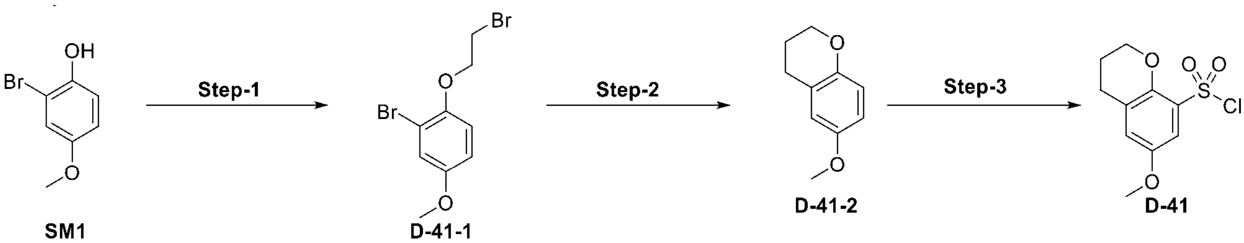

SM1 D-41-1 D-41-2 D-41

Preparation of compound D-41: Compound D-41 was synthesized according to procedures similar to Step-1 to Step-3 for compound D-31, with SM1 as a starting material.

Sulfonyl compound D-41: $^1$H NMR (400 MHz, $CDCl_3$-*d*) δ (ppm): 7.26 (d, *J* = 2.4 Hz, 1H), 6.96 (d, *J* = 2.4 Hz, 1H), 4.39 (t, *J* =5.2, 2H), 3.79 (s, 3H), 2.85 (t, *J* =6.4, 2H), 2.13-2.07 (m, 2H).

**XXXI.** A preparation method for a sulfonyl compound was different from Example 1 in that C09A was replaced by a structure represented by D-42:

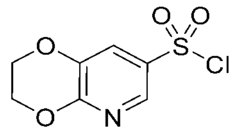

(D-42)

The synthetic route of D-42 is as follows:

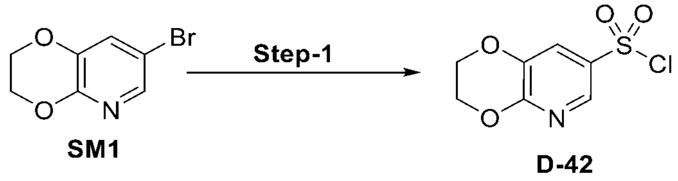


Preparation of compound D-42: Compound D-42 was synthesized according to a procedure similar to Step-3 for compound D-15, with SM1 as a starting material.

Sulfonyl compound D-42: $^1H$ NMR (400 MHz, $CDCl_3$-*d*) δ (ppm): 8.49 (s, 1H), 7.55 (d, *J* = 2.0 Hz, 1H), 4.61-4.59 (m, 2H), 4.39-4.37 (m, 2H).

**XXXII.** A preparation method for a sulfonyl compound was different from Example 1 in that C09A was replaced by a structure represented by D-43:

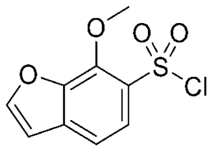

(D-43)

The synthetic route of D-43 is as follows:

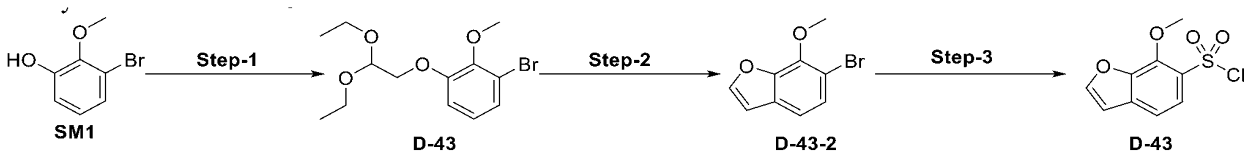


Preparation of compound D-43: Compound D-43 was synthesized by procedures similar to Step-1 to Step-3 for D-15 according to a preparation method similar to that for compound D-15, with SM1 as a starting material.

Sulfonyl compound D-43: $^1H$ NMR (400 MHz, $CDCl_3$-*d*) δ (ppm): 7.64 (d, *J* = 2.0 Hz, 1H), 7.38 (d, *J* = 8.4 Hz, 1H), 6.84 (d, *J* = 2.4 Hz, 1H), 6.66 (d, *J* = 8.4 Hz, 1H), 4.04 (s, 3H).

**XXXIII.** A preparation method for a sulfonyl compound was different from Example 1 in that C09A was replaced by a structure represented by D-44:

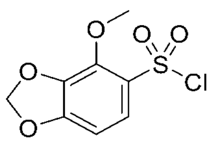

(D-44)

The synthetic route of D-44 is as follows:

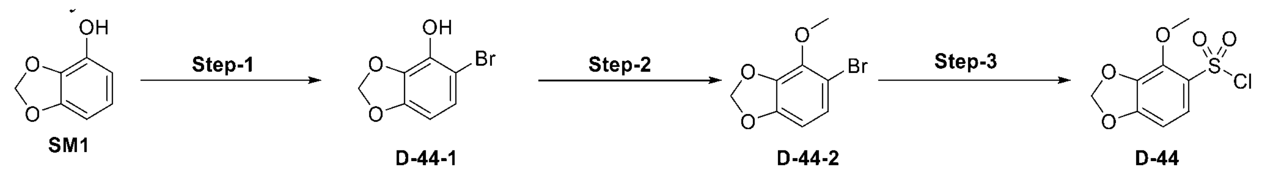


SM1 D-44-1 D-44-2 D-44

Step-1: Preparation of compound D-44-1: A solution of liquid bromine (0.15 mL) in trichloromethane (5 mL) was added dropwise to a stirred mixture of SM1 (0.4 g, 1 eq), silver trifluoroacetate (0.64 g, 1 eq), and trichloromethane (10 mL) at 0 °C. The mixture was stirred for 10 min, and TLC showed that the reaction was completed. The reaction system was added to a saturated aqueous sodium carbonate solution to quench the reaction, and the reaction mixture was extracted three times with dichloromethane. The organic phases were combined, washed 3 times with a saturated sodium chloride solution, dried over anhydrous sodium sulfate, subjected to suction filtration, and concentrated by rotary evaporation. The crude product was purified by normal phase column chromatography to give D-44-1 (0.35 g, yield: 56.4%). $^1$H NMR (400 MHz, $CDCl_3$-*d*) δ (ppm): 6.96 (d, J = 8.4 Hz, 1H), 6.40 (d, J = 8.8 Hz, 1H), 6.00 (s, 2H).

Preparation of compound D-44: Compound D-44 was synthesized according to procedures similar to Step-2 and Step-3 for compound D-21, with D-44-1 as a starting material.

Sulfonyl compound D-44: $^1$H NMR (400 MHz, $CDCl_3$-*d*) δ (ppm): 7.06 (d, J = 8.4 Hz, 1H), 6.56 (d, J = 8.4 Hz, 1H), 6.11 (s, 2H), 4.03 (s, 3H).

**XXXIV.** A preparation method for a sulfonyl compound was different from Example 1 in that C09A was replaced by a structure represented by D-45:

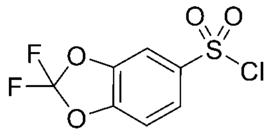

(D-45)

The synthetic route of D-45 is as follows:

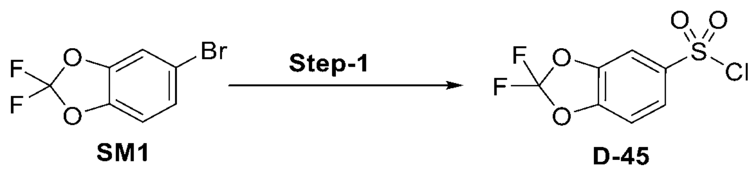


SM1 D-45

Step-1: Preparation of compound D-45: The sulfonyl chloride compound D-45 was prepared according to the procedure of Step-3 for compound D-15.

Sulfonyl compound D-45: $^1$H NMR (400 MHz, $CDCl_3$-*d*) δ (ppm): 7.59-7.57 (m, 1H), 7.38-7.36 (m, 1H), 7.26-7.24 (m, 1H).

**XXXV.** A preparation method for a sulfonyl compound was different from Example 1 in that C09A was replaced by a structure represented by D-46:

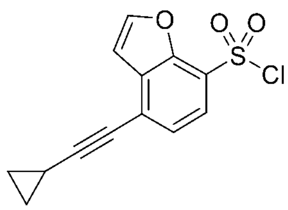

(D-46)

The synthetic route of D-46 is as follows:

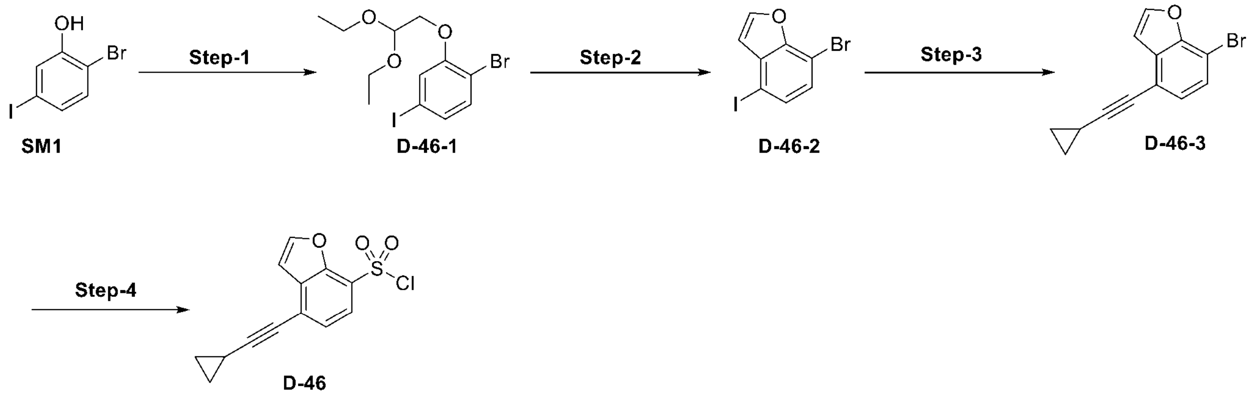


SM1 D-46-1 D-46-2 D-46-3 D-46

Step-1 to Step-2: Preparation of compound D-46-2: Compound D-46-2 was synthesized by procedures similar to Step-1 to Step-2 for D-15 according to a preparation method similar to that for compound D-15, with SM1 as a starting material. $^1$H NMR (400 MHz, $CDCl_3$-*d*) δ (ppm): 7.76 (s, 1H), 7.51 (d, *J* = 8.0 Hz, 1H), 7.24 (d, *J* = 8.0 Hz, 1H), 6.79 (s, 1H).
Step-3: Preparation of compound D-46-3: CuI (35.4 mg, 0.1 equiv) and Pd(dppf)$Cl_2$.$CH_2Cl_2$ (151.4 mg, 0.1 equiv) were added to a mixture of D-46-2 (600 mg, 1 equiv), THF (6.00 mL), and DIEA (6.00 mL). Under nitrogen atmosphere, cyclopropylacetylene (184.2 mg, 1.5 equiv) was added at 0 °C, and the mixture was stirred vigorously at room temperature overnight. TLC showed that the reaction was completed. The reaction system was added to water to quench the reaction, and the reaction mixture was extracted three times with ethyl acetate. The organic phases were combined, washed 3 times with a saturated sodium chloride solution, dried over anhydrous sodium sulfate, subjected to suction filtration, and concentrated by rotary evaporation. The crude product was purified by normal phase column chromatography to give compound D-46-3 (380 mg, yield: 76.8%). $^1$H NMR (400 MHz, $CDCl_3$-*d*) δ (ppm): 7.71 (s, 1H), 7.31 (d, *J* = 8.0 Hz, 1H), 7.17 (d, *J* = 8.0 Hz, 1H), 6.97 (s, 1H), 1.56-1.54(m, 1H), 0.97-0.88 (m, 4H). Preparation of compound D-46: The sulfonyl chloride compound D-46 was prepared according to the procedure of Step-3 for compound D-15.
Sulfonyl compound D-46: $^1$H NMR (400 MHz, $CDCl_3$-*d*) δ (ppm): 7.69 (s, 1H), 7.28 (d, *J* = 8.0 Hz, 1H), 7.15 (d, *J* = 8.0 Hz, 1H), 6.91 (s, 1H), 1.63-1.61(m, 1H), 0.99-0.88(m, 4H).

**XXXVI.** A preparation method for a sulfonyl compound was different from Example 1 in that C09A was replaced by a structure represented by D-47:

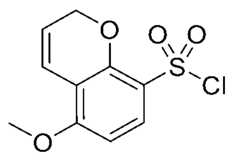

(D-47)

The synthetic route of D-47 is as follows:

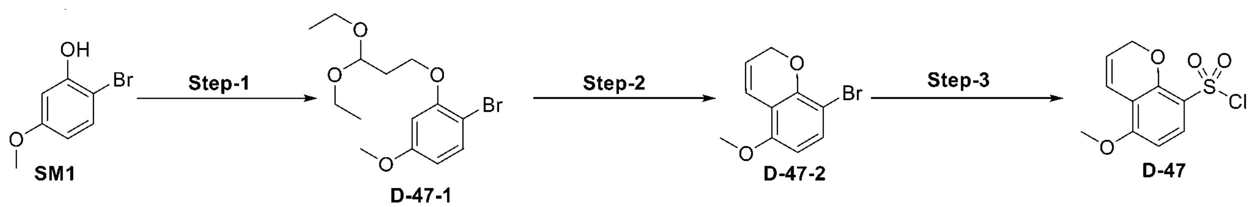


SM1 D-47-1 D-47-2 D-47

Preparation of compound D-47: The sulfonyl chloride compound D-47 was prepared according to the procedures of Step-1 to Step-3 for compound D-15.
Sulfonyl compound D-47: $^1$H NMR (400 MHz, $CDCl_3$-*d*) δ (ppm): 7.77 (d, *J* = 8.8 Hz, 1H), 6.78-6.76 (m, 1H), 6.66 (d, *J* = 8.8 Hz, 1H), 5.88-5.86 (m, 1H), 5.12 (s, 2H), 3.94 (s, 3H).

**XXXVII.** A preparation method for a sulfonyl compound was different from Example 1 in that C09A was replaced by a structure represented by D-48:

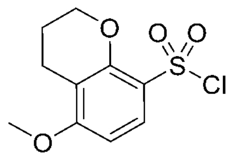

(D-48)

The synthetic route of D-48 is as follows:

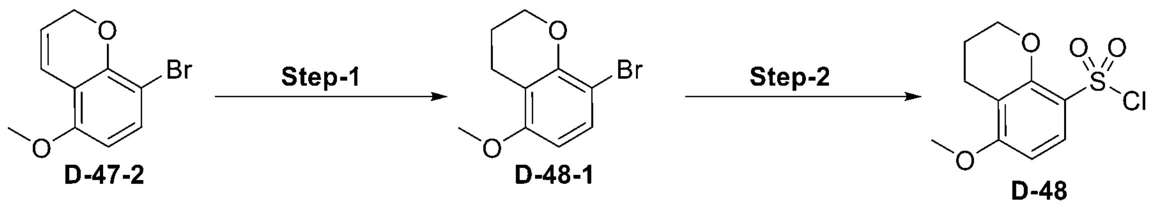


D-47-2 D-48-1 D-48

Preparation of compound D-48: The sulfonyl chloride compound D-48 was prepared according to the procedures of Step-1 to Step-2 for compound D-16.

Sulfonyl compound D-48: $^1$H NMR (400 MHz, $CDCl_3$-*d*) δ (ppm): 7.80 (d, *J* = 8.8 Hz, 1H), 6.53 (d, *J* = 8.8 Hz, 1H), 4.41 (t, *J* = 5.2 Hz, 2H), 3.94 (s, 3H), 2.71 (t, *J* = 7.2 Hz, 1H), 2.12-2.07(m, 2H).

**XXXVIII.** A preparation method for a sulfonyl compound was different from Example 1 in that C08 was replaced by a structural formula represented by I-1:

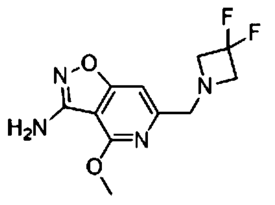

I-1

The synthetic route of compound I-1 is as follows:

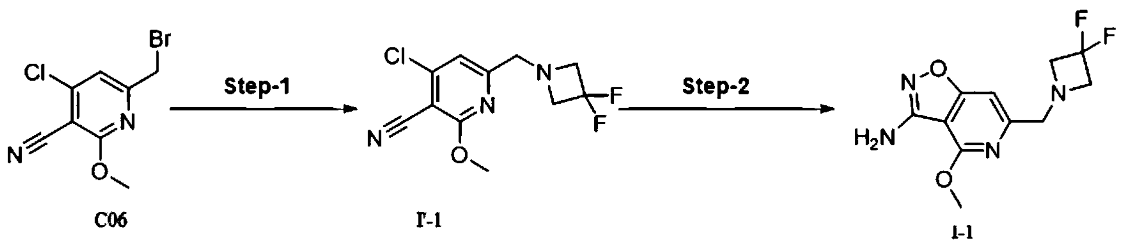


C06 I'-1 I-1

Step-1: Intermediate C06 (1.8 g, 1 eq) and 3,3-difluoroazetidine hydrochloride (1.34 g, 1.5 eq) were dissolved in dichloromethane (70 mL). A solution of N,N-diisopropylethylamine (DIEA, 2.68 g, 3 eq) in dichloromethane (10 mL) was added dropwise at 0 °C, and the mixture was stirred at 0 °C for 5 h under $N_2$ atmosphere. LCMS showed that the reaction was completed. Water was added to quench the reaction, and the mixture was extracted three times with dichloromethane. The organic phases were combined, washed with a saturated aqueous sodium chloride solution, dried over anhydrous sodium sulfate, subjected to suction filtration, and concentrated by rotary evaporation. The resulting solid was purified by normal phase column chromatography to give intermediate I'-1 (0.85 g, yield: 45.5%). $[M+1]^+$: 273.05.

Step-2: According to the procedure of Step-8 for C08, intermediate I'-1 (0.85 g, 1 eq) was reacted with

acetohydroxamic acid to give key intermediate I-1 (0.33 g, yield: 41.6%). $[M+1]^+$: 271.10.

**XXXIX.** A preparation method for a sulfonyl compound was different from Example 1 in that C08 was replaced by a structural formula represented by I-2:

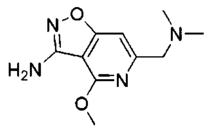

I-2

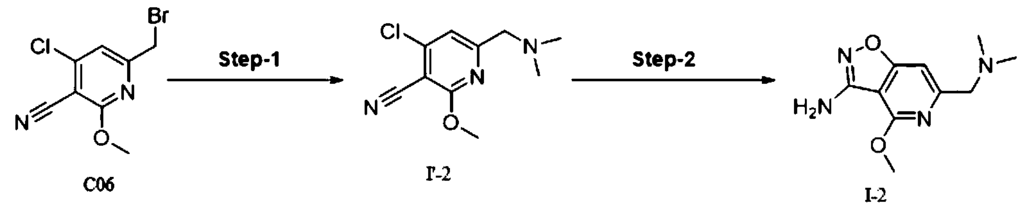


C06 I'-2 I-2

This preparation method was different from that for compound I-1 in that "3,3-difluoroazetidine hydrochloride" in Step-1 for compound I-1 was replaced by "dimethylamine" to give intermediate I-2. $[M+1]^+$: 223.10.

**XL.** A preparation method for a sulfonyl compound was different from Example 1 in that C08 was replaced by a structural formula represented by I-3:

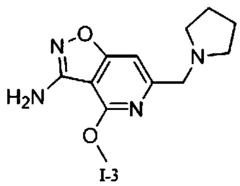

I-3

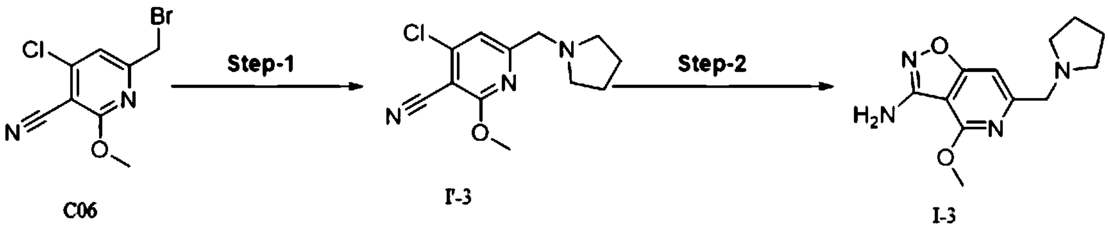


C06 I'-3 I-3

This preparation method was different from that for compound I-1 in that "3,3-difluoroazetidine hydrochloride" in Step-1 for compound I-1 was replaced by "tetrahydropyrrole" to give intermediate I-3. $[M+1]^+$: 249.10.

**XLI.** A preparation method for a sulfonyl compound, in which C08 was replaced by structural formulas represented by I-4A and 1-4B:

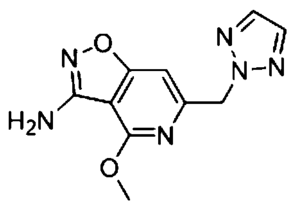

I-4A

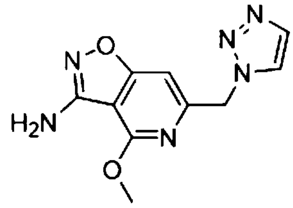

I-4B

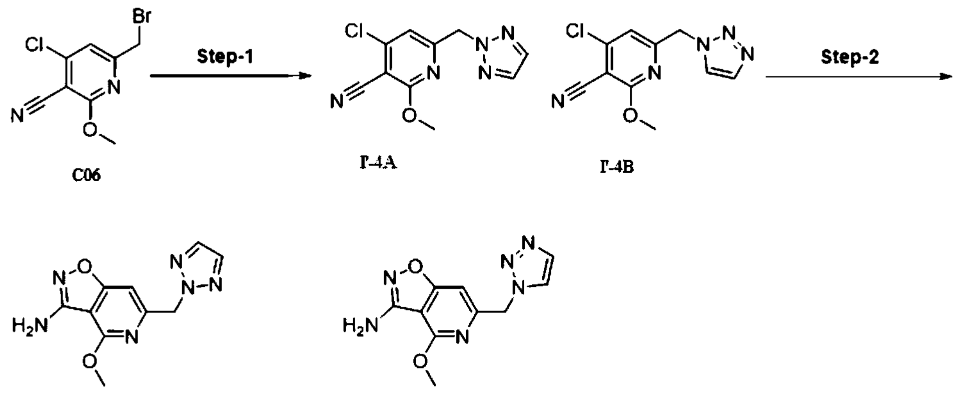


C06 I'-4A I'-4B

I-4A I-4B

This preparation method was different from that for compound I-1 in that "3,3-difluoroazetidine hydrochloride" in Step-1 for compound I-1 was replaced by "triazole" to give intermediate I-4A, $[M+1]^+$: 246.10, and intermediate 1-4B, $[M+1]^+$: 246.10.

**XLII.** A preparation method for a sulfonyl compound, in which compound C08 was replaced by a structural formula represented by I-5:

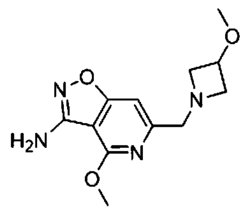

I-5

The synthetic route of compound I-5 is as follows:

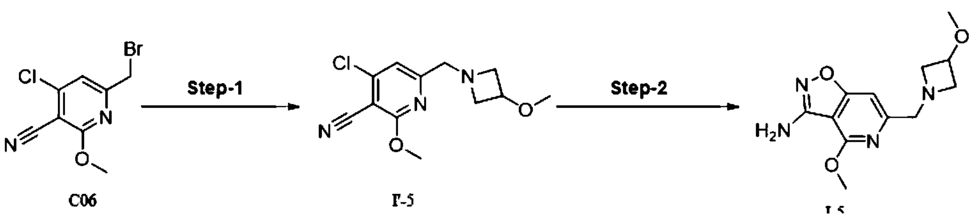


C06 I'-5 I-5

This preparation method was different from that for compound I-1 in that "3,3-difluoroazetidine hydrochloride" in Step-1 for compound I-1 was replaced by "3-methoxyazetidine hydrochloride" to give intermediate 1-5. $[M+1]^+$: 265.10.

**XLIII.** A preparation method for a sulfonyl compound was different from Example 1 in that compound C08 was replaced by structural formulas represented by I-6 and 1-7:

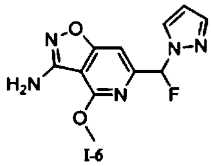
I-6

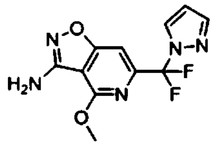
I-7

Specifically, the synthetic routes of compound I-6 and compound I-7 are as follows:

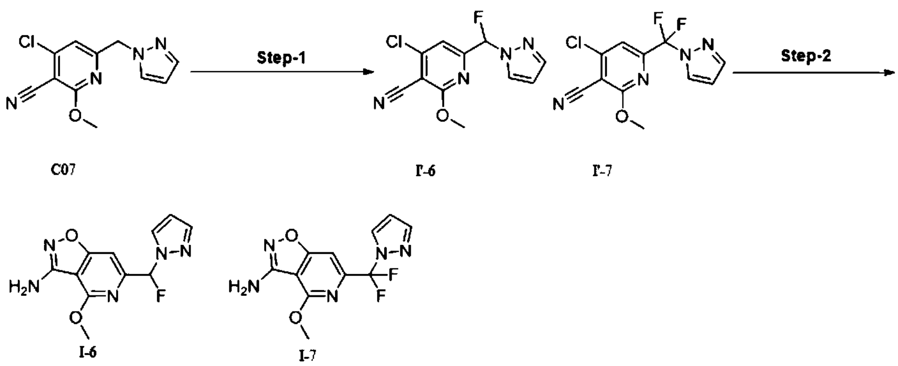

C07 I'-6 I'-7

I-6 I-7

Step-1: Intermediate C07 (1 g, 1 eq) was dissolved in freshly distilled tetrahydrofuran (50 mL). Under $N_2$ atmosphere, the mixture was cooled to -78 °C, and a solution of n-butyllithium in n-hexane (2.5 M in hexane, 4.8 mL, 3 eq) was added dropwise under stirring. The mixture was stirred at -78 °C for 5 min, and a solution of N-fluorobisbenzenesulfonamide (3.8 g, 3 eq) in tetrahydrofuran (30 mL) was added. The mixture was slowly warmed from -78 °C to room temperature under stirring overnight. LCMS showed that the reaction was completed. The reaction system was added to a saturated aqueous ammonium chloride solution to quench the reaction, and the mixture was extracted with ethyl acetate and washed with a saturated aqueous sodium chloride solution. The organic phase was dried over anhydrous sodium sulfate, subjected to suction filtration, and concentrated by rotary evaporation. The resulting crude product was purified by high-pressure preparative liquid chromatograph to give compound I'-6 (195 mg, yield: 18.1%), $[M+1]^+$: 267.10, and compound I'-7 (98 mg, yield: 8.1%), $[M+1]^+$: 285.10.

Step-2: According to the procedure of Step-8 for C08, intermediate I'-6 or intermediate I'-7 was reacted with acetohydroxamic acid to give key intermediates 1-6, $[M+1]^+$: 264.10, and I-7, $[M+1]^+$: 282.05.

I-6: $^1$H NMR (400 MHz, DMSO-$d_6$) δ (ppm): 8.03 (d, $J$ = 2.8 Hz, 1H), 7.66 (s, 1H), 7.51 (s, 1H), 7.45 (d, $J$ = 46.8 Hz, 1H), 6.42 (s, 1H), 6.30 (s, 2H), 3.90 (s, 3H).

I-7: $^1$H NMR (400 MHz, DMSO-$d_6$) δ (ppm): 8.18 (d, $J$ = 2.4 Hz, 1H), 7.75 (s, 1H), 7.71 (s, 1H), 6.52-6.51(m, 1H), 6.39 (s, 2H), 3.91 (s, 3H).

**XLIV.** A preparation method for a sulfonyl compound was different from Example 1 in that compound C08 was replaced by a structural formula represented by I-8:

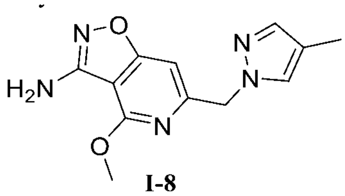
I-8

Specifically, the synthetic route of compound I-8 is as follows:

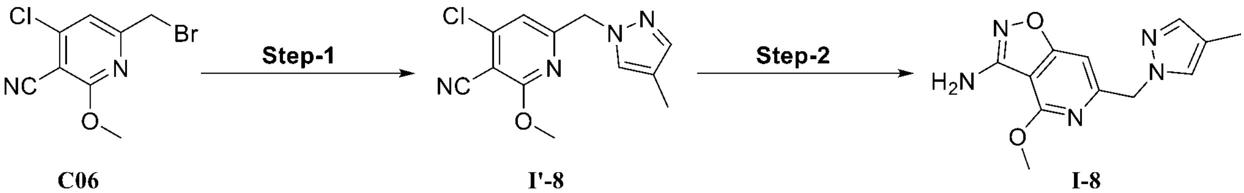

C06 I'-8 I-8

This preparation method was different from that for compound I-1 in that "3,3-difluoroazetidine hydrochloride" in Step-1 for compound I-1 was replaced by "4-methylpyrazole" to give intermediate I-8. $[M+1]^+$: 260.10.

I-8: $^1$H NMR (400 MHz, $CDCl_3$-*d*) δ (ppm): 7.39 (s, 1H), 7.33-7.29 (m, 1H), 6.49 (d, *J* = 0.8 Hz, 1H), 5.33 (s, 2H), 4.75 (s, 3H), 4.08 (s, 3H), 2.11 (s, 3H).

**XLV.** A preparation method for a sulfonyl compound was different from Example 1 in that compound C08 was replaced by a structural formula represented by I-9:

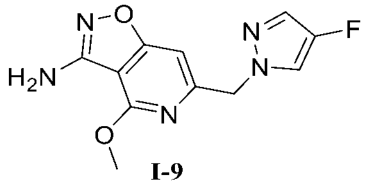

I-9

Specifically, the synthetic route of compound I-9 is as follows:

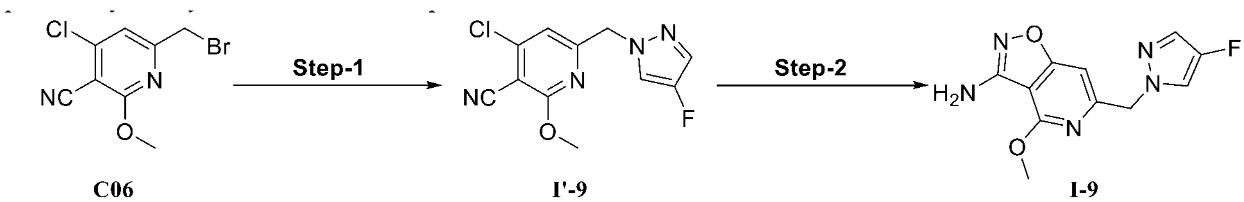

C06 I'-9 I-9

This preparation method was different from that for compound I-1 in that "3,3-difluoroazetidine hydrochloride" in Step-1 for compound I-1 was replaced by "4-fluoropyrazole" to give intermediate I-9. $[M+1]^+$: 264.15.

I-9: $^1$H NMR (400 MHz, $CDCl_3$-*d*) δ (ppm): 7.45-7.41 (m, 2H), 6.63 (s, 1H), 5.28 (s, 2H), 4.67 (s, 2H), 4.07 (s, 3H).

**XLVI.** A preparation method for a sulfonyl compound was different from Example 1 in that compound C08 was replaced by a structural formula represented by I-10:

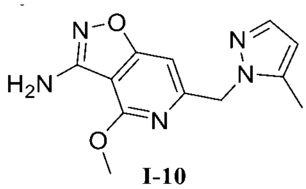

I-10

Specifically, the synthetic route of compound I-10 is as follows:

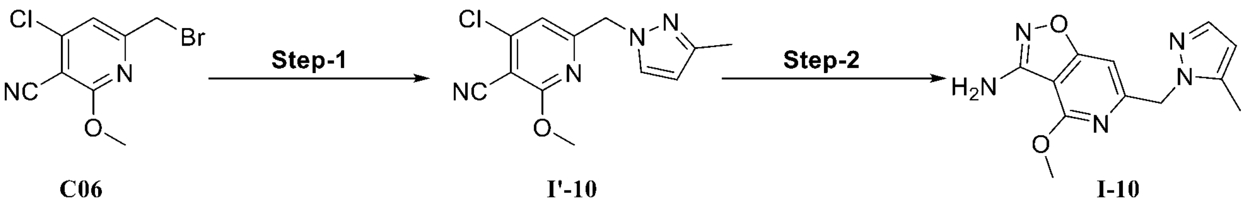

C06 I'-10 I-10

This preparation method was different from that for compound I-1 in that "3,3-difluoroazetidine hydrochloride" in Step-1 for compound I-1 was replaced by "3-methylpyrazole" to give intermediate I-10. [M+1]$^+$: 260.10.

I-10: $^1$H NMR (400 MHz, $CDCl_3$-*d*) δ (ppm): 7.45 (d, *J* = 2.0 Hz, 1H), 6.52 (d, *J* = 1.6 Hz, 1H), 6.11 (s, 1H), 5.32 (s, 2H), 4.67 (s, 2H), 4.07 (s, 3H), 2.31 (s, 3H).

Example 2:

Compound 1-2: *N*-(6-((1H-pyrazol-1-yl)methyl)-4-methoxyisoxazolo[4,5-c]pyridin-3-yl)-3-methoxy-5,6,7,8-tetrahydronaphthalene-2-sulfonamide;

Compound 1-3: *N*-(6-((1H-pyrazol-1-yl)methyl)-4-methoxyisoxazolo[4,5-c]pyridin-3-yl)-1-methoxy-5,6,7,8-tetrahydronaphthalene-2-sulfonamide;

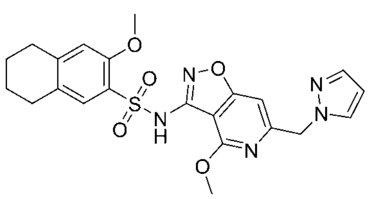

1-2

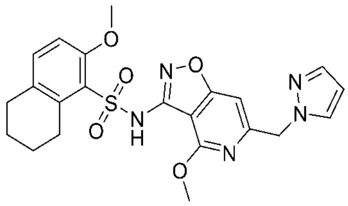

1-3

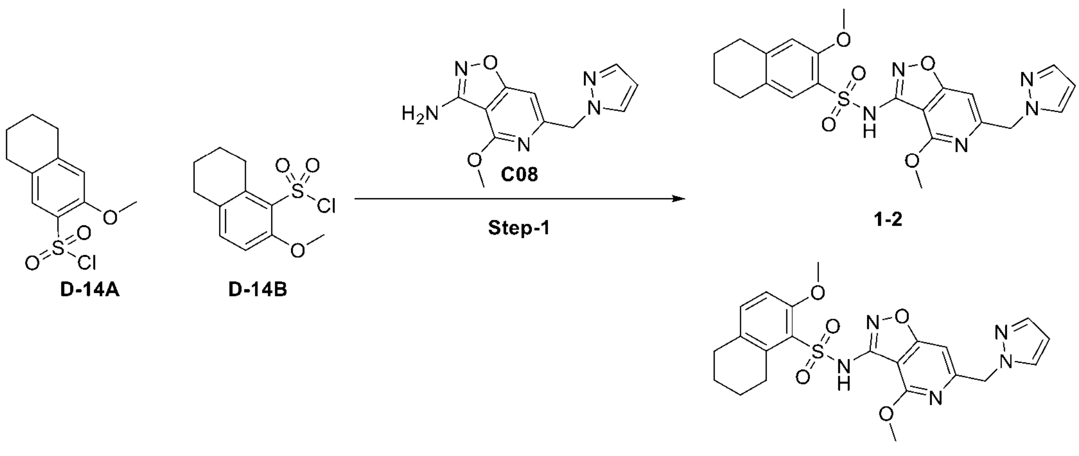


C08

D-14A

D-14B

1-2

1-3

Step-1: Preparation of compounds 1-2 and 1-3: According to the procedure of Step-1 for compound 1-1, the key intermediate C08 and a mixture of D-14A and D-14B were subjected to a condensation reaction, and the reaction mixture was purified by high-pressure preparative liquid chromatography to give compounds 1-2 and 1-3. Compound 1-2: [M+1]$^+$: 470.15. Compound 1-3: [M+1]$^+$: 470.00.

Compound 1-2: $^1$H NMR (400 MHz, DMSO-$d_6$) δ (ppm):10.61 (s, 1H), 7.90 (s, 1H), 7.50 (d, J = 10.8 Hz, 2H), 6.86 (s, 1H), 6.70 (s, 1H), 6.33 (s, 1H), 5.45 (s, 2H), 3.95 (s, 3H), 3.72 (s, 3H), 2.75-2.68 (m, 4H), 1.72 (s, 4H). Compound 1-3: $^1$H NMR (400 MHz, DMSO-$d_6$) δ (ppm): 10.25 (s, 1H), 7.90 (s, 1H), 7.52 (s, 1H), 7.29 (s, 1H), 6.99 (s, 1H), 6.72 (s, 1H), 6.33 (s, 1H),5.44 (s, 2H), 3.95 (s, 3H), 3.76 (s, 3H), 3.11 (s, 2H), 2.72 -2.68 (m, 2H), 1.68 - 1.64 (m, 4H).

According to preparation methods similar to those in Examples 1-2, the sulfonyl compounds in Examples 1-97 below were prepared by reacting the intermediates of the D series with C08, C09, or the intermediates of the I series, as shown in Table 2:

Table 2. Sulfonyl compounds

| Example | Structure/Compound No. | [M+1]$^+$ |
|---|---|---|
| 1 | *N*-(6-((1H-Pyrazol-1-yl)methyl)-4-methoxyisoxazolo[4,5-c]pyridin-3-yl)-2,3-dihydrobenzofuran-7-sulfonamide | 427.95 |

(continued)

| Example | Structure/Compound No. | [M+1]$^+$ |
|---|---|---|
| | 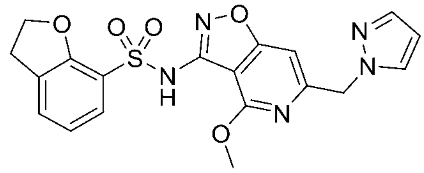 **1-1** | |
| | $^{1}$H NMR (400 MHz, DMSO-$d_6$) δ (ppm): 11.05(s, 1H), 7.89(s, 1H), 7.78-7.69(m, 2H), 7.52(s, 1H), 6.86(s, 1H), 6.62(s, 1H), 6.33(s, 1H), 5.43(s, 2H), 4.64-4.59 (m, 2H), 3.98(s, 3H), 3.25-3.20(m, 2H). | |
| 2 | *N*-(6-((1H-Pyrazol-1-yl)methyl)-4-methoxyisoxazolo[4,5-c]pyridin-3-yl)-3-methoxy-5,6,7,8-tetrahydronaphthalene-2-sulfonamide | 470.15 |
| | 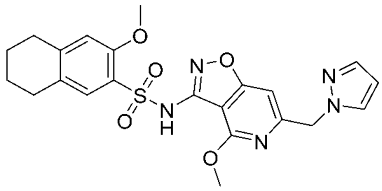 **1-2** | |
| | $^{1}$H NMR (400 MHz, DMSO-$d_6$) δ (ppm): 10.61(s, 1H), 7.90(s, 1H), 7.52-7.49(m, 2H), 6.86(s, 1H), 6.70(s, 1H), 6.33(s, 1H), 5.45(s, 2H), 3.95(s, 3H), 3.72(s, 3H), 2.75-2.68(m, 4H), 1.72-1.70(m, 4H). | |
| 3 | *N*-(6-((1H-Pyrazol-1-yl)methyl)-4-methoxyisoxazolo[4,5-c]pyridin-3-yl)-1-methoxy-5,6,7,8-tetrahydronaphthalene-2-sulfonamide | 470.00 |
| | 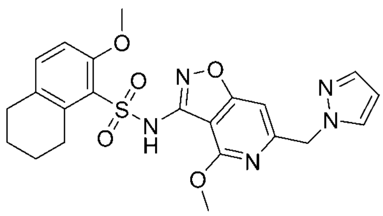 **1-3** | |
| | $^{1}$H NMR (400 MHz, DMSO-$d_6$) δ (ppm): 10.24 (s, 1H), 7.89(s, 1H), 7.51(s, 1H), 7.29(s, 1H), 6.99(s, 1H), 6.72(s, 1H), 6.33(s, 1H),5.44(s, 2H), 3.95(s, 3H), 3.76(s, 3H), 3.11(s, 2H), 2.72-2.68(m, 2H), 1.67-1.66(m, 4H). | |
| 4 | *N*-(6-((1H-Pyrazol-1-yl)methyl)-4-methoxyisoxazolo[4,5-c]pyridin-3-yl)-2,3-dihydrobenzo[b][1,4]dioxine-5-sulfonamide | 443.95 |
| | 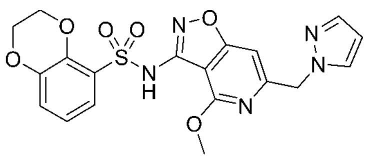 **1-4** | |
| | $^{1}$H NMR (400 MHz, DMSO-$d_6$) δ (ppm): 10.92 (s, 1H), 7.89 (d, *J* = 2.4 Hz, 1H), 7.51 (d, *J* = 1.6 Hz, 1H), 7.34-7.32(m, 1H), 7.09(s, 1H), 6.96 (s, 1H), 6.66 (s, 1H), 6.33 (s, 1H), 5.44(s, 2H), 4.22-4.19(m, 4H), 3.931(s, 3H). | |
| 5 | *N*-(6-((1H-Pyrazol-1-yl)methyl)-4-methoxyisoxazolo[4,5-c]pyridin-3-yl)benzofuran-7-sulfonamide | 426.05 |
| | 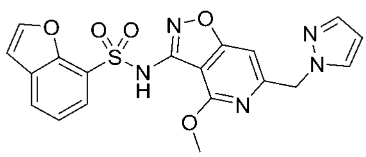 **1-5** | |

(continued)

| Example | Structure/Compound No. | [M+1]+ |
|---|---|---|
| | $^1$H NMR (400 MHz, DMSO-$d_6$) δ (ppm): 7.87(d, *J* = 2.0 Hz, 1H), 7.72-7.71(m, 1H), 7.60(d, *J* = 8.4 Hz, 1H), 7.50 (d, *J* = 1.6 Hz, 1H), 7.42-7.38(m, 1H), 7.32-7.28(m, 2H), 6.51(s, 1H),6.31(s, 1H), 5.40(s, 2H), 3.89 (s, 3H). | |
| 6 | *N*-(6-((1H-Pyrazol-1-yl)methyl)-4-methoxyisoxazolo[4,5-c]pyridin-3-yl)-1-methyl-1H-indole-4-sulfonamide | 439.15 |
| | 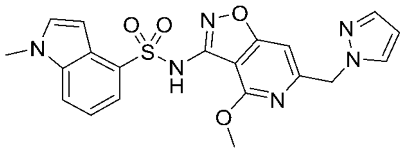 **1-6** | |
| | $^1$H NMR (400 MHz, DMSO- $d_6$) δ (ppm): 11.25 (s, 1H), 7.86 (s, 1H), 7.58-7.49(m, 2H), 7.34(s, 1H), 7.18-7.176(m, 1H), 7.15(s, 1H), 6.88 (s, 1H), 6.43(s, 1H), 6.31(s, 1H), 5.37(s, 2H), 3.94(s, 3H), 3.78(s, 3H). | |
| 7 | *N*-(6-((1H-Pyrazol-1-yl)methyl)-4-methoxyisoxazolo[4,5-c]pyridin-3-yl)-3,4-dihydro-1H-benzopyran-8-sulfonamide | 442.15 |
| | 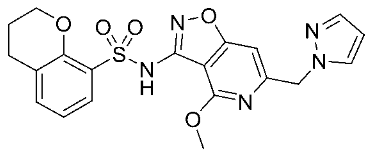 **1-7** | |
| | $^1$H NMR (400 MHz, DMSO-$d_6$) δ (ppm): 10.61(s, 1H), 7.89 (d, *J* = 2.4 Hz, 1H), 7.58 (d, *J* = 8.0 Hz, 1H), 7.51 (d, *J* = 1.6 Hz, 1H), 7.26(s, 1H), 6.97 (s, 1H), 6.63(s, 1H), 6.33 (s, 1H), 5.43(s, 2H), 4.10-4.09(m, 2H), 4.074(s, 3H), 2.76-7.74(m, 2H), 1.86-1.84(m, 2H). | |
| 8 | *N*-(6-((1H-Pyrazol-1-yl)methyl)-4-methoxyisothiazolo[4,5-c]pyridin-3-yl)-6-methoxy-2,3-dihvdrobenzofuran-7-sulfonamide | 474.10 |
| | 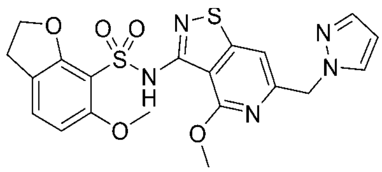 **1-8** | |
| 9 | *N*-(6-((1H-Pyrazol-1-yl)methyl)-4-methoxyisoxazolo[4,5-c]pyridin-3-yl)-6-methoxy-2,3-dihydrobenzofuran-7-sulfonamide | 458.10 |
| | 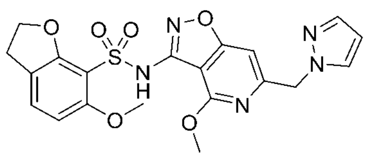 **1-9** | |
| | $^1$H NMR (400 MHz, DMSO-$d_6$) δ (ppm): 7.89 (s, 1H), 7.51 (s, 1H), 7.09 -7.07 (m, 2H), 6.37 (s,1H), 6.32 (s,1H), 5.34(s,2H), 4.40-4.36(m, 2H), 3.94 (s, 3H), 3.57(s, 3H), 3.03-2.99(m, 2H). | |
| 10 | *N*-(6-((1H-Pyrazol-1-yl)methyl)-4-methoxyisoxazolo[4,5-c]pyridin-3-yl)-6-methoxybenzofuran-7-sulfonamide | 456.10 |
| | 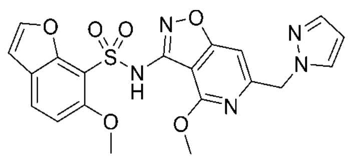 **1-10** | |

(continued)

| Example | Structure/Compound No. | [M+1]+ |
|---|---|---|
| | $^1H$ NMR (400 MHz, DMSO-$d_6$) δ (ppm): 11.09 (s, 1H), 7.95 (s, 1H), 7.89 (s, 1H), 7.81 (s, 1H), 7.51 (s, 1H), 7.24 (s, 1H), 7.12-7.11 (d, *J* =6 Hz, 1H), 6.54 (s, 1H), 6.32 (s, 1H), 5.43 (s, 2H), 3.87 (s, 3H), 3.79 (s, 3H). | |
| 11 | *N*-(6-((1H-Pyrazol-1-yl)methyl)-4-methoxyisoxazolo[4,5-c]pyridin-3-yl)-7-methoxyquinoline-8-sulfonamide | 467.10 |
| | 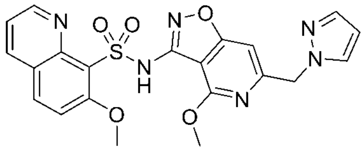 **1-11** | |
| | $^1H$ NMR (400 MHz, DMSO-$d_6$) δ (ppm): 11.85(s. 1H), 9.04(s, 1H), 8.58 (s, 1H), 8.33 (s, 1H), 7.87(s, 1H), 7.78 (s, 1H), 7.62 (s, 1H), 7.49(s, 1H), 6.59 (s, 1H), 6.31 (s, 1H), 5.40 (s, 2H), 4.00(s, 6H). | |
| 12 | *N*-(6-((2H-1,2,3-Triazol-2-yl)methyl)-4-methoxyisoxazolo[4,5-c]pyridin-3-yl)-2,3-dihydrobenzofuran-7-sulfonamide | 429.10 |
| | 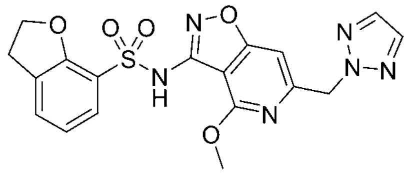 **1-12** | |
| | $^1H$ NMR (400 MHz, DMSO-$d_6$) δ (ppm): 11.07 (s, 1H), 7.90 (s, 2H), 7.82 (s, 1H), 7.76 -7.74 (m, 1H), 6.93-6.91 (d, *J* =8.4 Hz, 1H), 6.87 (s, 1H), 5.77 (s, 2H), 4.66-4.55 (m, 2H), 3.93 (s, 3H), 3.26-3.16 (m, 2H) | |
| 13 | *N*-(4-Methoxy-6-(tetrahydropyrrol-1-ylmethyl)isoxazolo[4,5-c]pyridin-3-yl)-2,3-dihydrobenzofuran-7-sulfonamide | 431.10 |
| | 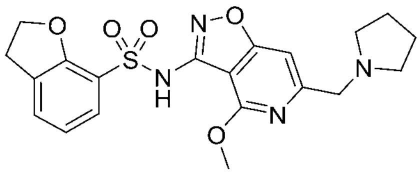 **1-13** | |
| | $^1H$ NMR (400 MHz, DMSO-$d_6$) δ (ppm): 9.78 (s, 1H), 7.68 (s, 1H), 7.60-7.58 (d, J=8.4Hz, 1H), 7.07 (s, 1H), 6.72-6.70 (m, 1H), 4.57-4.52 (m, 2H), 4.30 (s, 2H), 4.02 (s, 3H), 3.20-3.15 (m, 4H), 2.39 (s, 2H), 1.92 (s, 4H). | |
| 14 | *N*-(6-((3,3-Difluoroazetidin-1-yl)methyl)-4-methoxyisoxazolo[4,5-c]pyridin-3-yl))-2,3-dihydrobenzofuran-7-sulfonamide | 453.10 |
| | 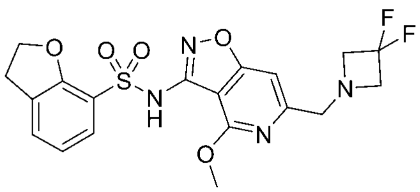 **1-14** | |
| | *N*-(4-Methoxy-6-((3-methoxyazetidin-1-yl)methyl)isoxazolo[4,5-c]pyridin-3-yl)-2,3-benzofuran-7-sulfonamide | |

(continued)

| Example | Structure/Compound No. | [M+1]+ |
|---|---|---|
| 15 | 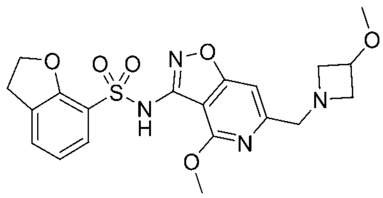 **1-15** | 447.10 |
| | $^1$H NMR (400 MHz, DMSO-$d_6$) δ (ppm): 10.33-9.92 (m, 1H), 7.66 (s, 1H), 7.60-7.58 (m, 1H), 7.00 (s, 1H), 6.72-6.70 (m, 1H), 4.56-4.52 (m, 2H), 4.15-3.97 (m, 8H), 3.62-3.61 (d, *J* = 3.2 Hz, 2H), 3.21-3.15 (m, 5H). | |
| 16 | *N*-(6-((1H-1,2,3-Triazol-1-yl)methyl)-4-methoxyisoxazolo[4,5-c]pyridin-3-yl)-2,3-dihydrobenzofuran-7-sulfonamide | 429.09 |
| | 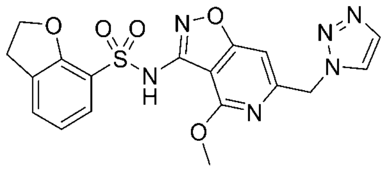 **1-16** | |
| | $^1$H NMR (400 MHz, DMSO-$d_6$) δ (ppm): 11.09 (s, 1H), 8.24 (s, 1H), 7.79-7.69 (m, 2H), 7.22-7.10 (m, 1H), 7.00 (s, 1H), 6.84-6.83 (m, 1H), 5.71 (s, 2H), 4.63-4.59 (m, 2H), 3.93 (s, 3H), 3.24-3.20 (m, 2H) | |
| 17 | *N*-(6-(Difluoro(1H-pyrazol-1-yl)methyl)-4-methoxyisoxazolo[4,5-c]pyridin-3-yl)-2,3-dihydrobenzofuran-7-sulfonamide | 464.10 |
| | 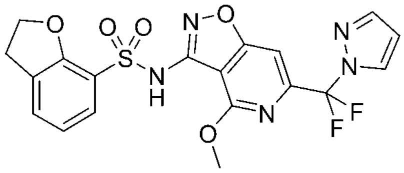 **1-17** | |
| 18 | *N*-(6-(Fluoro(1H-pyrazol-1-yl)methyl)-4-methoxyisoxazolo[4,5-c]pyridin-3-yl)-2,3-dihydrobenzofuran-7-sulfonamide | 446.10 |
| | 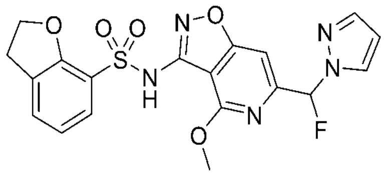 **1-18** | |
| 19 | *N*-(6-((Dimethylamino)methyl)-4-methoxyisoxazolo[4,5-c]pyridin-3-yl)-2,3-dihydrobenzofuran-7-sulfonamide | 405.1 |
| | 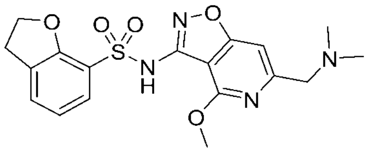 **1-19** | |
| | $^1$H NMR (400 MHz, DMSO-$d_6$) δ (ppm): 9.58 (s, 1H), 7.70 (s, 1H), 7.60-7.58 (d, *J* =8.0 Hz, 1H), 7.03 (s, 1H), 6.72-6.70 (s, 1H), 4.56-4.52 (m, 2H), 4.00 (s, 1H), 3.20-3.15 (m, 2H), 3.00 (m, 6H). | |
| | *N*-(6-(Azetidin-1-ylmethyl)-4-methoxyisoxazolo[4,5-c]pyridin-3-yl)-2,3-dihydrobenzofuran-7-sulfonamide | |

(continued)

| Example | Structure/Compound No. | [M+1]+ |
|---|---|---|
| 20 | 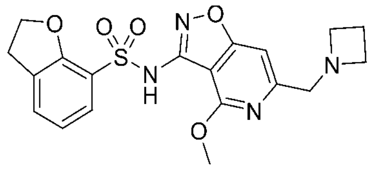 1-20 | 417.12 |
| | $^1H$ NMR (400 MHz, DMSO-$d_6$) δ (ppm): 7.66 (s, 1H), 7.59-7.57 (m, 2H), 6.71-6.68 (s, 1H), 4.53 (s, 2H), 4.26 (s, 2H), 3.99 (s, 6H), 3.16 (s, 4H), 1.24 (s, 3H). | |
| 21 | *N*-(6-((2H-1,2,3-Triazol-2-yl)methyl)-4-methoxyisoxazolo[4,5-c]pyridin-3-yl)-1-methyl-1H-indole-4-sulfonamide | 440.10 |
| | 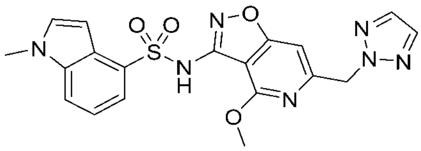 1-21 | |
| 22 | *N*-(4-Methoxy-6-(tetrahydropyrrol-1-ylmethyl)isoxazolo[4,5-c]pyridin-3-yl)-1-methyl-1H-indole-4-sulfonamide | 442.10 |
| | 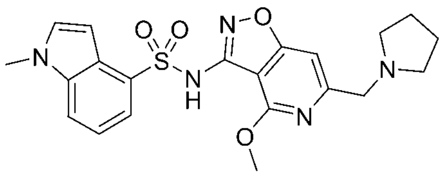 1-22 | |
| 23 | *N*-(6-((3,3-Difluoroazetidin-1-yl)methyl)-4-methoxyisoxazolo[4,5-c]pyridin-3-yl)-1-methyl-1H-indole-4-sulfonamide | 464.10 |
| | 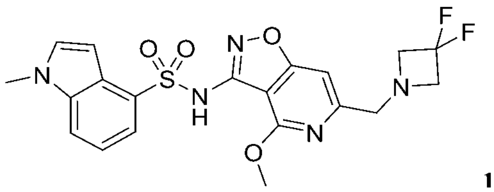 1-23 | |
| 24 | *N*-(4-Methoxy-6-((3-methoxyazetidin-1-yl)methyl)isoxazolo[4,5-c]pyridin-3-yl)-1-methyl-1H-indole-4-sulfonamide | 458.10 |
| | 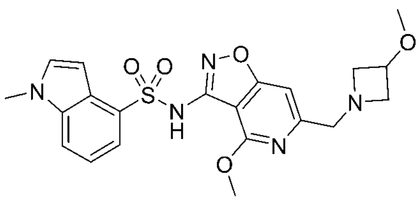 1-24 | |
| 25 | *N*-(6-((Dimethylamino)methyl)-4-methoxyisoxazolo[4,5-c]pyridin-3-yl)-1-methyl-1H-indole-4-sulfonamide | 416.09 |
| | 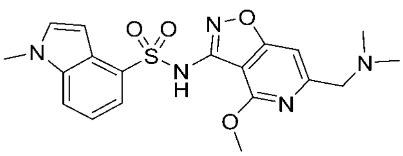 1-25 | |
| | *N*-(6-((1H-Pyrazol-1-yl)methyl)-4-methoxyisoxazolo[4,5-c]pyridin-3-yl)-2,3-dihydro-1H-indene-4-sulfonamide | |

(continued)

| Example | Structure/Compound No. | $[M+1]^+$ |
|---|---|---|
| 26 | 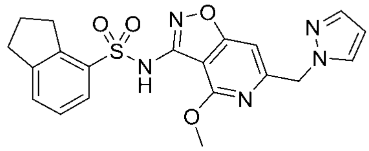 1-26 | 426.10 |
| 27 | *N*-(4-Methoxy-6-(methoxymethyl)isoxazolo[4,5-c]pyridin-3-yl)-2,3-dihydrobenzofuran-7-sulfonamide | 392.09 |
| | 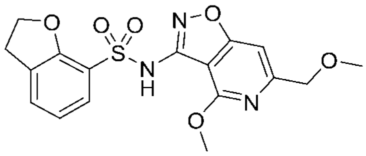 1-27 | |
| 28 | *N*-(6-((1H-Pyrazol-1-yl)methyl)-4-methoxyisoxazolo[4,5-c]pyridin-3-yl)-1,3-benzodioxole-4-sulfonamide | 430.1 |
| | 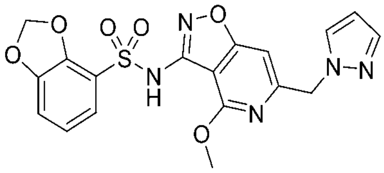 1-28 | |
| | $^1$H NMR (400 MHz, DMSO-$d_6$) δ (ppm): 7.86 (d, *J* = 2.0Hz, 1H), 7.53 (d, *J* = 2.0Hz, 1H), 7.25(s, 1H), 7.22-7.18 (m, 1H), 7.12 (s, 1H), 7.00-6.97 (m, 2H), 6.84 (t, *J* = 8.0Hz, 1H), 6.51(s, 1H), 6.32 (t, *J* = 2.0Hz, 1H), 6.03 (s, 2H), 5.40 (s, 2H), 3.94 (s, 3H). | |
| 29 | *N*-(6-((2H-1,2,3-Triazol-2-yl)methyl)-4-methoxyisoxazolo[4,5-c]pyridin-3-yl)-2,3-dihydrobenzo[b][1,4]dioxine-5-sulfonamide | 445.10 |
| | 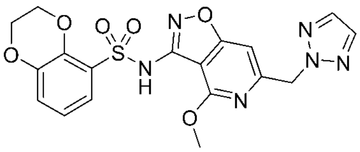 1-29 | |
| 30 | *N*-(6-Benzyl-4-methoxyisoxazolo[4,5-c]pyridin-3-yl)-2,3-dihydrobenzo[b][1,4]dioxine-5-sulfonamide | 454.10 |
| | 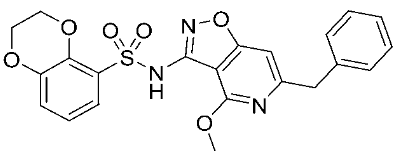 1-30 | |
| 31 | *N*-(4-Methoxy-6-(oxazol-4-ylmethyl)isoxazolo[4,5-c]pyridin-3-yl)-2,3-dihydrobenzo[b][1,4]dioxine-5-sulfonamide | 445.10 |
| | 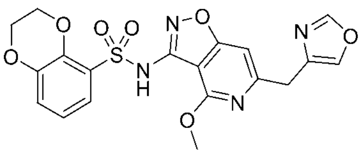 1-31 | |
| | *N*-(4-Methoxy-6-(pyridin-2-ylmethyl)isoxazolo[4,5-c]pyridin-3-yl)-2,3-dihydrobenzo[b][1,4]dioxine-5-sulfonamide | |

(continued)

| Example | Structure/Compound No. | $[M+1]^+$ |
|---|---|---|
| 32 | 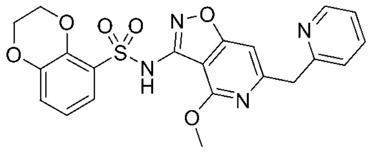<br>1-32 | 455.10 |
| 33 | *N*-(6-((1H-Pyrazol-1-yl)methyl)-4-methoxyisoxazolo[4,5-c]pyridin-3-yl)-4-ethynyl-2,3-dihydrobenzofuran-7-sulfonamide<br>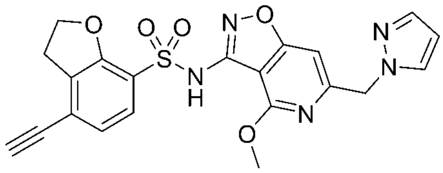<br>1-33 | 452.10 |
| 34 | *N*-(6-((2H-1,2,3-Triazol-2-yl)methyl)-4-methoxyisoxazolo[4,5-c]pyridin-3-yl)-3-methoxy-5,6,7,8-tetrahydronaphthalene-2-sulfonamide<br>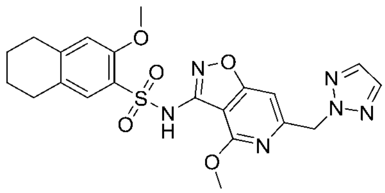<br>1-34 | 471.15 |
| 35 | 3-Methoxy-*N*-(4-methoxy-6-(tetrahydropyrrol-1-ylmethyl)isoxazolo[4,5-c]pyridin-3-yl)-5,6,7,8-tetrahydronaphthalene-2-sulfonamide<br>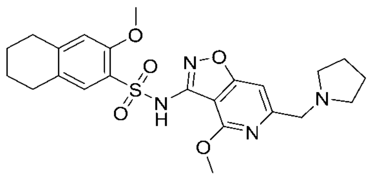<br>1-35 | 473.20 |
| 36 | 1-Methoxy-*N*-(4-methoxy-6-(tetrahydropyrrol-1-ylmethyl)isoxazolo[4,5-c]pyridin-3-yl)-5,6,7,8-tetrahydronaphthalene-2-sulfonamide<br>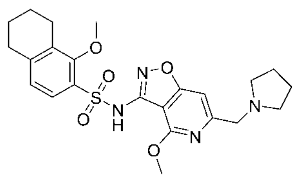<br>1-36 | 473.15 |
| 37 | *N*-(6-(azetidin-1-ylmethyl)-4-methoxyisoxazolo[4,5-c]pyridin-3-yl)-3-methoxy-5,6,7,8-tetrahydronaphthalene-2-sulfonamide<br>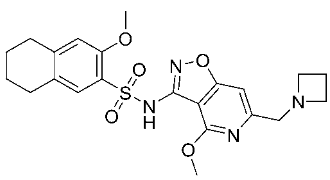<br>1-37 | 459.10 |
| | *N*-(6-(Dimethylamino)methyl)-4-methoxyisoxazolo[4,5-c]pyridin-3-yl)-3-methoxy-5,6,7,8-tetrahydronaphthalene-2-sulfonamide | |

(continued)

| Example | Structure/Compound No. | [M+1]+ |
|---|---|---|
| 38 | 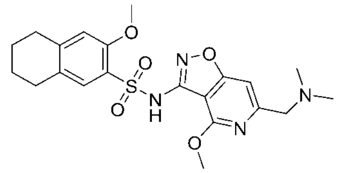 1-38 | 447.10 |
| 39 | 3-Methoxy-*N*-(4-methoxy-6-(methoxymethyl)isoxazolo[4,5-c]pyridin-3-yl)-5,6,7,8-tetrahydronaphthalene-2-sulfonamide | 434.10 |
| | 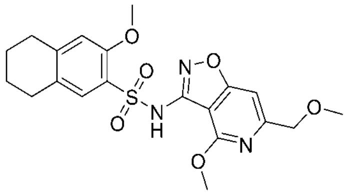 1-39 | |
| 40 | 1-Methoxy-*N*-(4-methoxy-6-(methoxymethyl)isoxazolo[4,5-c]pyridin-3-yl)-5,6,7,8-tetrahydronaphthalene-2-sulfonamide | 434.10 |
| | 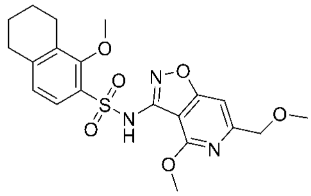 1-40 | |
| 41 | *N*-(6-((3,3-Difluoroazetidin-1-yl)methyl)-4-methoxyisoxazolo[4,5-c]pyridin-3-yl)-3-methoxy-5,6,7,8-tetrahydronaphthalene-2-sulfonamide | 495.10 |
| | 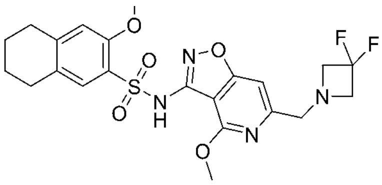 1-41 | |
| 42 | *N*-(6-((3,3-Difluoroazetidin-1-yl)methyl)-4-methoxyisoxazolo[4,5-c]pyridin-3-yl)-1-methoxy-5,6,7,8-tetrahydronaphthalene-2-sulfonamide | 495.00 |
| | 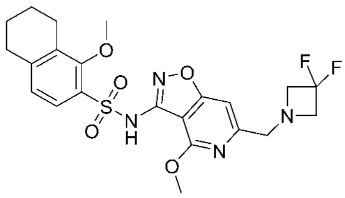 1-42 | |
| 43 | 3-Methoxy-*N*-(4-methoxy-6-((3-methoxyazetidin-1-yl)methyl)isoxazolo[4,5-c]pyridin-3-yl)-5,6,7,8-tetrahydronaphthalene-2-sulfonamide | 489.10 |
| | 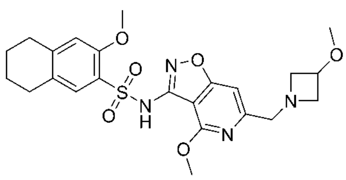 1-43 | |
| | *N*-(6-((1H-Pyrazol-1-yl)methyl)-4-methoxyisoxazolo[4,5-c]pyridin-3-yl)-5-methoxybenzofuran-6-sulfonamide | |

(continued)

| Example | Structure/Compound No. | [M+1]+ |
|---|---|---|
| 44 | 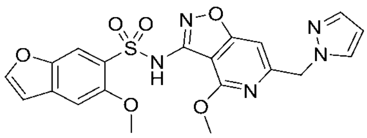 **1-44** | 456.10 |
| | $^1$H NMR (400 MHz, $CDCl_3$-*d*) δ (ppm): 8.00 (s, 1H), 7.67 (d, *J* = 2.4 Hz, 1H), 7.57 (dd, *J* = 9.2, 1.2 Hz, 1H), 7.53 (d, *J* = 2.0 Hz, 1H), 7.49 (d, *J* = 2.4 Hz, 1H), 7.46-7.45 (m, 1H), 6.85 (d, *J* = 9.2 Hz, 1H), 6.48 (s, 1H), 6.28 (t, *J* = 2.0 Hz, 1H), 5.36 (s, 2H), 4.06 (s, 3H), 3.90 (s, 3H). | |
| 45 | *N*-(6-((1H-Pyrazol-1-yl)methyl)-4-methoxyisoxazolo[4,5-c]pyridin-3-yl)-5-methoxybenzofuran-4-sulfonamide | 456.10 |
| | 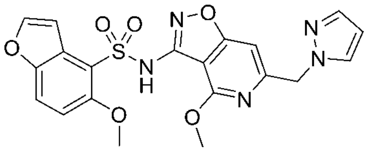 **1-45** | |
| | $^1$H NMR (400 MHz, $CDCl_3$-*d*) δ (ppm): 8.30 (d, *J* = 1.2 Hz, 1H), 7.97 (s, 1H), 7.77 (d, *J* = 2.4 Hz, 1H), 7.65 - 7.58 (m, 2H), 7.10 (s, 1H), 6.62 (s, 1H), 6.38 (s, 1H), 5.47 (s, 2H), 4.13 (s, 3H), 3.96 (s, 3H) | |
| 46 | *N*-(6-(((1H-Pyrazol-1-yl)methyl)-4-methoxyisoxazolo[4,5-c]pyridin-3-yl)quinoline-8-sulfonamide | 437.10 |
| | 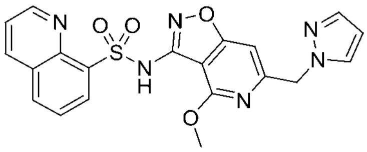 **1-46** | |
| | $^1$H NMR (400 MHz, DMSO-$d_6$) δ (ppm): 9.01 (s, 1H), 8.53 (s, 1H), 8.40 (d, *J* =7.2Hz, 1H), 8.291 (s, 1H), 7.86 (d, *J* =2.0Hz, 1H), 7.85-7.68 (m, 2H), 7.49 (d, *J* =2.0Hz, 1H), 6.62 (s, 1H), 6.30 (s, 1H), 5.40 (s, 2H), 3.98 (s, 3H). | |
| 47 | *N*-(6-((1H-Pyrazol-1-yl)methyl)-4-methoxyisoxazolo[4,5-c]pyridin-3-yl)benzofuran-4-sulfonamide | 426.05 |
| | 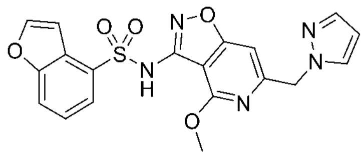 **1-47** | |
| 48 | *N*-(6-((1H-Pyrazol-1-yl)methyl)-4-methoxyisoxazolo[4,5-c]pyridin-3-yl)-2-methylbenzo[d]oxazole-7-sulfonamide | 441.00 |
| | 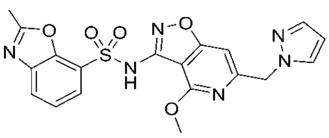 **1-48** | |
| | $^1$H NMR (400 MHz, DMSO-$d_6$) δ (ppm): 7.87 (s, 1H), 7.69-7.63 (m, 1H), 7.50 (s, 1H), 7.31-7.28 (m, 2H), 7.24-7.22 (m, 1H), 6.33-6.31 (m, 1H), 5.37 (s, 2H), 3.95 (m, 3H), 2.60 (s, 3H). | |
| | *N*-(6-((1H-Pyrazol-1-yl)methyl)-4-methoxyisoxazolo[4,5-c]pyridin-3-yl)-1-methyl-1H-indole-7-sulfonamide | |

(continued)

| Example | Structure/Compound No. | [M+1]+ |
|---|---|---|
| 49 | 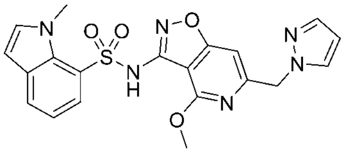 **1-49** | 439.09 |
| | $^1$H NMR (400 MHz, DMSO-$d_6$) δ (ppm): 7.87 (s, 1H), 7.86-7.70 (m, 1H), 7.33 (s, 1H), 7.22 (s, 1H), 7.09 (s, 1H), 7.05-7.02 (m, 1H), 7.00 (s, 1H), 6.54-6.53 (m, 1H), 6.31-6.30 (m, 1H), 5.40 (s, 2H), 4.28 (s, 3H), 3.92 (s, 3H). | |
| 50 | *N*-(6-((1H-Pyrazol-1-yl)methyl)-4-methoxyisoxazolo[4,5-c]pyridin-3-yl)-3,4-dihydro-1H-benzopyran-6-sulfonamide | 442.05 |
| | 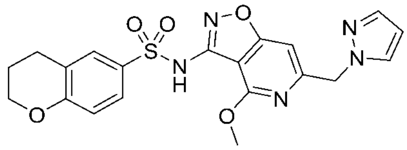 **1-50** | |
| | $^1$H NMR (400 MHz, DMSO-$d_6$) δ (ppm): 11.05 (s, 1H), 7.90-7.88 (m, 1H), 7.63-7.61 (m, 2H), 7.51 (s, 1H), 6.83-6.81 (d, *J* = 8.4 Hz, 1H), 6.59 (s, 1H), 6.32 (s, 1H), 5.42 (s, 2H), 4.19-4.18 (m, 2H), 4.00 (s, 3H), 2.78-2.75 (m, 2H), 1.94 -1.90 (m, 2H). | |
| 51 | *N*-(6-((1H-Pyrazol-1-yl)methyl)-4-methoxyisoxazolo[4,5-c]pyridin-3-yl)-2,2-difluorobenzodioxole-4-sulfonamide | 466.04 |
| | 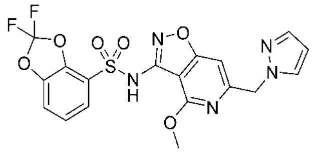 **1-51** | |
| | $^1$H NMR (400 MHz, DMSO-$d_6$) δ (ppm): 7.90(s, 1H), 7.55-7.51(m, 3H), 7.28-7.24(m, 1H), 6.53(s, 1H), 6.32(s, 1H), 5.41(s, 2H), 3.96(s, 3H). | |
| 52 | *N*-(6-((1H-Pyrazol-1-yl)methyl)-4-methoxyisoxazolo[4,5-c]pyridin-3-yl)-2-methylbenzo[d]oxazole-5-sulfonamide | 441.10 |
| | 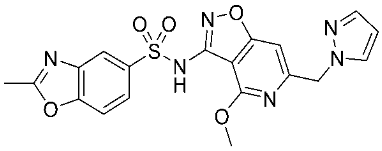 **1-52** | |
| 53 | *N*-(6-((1H-Pyrazol-1-yl)methyl)-4-methoxyisoxazolo[4,5-c]pyridin-3-yl)benzofuran-5-sulfonamide | 426.08 |
| | 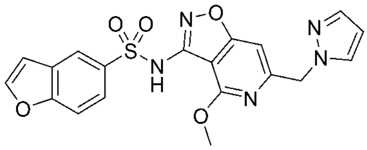 **1-53** | |
| 54 | *N*-(6-((1H-Pyrazol-1-yl)methyl)-4-methoxyisoxazolo[4,5-c]pyridin-3-yl)-3-methyl-2-oxo-3,4-dihydro-2H-benzoxazine-6-sulfonamide | 471.15 |
| | 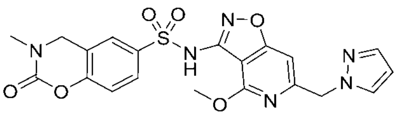 **1-54** | |

(continued)

| Example | Structure/Compound No. | [M+1]+ |
|---|---|---|
| | $^1$H NMR (400 MHz, DMSO-$d_6$) δ (ppm): 7.89 (s, 1H), 7.83-7.79 (m, 2H), 7.51 (s, 1H), 7.214-6.97 (m, 1H), 6.57 (s, 1H), 6.33 (s, 1H), 5.42 (s, 2H), 4.55 (s, 2H), 3.97 (s, 3H), 2.97 (s, 3H). | |
| 55 | *N*-(6-((1H-Pyrazol-1-yl)methyl)-4-methoxyisoxazolo[4,5-c]pyridin-3-yl)benzothiazole-6-sulfonamide<br>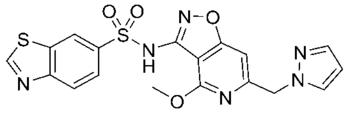<br>1-55<br>$^1$H NMR (400 MHz, DMSO-$d_6$) δ (ppm): 9.63 (s, 1H), 8.77 (s, 1H), 8.28-8.18 (m, 1H), 8.09-8.04 (m, 1H), 7.88 (s, 1H), 7.51 (s, 1H), 6.57 (s, 1H), 6.32 (t, *J* = 2.0Hz, 1H), 5.41 (s, 2H), 3.96 (s, 3H). | 443.15 |
| 56 | *N*-(6-((1H-Pyrazol-1-yl)methyl)-4-methoxyisoxazolo[4,5-c]pyridin-3-yl)-6-methoxy-1-methyl-1H-indole-7-sulfonamide<br>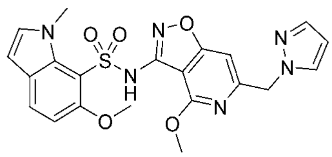<br>1-56<br>$^1$H NMR (400 MHz, DMSO-$d_6$) δ (ppm): 10.76 (s, 1H), 7.88 (s, 1H), 7.50 (s, 1H), 7.28-7.11 (m, 2H), 6.87-6.71 (m, 1H), 6.47-6.32 (m, 2H), 5.39 (s, 2H), 4.16 (s, 3H), 3.94 (s, 3H), 3.65 (s, 3H). | 469.15 |
| 57 | *N*-(6-((1H-Pyrazol-1-yl)methyl)-4-methoxyisoxazolo[4,5-c]pyridin-3-yl)-6-methoxy-1,3-benzodioxole-5-sulfonamide<br>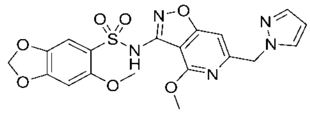<br>1-57<br>$^1$H NMR (400 MHz, DMSO-$d_6$) δ (ppm): 10.65 (s, 1H), 7.89 (d, *J* = 2.0Hz, 1H), 7.51 (s, 1H), 7.24 (s, 1H), 6.93(s, 1H), 6.67 (s, 1H), 6.33 (s, 1H), 6.09 (s, 2H), 5.44 (s, 2H), 3.96 (s, 3H), 3.73 (s, 3H). | 460.25 |
| 58 | *N*-(6-((1H-Pyrazol-1-yl)methyl)-4-methoxyisoxazolo[4,5-c]pyridin-3-yl)-5-methoxy-1,3-benzodioxole-4-sulfonamide<br>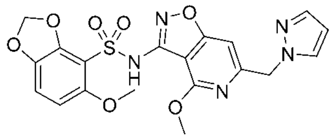<br>1-58<br>$^1$H NMR (400 MHz, DMSO-$d_6$) δ (ppm): 10.96 (s, 1H), 7.89 (d, J = 2.0Hz, 1H), 7.51 (d, J = 1.6Hz, 1H), 7.12-7.09 (m, 1H), 6.78-6.69 (m, 1H), 6.52-6.48 (m, 1H), 6.32 (s, 1H), 6.15-5.98 (m, 2H), 5.43 (s, 2H), 3.96 (s, 3H), 3.64 (s, 3H) | 460.20 |
| 59 | *N*-(6-((1H-Pyrazol-1-yl)methyl)-4-methoxyisoxazolo[4,5-c]pyridin-3-yl)-5-methoxy-2,3-dihydrobenzofuran-6-sulfonamide<br>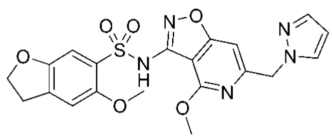<br>1-59 | 458.05 |

(continued)

| Example | Structure/Compound No. | $[M+1]^+$ |
|---|---|---|
| | $^1$H NMR (400 MHz, DMSO-$d_6$) δ (ppm): 7.86 (d, *J* = 2.0 Hz, 1H), 7.49 (d, *J* = 1.6 Hz, 1H), 7.09 (s, 1H), 7.03 (s, 1H), 6.57 (s, 1H), 6.32 (t, *J* = 2.0 Hz, 1H), 5.39 (s, 2H), 4.50 (t, *J* = 8.8 Hz, 2H), 3.93 (s, 3H), 3.67 (s, 3H), 3.18 (t, *J* = 8.8 Hz, 2H). | |
| 60 | *N*-(6-((1H-Pyrazol-1-yl)methyl)-4-methoxyisoxazolo[4,5-c]pyridin-3-yl)-6-methoxy-2,3-dihydrobenzofuran-5-sulfonamide | 458.20 |
| | 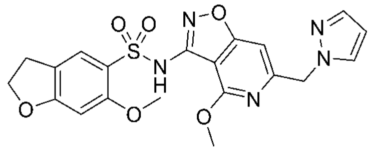 **1-60** | |
| | $^1$H NMR (400 MHz, DMSO-$d_6$) δ (ppm): 10.37 (s, 1H), 7.89 (d, *J* =2.0 Hz, 1H), 7.64 (s, 1H), 7.52 (d, *J* =1.6 Hz, 1H), 6.66-6.62 (m, 2H), 6.33 (t, *J=2.0* Hz, 1H), 5.44 (s, 2H), 4.63 (t, *J* =8.8 Hz, 2H), 3.97 (s, 3H), 3.73 (s, 3H), 3.15 (t, *J* =8.4 Hz, 2H). | |
| 61 | *N*-(6-((1H-Pyrazol-1-yl)methyl)-4-methoxyisoxazolo[4,5-c]pyridin-3-yl)quinoxaline-6-sulfonamide | 438.10 |
| | 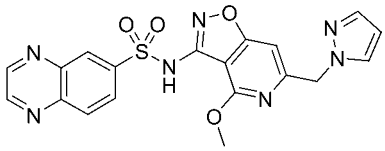 **1-61** | |
| | $^1$H NMR (400 MHz, DMSO-$d_6$) δ (ppm): 9.05 (d, *J* = 2.0Hz, 2H), 8.58(s, 1H), 8.28 (d, *J* = 8.8Hz, 1H), 8.21 (d, *J* = 8.8Hz, 1H), 7.87 (d, *J* = 2.0Hz, 1H), 7.50(s, 1H), 6.51(s, 1H), 6.32(s, 1H), 5.40(s, 2H), 3.95(s, 3H) | |
| 62 | *N*-(6-((1H-Pyrazol-1-yl)methyl)-4-methoxyisoxazolo[4,5-c]pyridin-3-yl)-4,6-dimethoxy-2,3-dihydro-1H-indene-5-sulfonamide | 486.20 |
| | 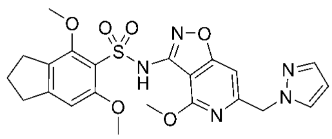 **1-62** | |
| | $^1$H NMR (400 MHz, DMSO-$d_6$) δ (ppm): 7.90 (d, *J* = 2.0 Hz, 1H), 7.51 (d, *J* = 1.2 Hz, 1H), 6.74 (s, 1H), 6.53 (s, 1H), 6.33 (t, *J* = 2.0 Hz, 1H), 5.42 (s, 2H), 3.96 (s, 3H), 3.71 (s, 3H), 3.65 (s, 3H), 2.84-2.81 (m, 4H), 2.01-1.97 (m, 2H) | |
| 63 | *N*-(6-((1H-Pyrazol-1-yl)methyl)-4-methoxyisoxazolo[4,5-c]pyridin-3-yl)-7-chloro-4,6-dimethoxy-2,3-dihydro-1H-indene-5-sulfonamide | 520.15 |
| | 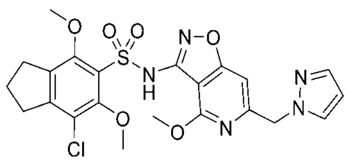 **1-63** | |
| | $^1$H NMR (400 MHz, DMSO-$d_6$) δ (ppm): 10.91 (s, 1H), 7.90 (d, *J* = 2.4 Hz, 1H), 7.51 (d, J = 1.6 Hz, 1H), 6.72 (s, 1H), 6.34-6.33 (m, 1H), 5.44 (s, 2H), 3.92 (s, 3H), 3.80 (s, 3H), 3.75 (s, 3H), 3.05-2.92 (m, 4H), 2.08-2.05 (m, 2H) | |
| | *N*-(6-((1H-Pyrazol-1-yl)methyl)-4-methoxyisoxazolo[4,5-c]pyridin-3-yl)-4-methoxybenzofuran-7-sulfonamide | |

(continued)

| Example | Structure/Compound No. | $[M+1]^+$ |
|---|---|---|
| 64 | 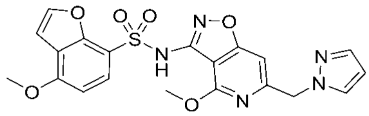 1-64 | 456.20 |
| | $^1H$ NMR (400 MHz, DMSO-$d_6$) δ (ppm): 11.4 (s, 1H), 8.03 (d, *J*=2.0 Hz, 1H), 7.88 (d, *J=2.0* Hz, 1H), 7.78 (d, *J*=8.4 Hz, 1H), 7.51 (s, 1H), 7.05 (d, *J*=2.4 Hz, 1H), 6.97 (d, *J*=8.4 Hz, 1H), 6.69 (s, 1H), 6.33-6.32 (m, 1H), 5.43 (s, 2H), 3.99 (s, 3H), 3.87 (s, 3H) | |
| 65 | *N*-(6-((1H-Pyrazol-1-yl)methyl)-4-methoxyisoxazolo[4,5-c]pyridin-3-yl)-6-methoxy-2-methylbenzofuran-7-sulfonamide | 470.05 |
| | 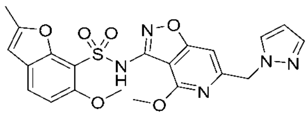 1-65 | |
| | $^1H$ NMR (400 MHz, DMSO-$d_6$) δ (ppm): 7.86 (d, *J* = 2.4 Hz, 1H), 7.49 (d, *J* = 1.6 Hz, 1H), 7.41 (d, *J* = 8.4 Hz, 1H), 6.87 (d, *J* = 8.8 Hz, 1H), 6.39-6.37 (m, 2H), 6.311 (d, *J* = 2.0 Hz, 1H), 5.35 (s, 2H), 3.94 (s, 3H), 3.65 (s, 3H), 2.16 (s, 3H) | |
| 66 | *N*-(6-((1H-Pyrazol-1-yl)methyl)-4-methoxyisoxazolo[4,5-c]pyridin-3-yl)-6-methoxy-2-methyl-2,3-dihvdrobenzofuran-7-sulfonamide | 472.10 |
| | 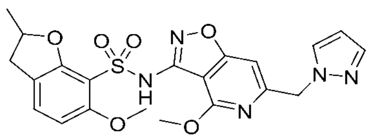 1-66 | |
| | $^1H$ NMR (400 MHz, DMSO-$d_6$) δ (ppm): 7.87 (d, *J* = 2.0 Hz, 1H), 7.50 (d, *J* = 1.2 Hz, 1H), 7.02 (d, *J* = 8.0 Hz, 1H), 6.36-6.32 (m, 3H), 5.36 (s, 2H), 4.68-4.59 (m, 1H), 3.92 (s, 3H), 3.58 (s, 3H), 3.13-3.07 (m, 1H), 2.51-2.50 (m, 1H), 1.29-1.23 (m, 3H) | |
| 67 | *N*-(6-((1H-Pyrazol-1-yl)methyl)-4-methoxyisoxazolo[4,5-c]pyridin-3-yl)-6-methoxy-3,4-dihydro-1H-benzopyran-7-sulfonamide | 472.15 |
| | 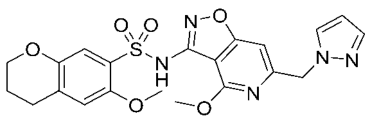 1-67 | |
| | $^1H$ NMR (400 MHz, DMSO-$d_6$) δ (ppm): 10.64 (s, 1H), 7.90 (d, *J* =1.6Hz, 1H), 7.52 (s, 1H), 7.13 (d, *J* =2.4Hz, 1H), 6.96 (s, 1H), 6.71 (s, 1H), 6.33 (s, 1H), 5.44 (s, 2H), 4.02-4.00 (m, 2H), 3.94 (s, 3H), 3.70 (s, 3H), 2.75-2.72 (m, 2H), 1.83-1.80 (m, 2H). | |
| 68 | *N*-(6-((4-Fluoro-1H-pyrazol-1-yl)methyl)-4-methoxyisoxazolo[4,5-c]pyridin-3-yl)-6-methoxv-benzofuran-7-sulfonamide | 474.20 |
| | 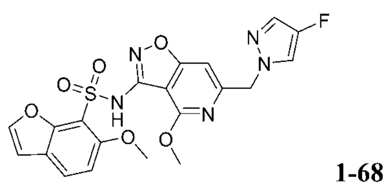 1-68 | |
| | $^1H$ NMR (400 MHz, DMSO-$d_6$) δ (ppm): 11.09 (s, 1H), 8.14 (s, 1H), 7.98 (s, 1H), ,7.77-7.52 (m, 2H), 7.23-6.54(m, 3H), 5.30(s, 2H), 3.89(s, 3H), 3.74(s, 3H) | |
| | *N*-(6-((1H-Pyrazol-1-yl)methyl)-4-methoxyisoxazolo[4,5-c]pyridin-3-yl)-6-methoxy-1-methyl-1H-indole-5-sulfonamide | |

(continued)

| Example | Structure/Compound No. | $[M+1]^+$ |
|---|---|---|
| 69 | 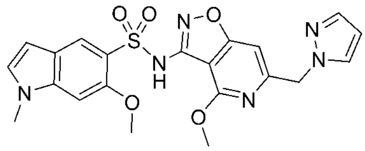 1-69 | 469.20 |
| | $^1H$ NMR (400 MHz, DMSO-$d_6$) δ (ppm): 10.19 (s, 1H), 8.05 (s, 1H), 7.89-7.87 (m, 1H), 7.52-7.50 (m, 1H), 7.29 (s, 1H), 7.11 (, 1H), 6.60-6.50 (m, 2H), 6.32-6.31 (m, 1H), 5.41 (s, 2H), 3.98 (s, 3H), 3.82 (s, 3H), 3.76 (s, 3H) | |
| 70 | *N*-(6-((1H-Pyrazol-1-yl)methyl)-4-methoxyisoxazolo[4,5-c]pyridin-3-yl)-6-methoxy-2,3-dihydro-1H-indene-5-sulfonamide | 456.20 |
| | 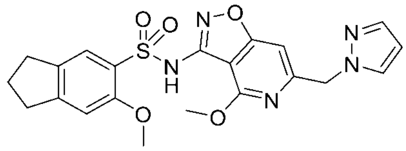 1-70 | |
| | $^1H$ NMR (400 MHz, DMSO-$d_6$) δ (ppm): 10.52 (s, 1H), 7.89 (d, *J*=2.0Hz, 1H), 7.63 (s, 1H), 7.51 (d, *J*=1.2Hz, 1H), 7.04 (s, 1H), 6.66 (s, 1H), 6.34-6.32 (m, 1H), 5.44 (s, 2H), 3.95 (s, 3H), 3.74 (s, 3H), 2.91-2.81 (m, 4H), 2.05-2.01 (m, 2H) | |
| 71 | 6-Methoxy-N-(4-methoxy-6-((3-methyl-1H-pyrazol-1-yl)methyl)isoxazolo[4,5-c]pyridin-3-yl)benzofuran-7-sulfonamide | 470.20 |
| | 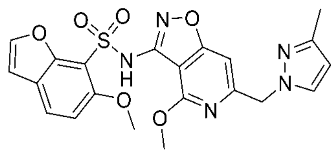 1-71 | |
| | $^1H$ NMR (400 MHz, DMSO-$d_6$) δ (ppm): 11.09 (s, 1H), 8.14 (s, 1H), 7.89 (s, 1H), 7.30 (s, 1H), 7.24-6.89 (m, 3H), 6.52-6.44 (m, 1H), 5.31 (s, 2H), 3.92 (s, 3H), 3.74 (s, 3H), 1.92 (s, 3H) | |
| 72 | *N*-(6-((1H-Pyrazol-1-yl)methyl)-4-methoxyisoxazolo[4,5-c]pyridin-3-yl)-4-methoxy-2,3-dihydrobenzofuran-7-sulfonamide | 458.20 |
| | 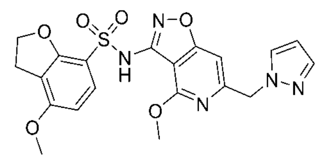 1-72 | |
| | $^1H$ NMR (400 MHz, DMSO-$d_6$) δ (ppm): 10.87 (s, 1H), 7.89 (d, *J*=2 Hz, 1H), 7.56-7.52 (m, 2H), 6.61 (d, *J*=9.2 Hz, 2H), 6.32 (d, *J*=1.6 Hz, 1H), 5.43 (s, 2H), 4.54 (t, *J*=8.8 Hz, 2H), 3.93 (s, 3H), 3.84 (s, 3H), 3.04 (t, *J*=8.8 Hz, 2H) | |
| 73 | *N*-(6-((1H-Pyrazol-1-yl)methyl)-4-methoxyisoxazolo[4,5-c]pyridin-3-yl)-6-methoxy-2,3-dihydrobenzo[b][1,4]dioxine-5-sulfonamide | 474.20 |
| | 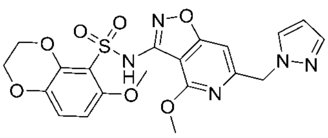 1-73 | |
| | $^1H$ NMR (400 MHz, DMSO-$d_6$) δ (ppm): 10.41 (s, 1H), 7.90 (d, *J* = 2.0 Hz, 1H), 7.52 (d, *J* = 1.6 Hz, 1H), 6.92 (s, 1H), 6.57-6.55(m, 2H), 6.33 (t, *J* = 2.0 Hz, 1H), 5.43 (s, 2H), 4.12 (s, 4H), 3.95 (s, 3H), 3.62 (s, 3H) | |

(continued)

| Example | Structure/Compound No. | [M+1]+ |
|---|---|---|
| 74 | *N*-(6-((1H-Pyrazol-1-yl)methyl)-4-methoxyisoxazolo[4,5-c]pyridin-3-yl)-5-methoxy-2,2-dimethyl-1,3-benzodioxolane-4-sulfonamide<br>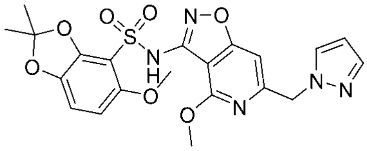<br>1-74<br>$^1$H NMR (400 MHz, DMSO-$d_6$) δ (ppm): 7.88 (d, *J* = 2.0 Hz, 1H), 7.51 (d, *J* = 1.6 Hz, 1H), 6.90(s, 1H), 6.65(s, 1H), 6.41-6.39(m, 1H), 6.33 (t, *J* = 2.0 Hz, 1H), 5.42 (s, 2H), 3.93(s, 3H), 3.64 (s, 3H), 1.45 (s, 6H) | 488.15 |
| 75 | *N*-(6-((1H-Pyrazol-1-yl)methyl)-4-methoxyisoxazolo[4,5-c]pyridin-3-yl)-6-methoxyquinoline-7-sulfonamide<br>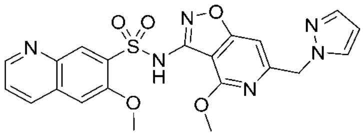<br>1-75<br>$^1$H NMR (400 MHz, DMSO-$d_6$) δ (ppm): 8.83 (d, *J* = 2.8Hz, 1H), 8.44 (s, 1H), 8.33 (d, *J*=3.6Hz, 1H), 7.89 (d, *J* = 2.0Hz, 1H), 7.59-7.50 (m, 3H), 6.60 (s, 1H), 6.33-6.32 (m, 1H), 5.42 (s, 2H), 3.91 (s, 3H), 3.88 (s, 3H) | 467.25 |
| 76 | *N*-(6-((1H-Pyrazol-1-yl)methyl)-4-methoxyisoxazolo[4,5-c]pyridin-3-yl)-7-methoxy-3,4-dihydro-1H-benzopyran-6-sulfonamide<br>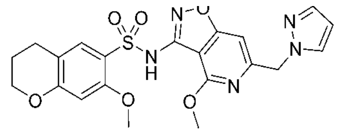<br>1-76<br>$^1$H NMR (400 MHz, DMSO-$d_6$) δ (ppm): 7.88-7.86 (m, 1H), 7.51-7.50 (m, 1H), 7.47 (s, 1H), 6.36 (s, 1H), 6.31 (t, *J* = 2.0 Hz, 1H), 6.28 (s, 1H), 5.36 (s, 2H), 4.11 (t, *J* = 6.4 Hz, 2H), 3.93 (s, 3H), 3.62 (s, 3H), 2.63 (t, *J* = 6.4 Hz, 2H), 1.92-1.85 (m, 2H). | 470.20 |
| 77 | *N*-(6-((1H-Pyrazol-1-yl)methyl)-4-methoxyisoxazolo[4,5-c]pyridin-3-yl)-5-methoxy-2,3-dihydro-1H-indene-4-sulfonamide<br>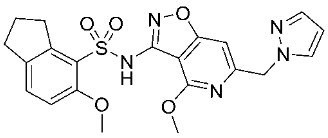<br>1-77<br>$^1$H NMR (400 MHz, DMSO-$d_6$) δ (ppm): 7.88 (s, 1H), 7.50 (s, 1H), 7.47-7.45 (m, 1H), 6.94-6.93 (m, 1H), 6.72 (s, 1H), 6.66 (s, 1H), 6.36 (s, 1H), 5.43 (s, 2H), 3.94 (s, 3H), 3.73 (s, 3H), 3.26-3.24 (m, 2H), 2.81-2.80 (m, 2H), 2.01 - 1.95 (m, 2H) | 456.25 |
| 78 | *N*-(6-((1H-Pyrazol-1-yl)methyl)-4-methoxyisoxazolo[4,5-c]pyridin-3-yl)-5-methoxybenzofuran-3-sulfonamide<br>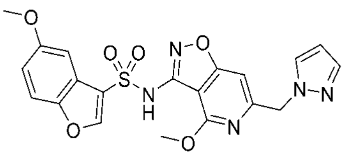<br>1-78<br>$^1$H NMR (400 MHz, DMSO-$d_6$) δ (ppm): 7.88 (d, *J*=2.0Hz, 1H), 7.52-7.50 (m, 2H), 7.28 (s, 1H), 7.22 (d, *J* = 2.8Hz, 1H), 7.01 (dd, *J*=9.2, 2.4Hz, 1H), 6.55 (s, 1H), 6.32 (t, *J*=2.0Hz, 1H), 5.41(s, 2H), 3.90 (s, 3H), 3.78 (s, 3H) | 456.20 |

(continued)

| Example | Structure/Compound No. | $[M+1]^+$ |
|---|---|---|
| 79 | Methyl (4-methoxy-3-((3-methoxy-5,6,7,8-tetrahydronaphthalene)-2-sulfonamido)isoxazolo [4,5-c]pyridin-6-yl)acetate<br>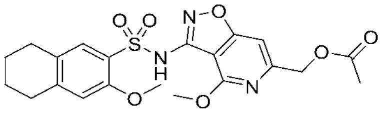 1-79<br>$^1$H NMR (400 MHz, DMSO-$d_6$) δ (ppm): 10.52 (s, 1H), 7.49 (s, 1H), 7.14 (s, 1H), 6.77 (s, 1H), 5.12 (s, 2H), 3.96 (s, 3H), 3.70 (s, 3H), 2.74-2.72 (m, 2H), 2.66-2.64 (m, 2H), 2.15 (s, 3H), 1.72-1.71 (m, 4H). | 462.25 |
| 80 | Methyl (4-methoxy-3-((2-methoxy-5,6,7,8-tetrahydronaphthalene)-1-sulfonamido)isoxazolo [4,5-c]pyridin-6-yl)acetate<br>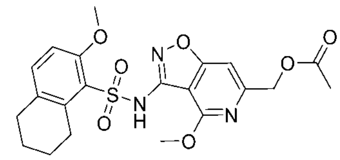 1-80<br>$^1$H NMR (400 MHz, DMSO-$d_6$) δ (ppm): 10.25 (s, 1H), 7.30-7.28 (m, 2H), 7.00 (d, *J*=8.4Hz, 1H), 5.14 (s, 2H), 3.98(s, 3H), 3.77 (s, 3), 3.13-3.12 (m, 2H), 2.75-2.73 (m, 2H), 2.16 (s, 3H), 1.68-1.67 (m, 4H). | 462.25 |
| 81 | *N*-(6-(Hydroxymethyl)-4-methoxyisoxazolo[4,5-c]pyridin-3-yl)-3-methoxy-5,6,7,8-tetrahydronaphthalene-2-sulfonamide<br>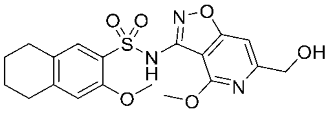 1-81<br>$^1$H NMR (400 MHz, DMSO-$d_6$) δ (ppm): 10.42 (s, 1H), 7.50 (s, 1H), 7.15 (s, 1H), 6.82 (s, 1H), 5.56 (s, 1H), 4.54 (d, *J* = 5.6Hz, 2H), 3.95 (s, 3H), 3.72 (s, 3H), 2.74-2.68 (m, 4H), 1.73-1.72 (m, 4H). | 420.20 |
| 82 | *N*-(6-(Hydroxymethyl)-4-methoxyisoxazolo[4,5-c]pyridin-3-yl)-2-methoxy-5,6,7,8-tetrahydronaphthalene-1-sulfonamide<br>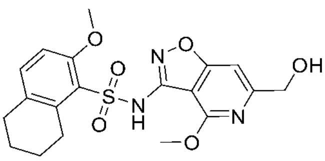 1-82<br>$^1$H NMR (400 MHz, DMSO-$d_6$) δ (ppm): 10.14 (s, 1H), 7.26-6.97 (m, 3H), 5.58 (s, 1H), 4.55 (d, *J* = 5.6Hz, 2H), 3.96 (s, 3H), 3.75 (s, 3H), 3.16-3.15 (m, 2H), 2.73-2.72 (m, 2H), 1.69-1.68 (m, 4H). | 420.20 |
| 83 | 6-Methoxy-N-(4-methoxy-6-((4-methyl-1H-pyrazol-1-yl)methyl)isoxazolo[4,5-c]pyridin-3-yl) benzofuran-7-sulfonamide<br>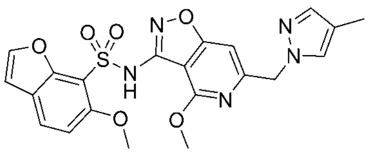 1-83<br>$^1$H NMR (400 MHz, DMSO-$d_6$) δ (ppm): 11.03 (s, 1H), 7.96 (s, 1H), 7.80-7.71 (m, 2H), 7.12 (s, 1H), 6.95 (s, 1H), 6.54-6.45 (m, 1H), 6.08 (d, *J* = 2.4Hz, 1H), 5.31 (s, 2H), 3.88 (s, 3H), 3.87 (s, 3H), 2.16 (s, 3H) | 470.20 |

(continued)

| Example | Structure/Compound No. | [M+1]+ |
|---|---|---|
| 84 | *N*-(6-((1H-Pyrazol-1-yl)methyl)-4-methoxyisoxazolo[4,5-c]pyridin-3-yl)-6-methoxy-3,4-dihydro-1H-benzopyran-8-sulfonamide<br>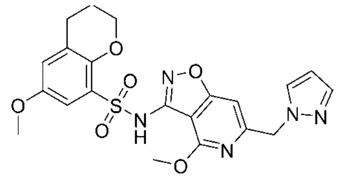<br>1-84<br>$^1$H NMR (400 MHz, DMSO-$d_6$) δ (ppm): 10.70 (s, 1H), 7.92 (d, *J* = 2.0 Hz, 1H), 7.55 (d, *J* = 2.0 Hz, 1H), 7.12 (d, *J* =5.2Hz, 1H), 6.91 (s, 1H), 6.61 (s, 1H), 6.34 (d, *J* = 10.8 Hz, 1H), 5.49 (s, 2H), 4.12-4.10 (m, 2H), 3.95 (s, 3H), 3.67 (s, 3H), 2.77-2.76 (m, 2H), 1.91-1.88 (m, 2H) | 472.20 |
| 85 | *N*-(6-((1H-Pyrazol-1-yl)methyl)-4-methoxyisoxazolo[4,5-c]pyridin-3-yl)-6-methoxy-2,3-dihydro[1,4]dioxino[2,3-b]pyridine-7-sulfonamide<br>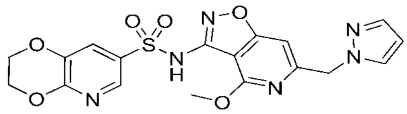<br>1-85<br>$^1$H NMR (400 MHz, DMSO-$d_6$) δ (ppm): 8.16 (d, *J* = 1.6Hz, 1H), 7.88 (d, *J* = 2.0Hz, 1H), 7.63 (d, *J* = 2.0Hz, 1H), 7.51 (d, *J* = 1.2Hz, 1H), 6.50 (s, 1H), 6.32 (t, *J* = 2.0Hz, 1H), 5.40 (s, 2H), 4.45-4.43 (m, 2H), 4.28-4.26 (m, 2H), 3.95 (s, 3H). | 445.20 |
| 86 | *N*-(6-((1H-Pyrazol-1-yl)methyl)-4-methoxyisoxazolo[4,5-c]pyridin-3-yl)-7-methoxybenzofuran-6-sulfonamide<br>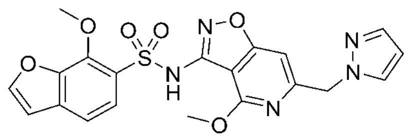<br>1-86<br>$^1$H NMR (400 MHz, DMSO-$d_6$) δ (ppm): 7.88 (d, *J* = 2.4 Hz, 1H), 7.50 (d, *J* = 2.0 Hz, 1H), 7.46 (s, 1H), 7.32 - 7.23 (m, 2H), 7.08-7.06 (m, 1H), 6.63 (s, 1H), 6.35-6.31 (m, 1H), 5.42 (s, 2H), 3.92 (s, 3H), 3.85 (s, 3H). | 456.20 |
| 87 | *N*-(6-((1H-Pyrazol-1-yl)methyl)-4-methoxyisoxazolo[4,5-c]pyridin-3-yl)-4-methoxy-1,3-benzodioxole-5-sulfonamide<br>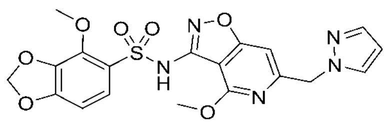<br>1-87<br>$^1$H NMR (400 MHz, DMSO-$d_6$) δ (ppm): 7.89 (d, *J* = 2.0Hz, 1H), 7.51 (s, 1H), 7.24 (s, 1H), 7.18-7.15 (m, 1H), 7.07-7.05 (m, 1H), 6.56 (s, 1H), 6.04 (s, 2H), 5.46 (s, 2H), 3.94 (s, 3H), 3.78 (s, 3H) | 460.20 |
| 88 | *N*-(6-(Fluoro(1H-pyrazol-1-yl)methyl)-4-methoxyisoxazolo[4,5-c]pyridin-3-yl)-3-methoxy-5,6,7,8-tetrahvdronaphthalene-2-sulfonamide<br>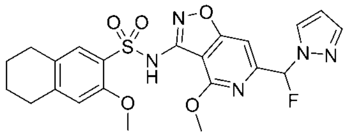<br>1-88 | 488.25 |

(continued)

| Example | Structure/Compound No. | $[M+1]^+$ |
|---|---|---|
| | $^1$H NMR (400 MHz, DMSO-$d_6$) δ (ppm): 10.41 (s, 1H), 8.03 (s, 1H), 7.71 (s, 2H), 7.55-7.42 (m, 1H), 7.31 (s, 1H), 7.02(s, 1H), 6.42 (s, 1H), 3.86 (s, 3H), 3.77 (s, 3H), 3.14 (s, 2H), 2.73 (s, 1H), 2.68 (s, 1H), 1.68 (s, 4H) | |
| 89 | *N*-(6-((1H-Pyrazol-1-yl)methyl)-4-methoxyisoxazolo[4,5-c]pyridin-3-yl)-2,2-difluoro-1,3-benzodioxole-5-sulfonamide | 466.20 |
| | 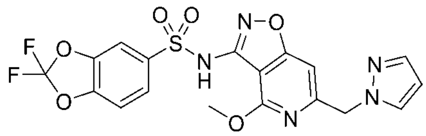 1-89 | |
| | $^1$H NMR (400 MHz, DMSO-$d_6$) δ (ppm): 7.88 (d, *J* = 2.4 Hz, 1H), 7.53 (d, *J* = 8.4 Hz, 1H), 7.51-7.50 (m, 1H), 7.21 (s, 1H), 7.08 (s, 1H), 6.95 (s, 1H), 6.31 (t, *J* = 2.4 Hz, 1H), 5.40 (s, 2H), 3.93 (s, 3H). | |
| 90 | *N*-(6-((1H-Pyrazol-1-yl)methyl)-4-methoxyisoxazolo[4,5-c]pyridin-3-yl)-4-(cyclopropylethynyl)benzofuran-7-sulfonamide | 490.10 |
| | 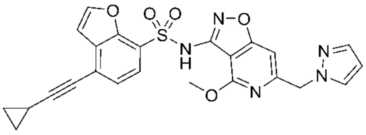 1-90 | |
| | $^1$H NMR (400 MHz, DMSO-$d_6$) δ (ppm): 8.10 (s, 1H), 7.88-7.87 (d, *J* =2.4 Hz, 1H), 7.66-7.64 (d, *J*=7.6 Hz, 1H), 7.50-7.50 (d, *J* =1.2 Hz, 1H), 7.30-7.28 (m, 1H), 7.00-6.98 (m, 1H), 6.48 (s, 1H), 6.31 (s, 1H), 5.39 (s, 2H), 3.90 (s, 3H), 1.67-1.63 (m, 1H), 0.98-0.94 (m, 2H), 0.86-0.83(m, 2H) | |
| 91 | *N*-(6-(Difluoro(1H-pyrazol-1-yl)methyl)-4-methoxyisoxazolo[4,5-c]pyridin-3-yl)-3-methoxy-5,6,7,8-tetrahydronaphthalene-2-sulfonamide | 506.20 |
| | 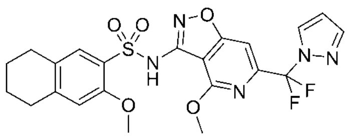 1-91 | |
| | $^1$H NMR (400 MHz, DMSO-$d_6$) δ (ppm): 10.82(s, 1H), 8.19(s, 1H), 7.75(s, 2H), 7.52(s, 1H), 6.82(s, 1H), 6.52(s, 1H), 3.87(s, 3H), 3.71(s, 3H), 2.75(s, 1H), 2.68(s, 1H), 2.51(s, 2H), 1.73(s, 4H). | |
| 92 | *N*-(6-(Difluoro(1H-pyrazol-1-yl)methyl)-4-methoxyisoxazolo[4,5-c]pyridin-3-yl)-2-methoxy-5,6,7,8-tetrahydronaphthalene-1-sulfonamide | 506.20 |
| | 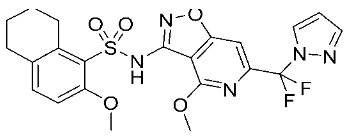 1-92 | |
| | $^1$H NMR (400 MHz, DMSO-$d_6$) δ (ppm): 10.55 (s, 1H), 8.20 (d, J = 2.0 Hz, 1H), 7.86(s, 1H), 7.75(s, 1H), 7.28 (s, 1H), 7.00 -6.98(d, J = 6.8 Hz, 1H), 6.52 (s, 1H), 3.88(s, 3H), 3.76(s, 3H), 3.157(s, 2H), 2.75-2.73(m, 2H), 1.69-1.67(m, 4H) | |
| | *N*-(6-(Fluoro(1H-pyrazol-1-yl)methyl)-4-methoxyisoxazolo[4,5-c]pyridin-3-yl)-2-methoxy-5,6,7,8-tetrahydronaphthalene-1-sulfonamide | |

(continued)

| Example | Structure/Compound No. | [M+1]+ |
|---|---|---|
| 93 | 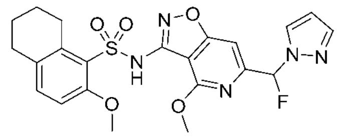 1-93 | 488.20 |
| | $^1$H NMR (400 MHz, DMSO-$d_6$) δ (ppm): 10.68 (s, 1H), 8.03 (s, 1H), 7.65 (s, 2H), 7.55-7.42 (m, 2H), 6.87(s, 1H), 6.42 (s, 1H), 3.86 (s, 3H), 3.72 (s, 3H), 2.76 (s, 2H), 2.69(s, 2H), 2, 1.73-1.72 (s, 4H) | |
| 94 | *N*-(6-((1H-Pyrazol-1-yl)methyl)-4-methoxyisoxazolo[4,5-c]pyridin-3-yl)-5-methoxy-2H-benzopyran-8-sulfonamide | 470.20 |
| | 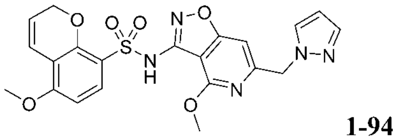 1-94 | |
| | $^1$H NMR (400 MHz, DMSO-$d_6$) δ (ppm): 7.83 (s, 1H), 7.55 (d, *J* = 8.8Hz, 1H), 7.48 (s, 1H), 6.59-6.54(m, 2H), 6.47 (s, 1H), 6.31 (s, 1H), 5.81-5.78 (m, 1H), 5.34 (s, 2H), 4.60(s, 2H), 3.91(s, 3H), 3.88(s, 3H). | |
| 95 | *N*-(6-((1H-Pyrazol-1-yl)methyl)-4-methoxyisoxazolo[4,5-c]pyridin-3-yl)-5-methoxy-3,4-dihydro-1H-benzopyran-8-sulfonamide | 472.25 |
| | 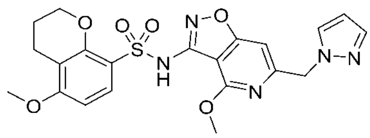 1-95 | |
| | $^1$H NMR (400 MHz, DMSO-$d_6$) δ (ppm): 10.32 (s, 1H), 7.90 (d, *J* = 2.0Hz, 1H), 7.63 (d, *J* = 8.8Hz, 1H), 7.53 (d, *J* = 11.6 Hz, 1H), 6.68-6.63 (m, 1H), 6.55 (s, 1H), 5.44 (s, 2H), 4.05-4.03 (m, 2H), 3.96(s, 3H), 3.83(s, 3H), 2.53-2.51 (m, 2H), 1.83-1.81 (m, 2H) | |

Example 96:

Compound 1-96: *N*-(6-((1H-pyrazol-1-yl)methyl)-4-methoxyisoxazolo[4,5-c]pyridin-3-yl)-4-ethynyl-benzofuran-7-sulfonamide;

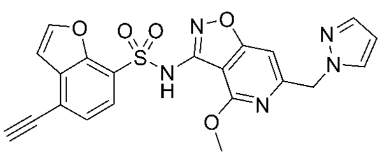

1-96

Synthetic route:

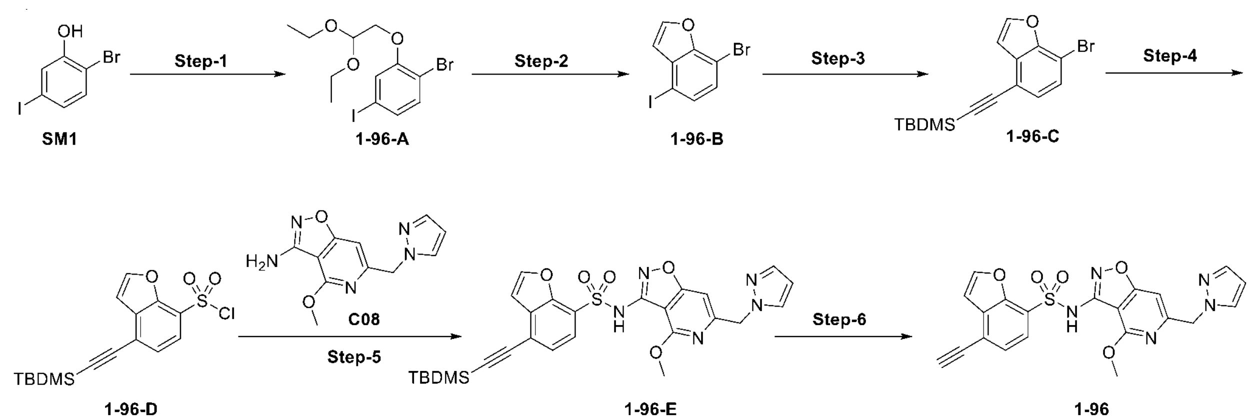


Preparation of compound 1-96-D: Compound 1-96-D was synthesized by procedures similar to Step-1 to Step-4 for D-46 according to a preparation method similar to that for compound D-46, with SM1 as a starting material. $^1H$ NMR (400 MHz, $CDCl_3$-*d*) δ (ppm): 7.76 (d, *J* = 8.4 Hz, 1H), 7.55-7.54 (m, 1H), 7.51-7.33 (m, 1H), 6.96 (d, *J*= 8.4 Hz, 1H), 1.14 (s, 9H), 0.24 (s, 6H).

Step-5: Preparation of compound 1-96-E: According to the procedure of Step-1 for 1-1, the key intermediate C08 and 1-96-D were subjected to a condensation reaction, and the reaction mixture was purified by high-pressure preparative liquid chromatography to give compound 1-96-E. $[M+1]^+$: 564.25.

Step-6: Preparation of compound 1-96: Compound 1-96-E (18 mg, 1 eq) was dissolved in tetrahydrofuran (0.5 mL), and tetramethylammonium fluoride (20.8 mg, 7 eq) was added at 0 °C. The mixture was stirred at room temperature for 16 h, and LCMS showed that the reaction was completed. The reaction solution was directly purified by high-pressure preparative liquid chromatography to give compound 1-96 (4.8 mg, yield: 32.3%). $[M+1]^+$: 450.15.

Compound 1-96: $^1H$ NMR (400 MHz, DMSO-$d_6$) δ (ppm): 8.15 (s, 1H), 7.87 (d, *J* =1.6Hz, 1H), 7.71 (d, *J* = 8.0 Hz, 1H), 7.5 (d, *J* = 1.2Hz, 1H), 7.43 (d, *J* =7.6Hz, 1H), 7.23 (s, 1H), 7.10 (s, 1H), 6.48 (s, 1H), 6.31 (s, 1H), 5.39 (s, 2H), 4.61 (s, 1H), 3.91 (s, 3H).

Example 97

Compound 1-97: *N*-(6-((1H-pyrazol-1-yl)methyl)-4-methoxyisoxazolo[4,5-c]pyridin-3-yl)-4,6-dimethoxy-2,3-dihydrobenzofuran-5-sulfonamide;

Compound 1-98: *N*-(6-((1H-pyrazol-1-yl)methyl)-4-methoxyisoxazolo[4,5-c]pyridin-3-yl)-4,6-dimethoxy-2,3-dihydrobenzofuran-7-sulfonamide;

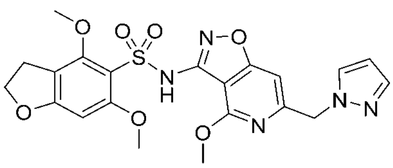

1-97

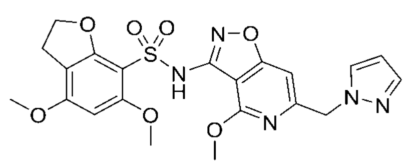

1-98

Synthetic route:

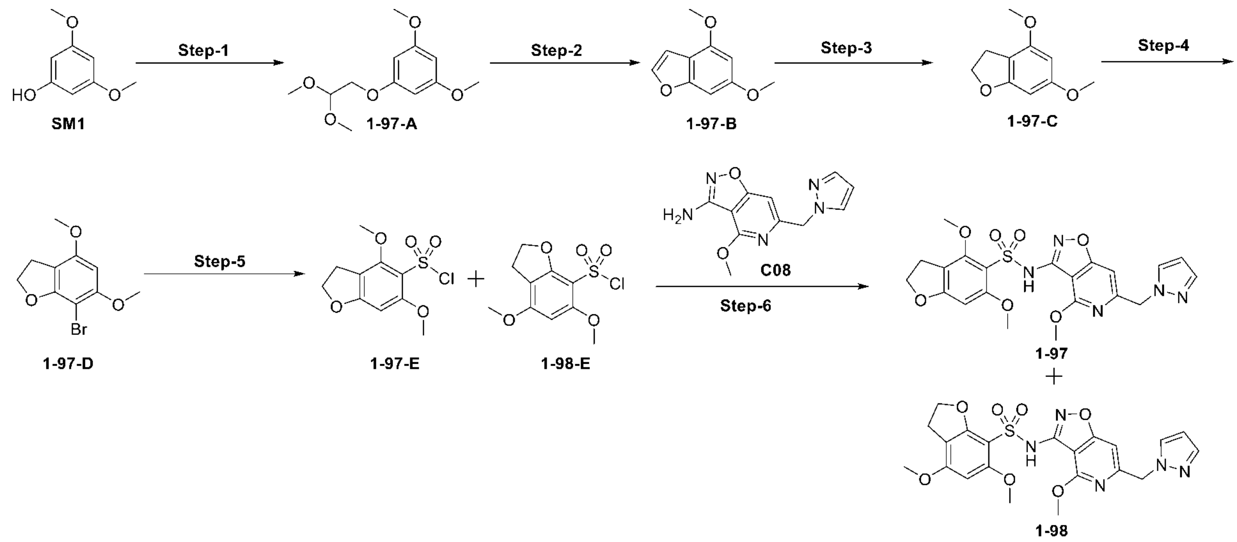


SM1, 1-97-A, 1-97-B, 1-97-C, 1-97-D, 1-97-E, 1-98-E, 1-97, 1-98

Preparation of compound 1-97-B: Compound 1-97-B was synthesized by procedures similar to Step-1 to Step-2 for D-15 according to a preparation method similar to that for compound D-15-2, with SM1 as a starting material.

Preparation of compound 1-97-C: Compound 1-97-C was synthesized by a procedure similar to Step-1 for D-16 according to a preparation method similar to that for compound D-16-1, with 1-97-B as a starting material.

Step-4: Preparation of compound 1-97-D: Compound 1-97-C (830 mg, 1 eq) was dissolved in acetonitrile (20 mL) at room temperature, and N-bromosuccinimide (NBS, 820.1 mg, 1 eq) was added. The mixture was stirred at room temperature for 16 h, and TLC showed that the reaction was completed. The reaction system was added to water to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give compound 1-97-D (1.0 g, yield: 84.1%). $^1$H NMR (400 MHz, $CDCl_3$-*d*) δ (ppm): 6.17 (s, 1H), 4.67 (t, *J*= 8.8 Hz, 2H), 3.89-3.83 (m, 6H), 3.19 (t, *J* = 8.8 Hz, 2H).

Step-5: Preparation of mixture of compound 1-97-E and compound 1-98-E: Tetramethylethylenediamine (161.6 mg, 1.2 eq) was dissolved in n-hexane (5 mL). Under nitrogen atmosphere, the mixture was cooled to -78 °C, and n-butyllithium (0.46 mL, 2.5 M in hexane, 1.0 eq) was added dropwise. The mixture was stirred at -50 °C for 1 h, and a solution of compound 1-97-D (0.3 g, 1.0 equiv) in tetrahydrofuran (5 mL) was added dropwise to the solution described above at -78 °C. The mixture was slowly heated to 0 °C and stirred for 2 h. Then, the reaction system was cooled to -65 °C. Sulfur dioxide gas was introduced for 10 min, and the mixture was naturally heated to 10 °C under stirring. The resulting precipitate was collected by filtration and then washed with diethyl ether (the filtration was performed under nitrogen atmosphere). The resulting solid was slurried with 10 mL of n-hexane again to form a homogeneous slurry. Under nitrogen atmosphere, the mixture was cooled to 0 °C, and sulfuryl chloride (62.7 mg, 0.4 eq) was added dropwise. After the dropwise addition, the reaction mixture was successively stirred at 0 °C for 1 h, diluted with ethyl acetate (40 mL), backwashed 2 times with a saturated sodium chloride solution, and dried over sodium sulfate. After the resulting mixture was filtered, the filtrate was concentrated under reduced pressure and purified by normal phase column chromatography to give a mixture of compound 1-97-E and compound 1-98-E (200 mg, yield: 63.6%). The resulting sulfonyl chloride compounds were confirmed by NOE.

Sulfonyl chloride compound 1-97-E: $^1$H NMR (400 MHz, $CDCl_3$-*d*) δ (ppm): 6.26 (s, 1H), 4.74 (t, *J* = 8.8 Hz, 2H), 4.02 (s, 6H), 3.34 (t, *J* = 8.8 Hz, 2H).

Sulfonyl chloride compound 1-98-E: $^1$H NMR (400 MHz, $CDCl_3$-*d*) δ (ppm): 6.04 (s, 1H), 4.82 (t, *J*= 8.8 Hz, 2H), 3.98-3.95 (m, 6H), 3.12 (t, *J* = 8.8 Hz, 2H).

Step-6: Preparation of compound 1-97 and compound 1-98: According to the procedure of Step-1 for 1-1, the key intermediate C08 and the mixture of 1-97-E and 1-98-E were subjected to a condensation reaction, and the reaction mixture was purified by high-pressure preparative liquid chromatography to give compounds 1-97 and 1-98. Compound 1-97: $[M+1]^+$: 488.25. $^1$H NMR (400 MHz, DMSO-$d_6$) δ (ppm): 9.99 (s, 1H), 7.90 (s, 1H), 7.52 (s, 1H), 6.69 (s, 1H), 6.39 (s, 1H), 6.34 (s, 1H), 5.45 (s, 2H), 4.60 (t, *J* = 8.8 Hz, 2H), 3.99 (s, 3H), 3.81 (s, 3H), 3.71 (s, 3H), 3.24 (t, *J* = 8.8 Hz, 2H).

Compound 1-98: $[M+1]^+$: 488.25. $^1$H NMR (400 MHz, DMSO-$d_6$) δ (ppm): 10.15 (s, 1H), 7.90 (s, 1H), 7.52 (s, 1H), 6.69 (s, 1H), 6.34 (s, 1H), 6.20 (s, 1H), 5.45 (s, 2H), 4.55 (t, *J*= 8.8 Hz, 2H), 3.97 (s, 3H), 3.84 (s, 3H), 3.74 (s, 3H), 2.97 (t, *J* = 8.8 Hz, 2H).

Example 99

Compound 1-99: *N*-(6-((1H-pyrazol-1-yl)methyl)-4-methoxyisoxazolo[4,5-c]pyridin-3-yl)-6-methoxy-2,2-dimethylbenzopyran-8-sulfonamide;

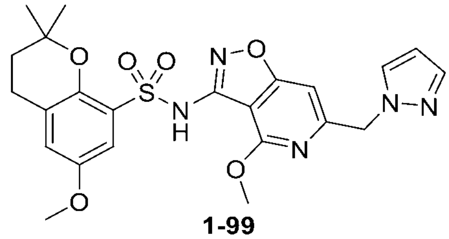

1-99

Synthetic route:

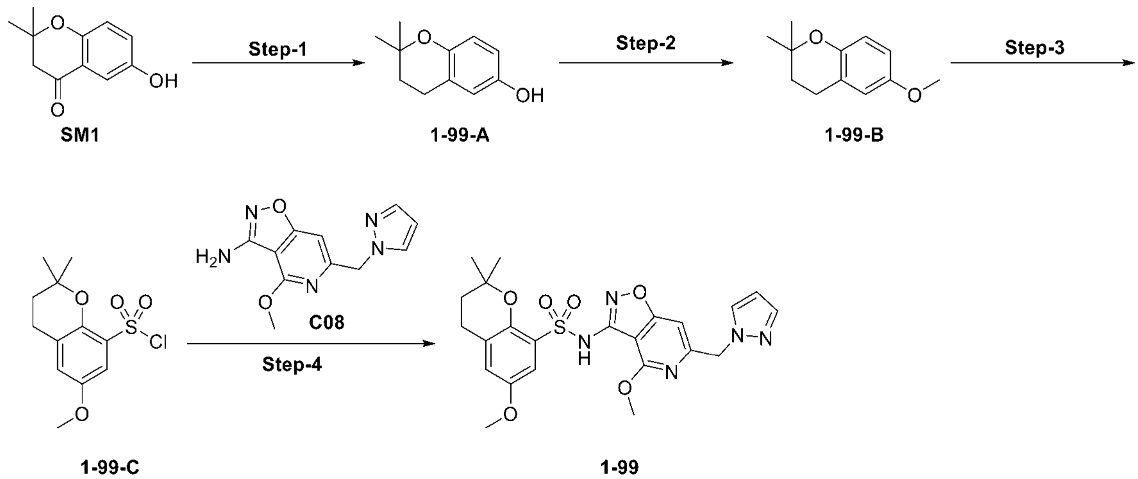


SM1 1-99-A 1-99-B C08 1-99-C 1-99

Step-1: Preparation of compound 1-99-A: SM1 (5 g, 1 eq) was dissolved in methanol (50 mL), and anhydrous palladium on carbon (1 g, 10%) was added under nitrogen atmosphere. The reaction system was purged several times with hydrogen and reacted at room temperature for 16 h under hydrogen atmosphere (1 atm). GCMS showed that the reaction was completed. The mixture was pressurized and filtered, and the filter cake was washed with ethanol and concentrated by rotary evaporation to give a crude product. The crude product was purified by normal phase column chromatography to give compound 1-99-A (1.57 g, yield: 33.9%). $^1$H NMR (400 MHz, DMSO-$d_6$) δ (ppm): 8.67 (s, 1H), 6.51-6.46 (m, 3H), 2.63 (t, *J* = 6.8 Hz, 2H), 1.69 (t, *J* = 6.8 Hz, 2H), 1.22 (s, 6H).

Step-2: Preparation of compound 1-99-B: 1-99-A (1.57 g, 1 eq) was dissolved in tetrahydrofuran (30 mL). Under nitrogen atmosphere, sodium hydride (0.53 g, 60% content, 1.5 eq) was added at 0 °C. Under nitrogen atmosphere, the mixture was stirred at 0 °C for 30 min, and then iodomethane (1.88 g, 1.5 equiv) was added to the reaction system. The mixture was stirred at 0 °C for 2 h, slowly warmed to room temperature, and then stirred for 16 h. GCMS showed that the reaction was completed. The reaction system was added to a saturated ammonium chloride solution to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give compound 1-99-B (1.3 g, yield: 76.7%). $^1$H NMR (400 MHz, DMSO-$d_6$) δ (ppm): 6.65-6.62 (m, 3H), 3.67 (s, 3H), 2.70 (t, *J*= 6.8 Hz, 2H), 1.72 (t, *J* = 6.8 Hz, 2H), 1.24 (s, 6H).

Step-3: Preparation of compound 1-99-C: 1-99-B (500 mg, 1 eq) was dissolved in dichloromethane (5 mL). Under nitrogen atmosphere, chlorosulfonic acid (0.52 mL, 3 equiv) was added dropwise at 0 °C. Under nitrogen atmosphere, the reaction mixture was stirred at 0 °C for 2 h, quenched with crushed ice at 0 °C, and extracted 3 times with dichloromethane. The organic phases were combined, washed with a saturated aqueous sodium chloride solution, dried over anhydrous sodium sulfate, subjected to suction filtration, concentrated by rotary evaporation, and purified by normal phase column chromatography to give compound 1-99-C (40 mg, yield: 5.3%). $^1$H NMR (400 MHz, $CDCl_3$-*d*) δ (ppm): 7.30 (d, *J*= 2.8 Hz, 2H), 7.01 (d, *J* = 2.8 Hz, 2H), 3.82 (s, 3H), 2.86 (t, *J*= 6.8 Hz, 2H), 1.93 (t, *J* = 6.8 Hz, 2H), 1.46 (s, 6H).

Step-4: Preparation of compound 1-99: According to the procedure of Step-1 for 1-1, a mixture of the key intermediate C08 and 1-99-C was subjected to a condensation reaction, and the reaction mixture was purified by high-pressure preparative liquid chromatography to give compound 1-99. $[M+1]^+$: 500.30. $^1H$ NMR (400 MHz, DMSO-$d_6$) δ (ppm): 7.87 (d, *J*=2.0 Hz, 1H), 7.50 (d, *J*=1.6 Hz, 1H), 7.17 (d, *J*=3.2 Hz, 1H), 6.78 (s, 1H), 6.46 (s, 1H), 6.33-6.32 (m, 1H), 5.39 (s, 2H), 3.94 (s, 3H), 3.68 (s, 3H), 2.67 (t, *J*= 6.8 Hz, 2H), 1.64 (t, *J*= 6.8 Hz, 2H), 1.03 (s, 6H).

Example 100

Compound 1-100: *N*-(6-((1H-pyrazol-1-yl)methyl)-4-methoxyisoxazolo[4,5-c]pyridin-3-yl)-6-methoxy-2,2-dimethyl-3H-benzofuran-7-sulfonamide

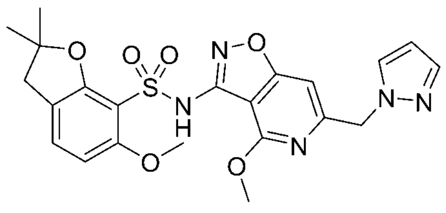

**1-100**

Synthetic route:

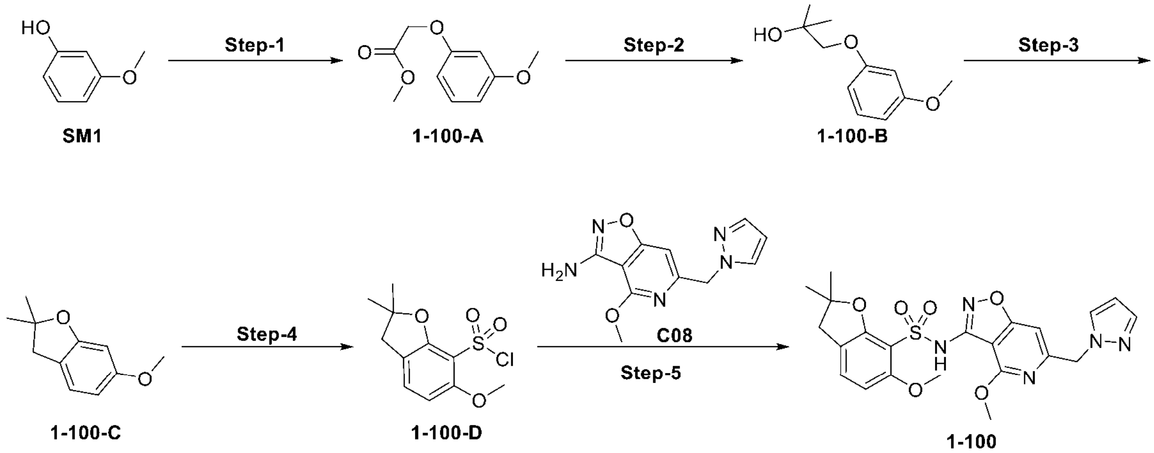


SM1, 1-100-A, 1-100-B, 1-100-C, 1-100-D, 1-100

Step-1: Preparation of compound 1-100-A: SM1 (20 g, 1 equiv) and potassium carbonate (111.3 g, 5 eq) were added to N,N-dimethylformamide (250 mL). Under nitrogen atmosphere, methyl bromoacetate (37.1 g, 1.5 equiv) was slowly added dropwise. Under nitrogen atmosphere, the mixture was heated to 65 °C and stirred for 16 h, and GCMS showed that the reaction was completed. The reaction system was added to water to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give compound 1-100-A (30 g, yield: 94.9%). $^1H$ NMR (400 MHz, $CDCl_3$-*d*) δ (ppm): 7.20 (t, *J*= 8.0 Hz, 1H), 6.59-6.58 (m, 1H), 6.52-6.48 (m, 2H), 4.63 (s, 2H), 3.81 (s, 3H), 3.79 (s, 3H).

Step-2: Preparation of compound 1-100-B: 1-100-A (20 g, 1 eq) was dissolved in tetrahydrofuran (200 mL). Under nitrogen atmosphere, methylmagnesium bromide (3 M in diethyl ether, 102.4 mL, 3 eq) was added dropwise under stirring at 0 °C. Under nitrogen atmosphere, the mixture was slowly warmed to room temperature and stirred for 3 h. The reaction system was added to a saturated ammonium chloride solution to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give compound 1-100-B (18.2 g, yield: 92.5%). $^1H$ NMR (400 MHz, $CDCl_3$-*d*) δ (ppm): 7.20 (t, *J* = 8.0 Hz, 1H), 6.56-6.51 (m, 3H), 3.81 (s, 3H), 3.79 (s, 2H), 2.38 (s, 1H), 1.36 (s, 6H).

Step-3: Preparation of compound 1-100-C: Under nitrogen atmosphere, methanesulfonic acid (200 mL) was added to phosphorus pentoxide (21.7 g, 3 eq), and the mixture was stirred homogeneously. 1-100-B (10 g, 1 eq) was added in batches at room temperature, and the mixture was stirred at room temperature for 3 h under nitrogen atmosphere. GCMS showed that the reaction was completed. The reaction system was added to ice water to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give compound 1-100-C (3.5 g, yield: 38.5%). $^1$H NMR (400 MHz, $CDCl_3$-*d*) δ (ppm): 7.04-7.01 (m, 1H), 6.42-6.37 (m, 2H), 3.78 (s, 3H), 2.96 (s, 2H), 1.49 (s, 6H).

Step-4: Preparation of compound 1-100-D: Compound 1-100-D was synthesized according to a procedure similar to Step-2 in the preparation method for D-26, with 1-100-C as a starting material. $^1$H NMR (400 MHz, $CDCl_3$-*d*) δ (ppm): 7.32 (d, *J* = 8.0 Hz, 1H), 6.45 (d, *J* = 8.0 Hz, 1H), 3.97 (s, 3H), 2.99 (s, 2H), 1.56 (s, 6H).

Step-5: Preparation of compound 1-100: According to the procedure of Step-1 for 1-1, a mixture of the key intermediate C08 and 1-100-D was subjected to a condensation reaction, and the reaction mixture was purified by high-pressure preparative liquid chromatography to give compound 1-100. [M+1]$^+$: 486.25. $^1$H NMR (400 MHz, DMSO-$d_6$) δ (ppm): 10.34 (s, 1H), 7.90 (d, *J=2.0* Hz, 1H), 7.52 (d, J=1.6 Hz, 1H), 7.27 (s, 1H), 6.68 (s, 1H), 6.53-6.51 (m, 1H), 6.33-6.32 (m, 1H), 5.45 (s, 2H), 3.97 (s, 3H), 3.73 (s, 3H), 2.87 (s, 2H), 1.18 (s, 6H).

Example 101

Compound 1-101: *N*-(6-((1H-pyrazol-1-yl)methyl)-4-methoxyisoxazolo[4,5-c]pyridin-3-yl)-5-methoxy-3,3-dimethyl-2H-benzofuran-6-sulfonamide

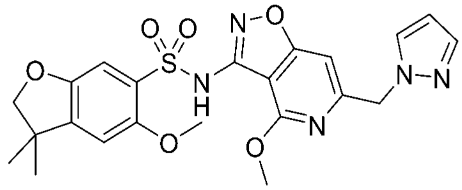

**1-101**

Synthetic route:

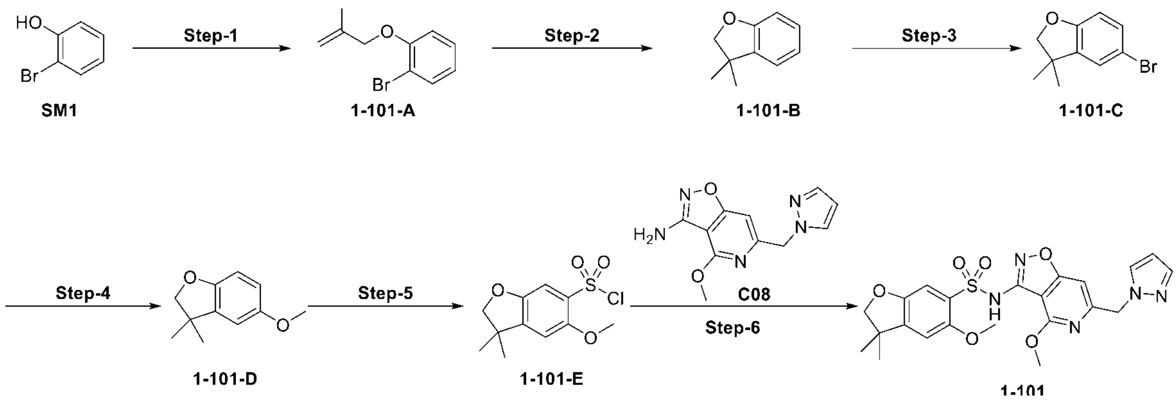


Step-1: Preparation of compound 1-101-A: SM1 (10 g, 1 eq) was dissolved in acetonitrile (100 mL), and 3-bromo-2-methylpropene (8.6 g, 1.1 equiv) and potassium carbonate (19.97 g, 2.5 eq) were sequentially added under stirring. Under nitrogen atmosphere, the mixture was heated to 85 °C and stirred for 16 h. GCMS showed that the reaction was completed. The reaction system was filtered under reduced pressure, and the filter cake was washed 3 times with ethyl acetate. The mother liquor was combined, concentrated by rotary evaporation, and purified by normal phase column chromatography to give compound 1-101-A (11 g, yield: 82.1%). $^1$H NMR (400 MHz, $CDCl_3$-*d*) δ (ppm): 7.58-7.55 (m, 1H), 7.28-7.24 (m, 1H), 6.92-6.90 (m, 1H), 6.87-6.83 (m, 1H), 5.19 (s, 1H), 5.04 (s, 1H), 4.52 (s, 2H), 1.88 (s, 3H).

Step-2: Preparation of compound 1-101-B: 1-101-A (9 g, 1 eq) was dissolved in toluene (100 mL). Under nitrogen atmosphere, tri-n-butyltin hydride (17.3 g, 1.5 eq) and azobisisobutyronitrile (0.65 g, 0.1 eq) were sequentially added. Under nitrogen atmosphere, the mixture was heated to reflux and stirred for 16 h. After the mixture was cooled to room temperature, potassium fluoride (w/w%, 10%, 30 mL) was added. The mixture was stirred for 2 h. After liquid separation, the organic phase was washed three times with a saturated aqueous sodium bicarbonate solution, washed with a saturated aqueous sodium chloride solution, dried over anhydrous sodium sulfate, subjected to suction filtration, concentrated by rotary evaporation, and purified by normal phase column chromatography to give compound 1-101-B (2.0 g, yield: 34.1%). $^1$H NMR (400 MHz, $CDCl_3$-*d*) δ (ppm): 7.20-7.15 (m, 2H), 6.93-6.91 (m, 1H), 6.88-6.86 (m, 1H), 6.87-6.83 (m, 1H), 4.27 (s, 2H), 1.39 (s, 6H).

Step-3: Preparation of compound 1-101-C: 1-101-B (2.0 g, 1 eq) was dissolved in *N,N*-dimethylformamide (100 mL), and *N*-bromosuccinimide (NBS, 4.8 g, 2 eq) was added. The mixture was stirred at room temperature overnight under nitrogen atmosphere. GCMS showed that the reaction was completed. A saturated aqueous sodium bisulfite solution was added to the reaction solution to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give compound 1-101-C (2.0 g, yield: 62.1%). $^1$H NMR (400 MHz, $CDCl_3$-*d*) δ (ppm): 7.24-7.20 (m, 2H), 6.69 (d, *J*= 8.4 Hz, 1H), 4.26 (s, 2H), 1.35 (s, 6H).

Step-4: Preparation of compound 1-101-D: 1-101-C (1.0 g, 1 eq) was dissolved in N,Ndimethylformamide (10 mL). After complete dissolution, copper(I) iodide (0.17 g, 0.2 equiv) and sodium methoxide (0.95 g, 4 equiv) were added. Under nitrogen atmosphere, the mixture was stirred at 145 °C for 3 h. GCMS showed that the reaction was completed. The reaction system was added to ice water to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give compound 1-101-D (0.5 g, yield: 62.4%). $^1$H NMR (400 MHz, $CDCl_3$-*d*) δ (ppm): 6.71-6.64 (m, 3H), 4.21 (s, 2H), 3.81 (s, 3H), 1.35 (s, 6H).

Step-5: Preparation of compound 1-101-E: Compound 1-101-E was synthesized according to a procedure similar to Step-2 in the preparation method for D-26, with 1-101-D as a starting material.

Step-6: Preparation of compound 1-101: According to the procedure of Step-1 for 1-1, a mixture of the key intermediate C08 and 1-101-E was subjected to a condensation reaction, and the reaction mixture was purified by high-pressure preparative liquid chromatography to give compound 1-101. [M+1]$^+$: 486.25. $^1$H NMR (400 MHz, DMSO-$d_6$) δ (ppm): 7.88 (d, *J* =2.4Hz, 1H), 7.51 (d, *J* =1.6Hz, 1H), 7.08 (d, *J* =2.4Hz, 1H), 6.88 (s, 1H), 6.52 (s, 1H), 6.33-6.31 (m, 1H), 5.39 (s, 2H), 4.13 (s, 2H), 3.93 (s, 3H), 3.68 (s, 3H), 1.24 (s, 6H).

Example 102

Compound 1-102: *N*-(6-((1H-pyrazol-1-yl)methyl)-4-methoxyisoxazolo[4,5-c]pyridin-3-yl)-6-methoxybenzofuran-7-sulfonamide-2-*d*;

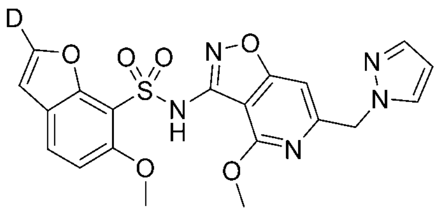

**1-102**

Synthetic route:

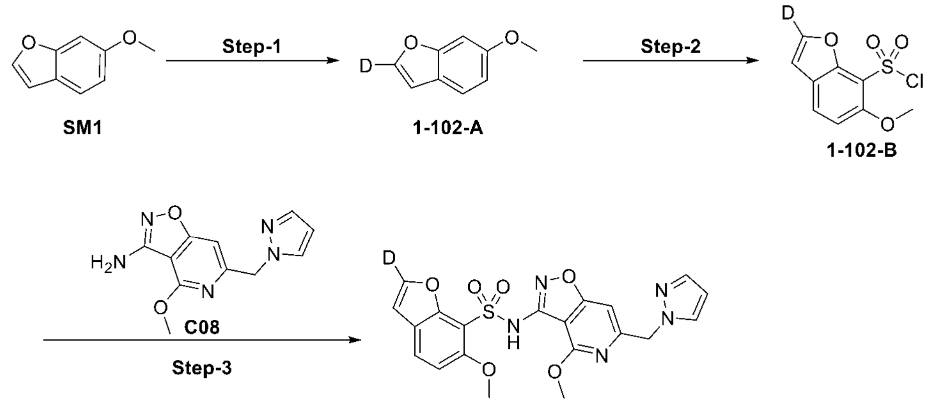


SM1 1-102-A 1-102-B

1-102

Step-1: Preparation of compound 1-102-A: SM1 (2 g, 1 eq) was dissolved in extra dry tetrahydrofuran (500 mL). Under nitrogen atmosphere, the mixture was cooled to -78 °C, and n-butyllithium (2.5 M in n-hexane, 6.48 mL, 1.2 eq) was added dropwise for reaction. The mixture was stirred for 30 min, and deuterium oxide ($D_2O$, 540.7 mg, 2 eq) was slowly added dropwise at -78 °C. The mixture was slowly and naturally warmed to room temperature under stirring. GCMS showed that the reaction was completed. A saturated aqueous ammonium chloride solution was added to the reaction solution to quench the reaction, and the mixture was extracted 3 times with ethyl acetate. The organic phases were combined, washed with a saturated aqueous sodium chloride solution, dried over anhydrous sodium sulfate, subjected to suction filtration, concentrated by rotary evaporation, and purified by normal phase column chromatography to give compound 1-102-A (2 g, yield: 99.3%). $^1H$ NMR (400 MHz, $CDCl_3$-*d*) δ (ppm): 7.49(d, *J* =8.4 Hz, 1H), 7.08(d, *J* =2.0 Hz, 1H), 6.92 (dd, *J* =8.4, 2.0 Hz, 1H), 6.72 (s, 1H), 3.88 (s, 3H).

Step-2: Preparation of compound 1-102-B: Compound 1-102-B was synthesized according to a procedure similar to Step-2 in the preparation method for D-26, with 1-102-A as a starting material. $^1H$ NMR (400 MHz, $CDCl_3$-*d*) δ (ppm): 7.88(d, *J* =8.0 Hz, 1H), 7.07(d, *J* =8.0 Hz, 1H), 6.82 (s, 1H), 4.12 (s, 3H).

Step-3: Preparation of compound 1-102: According to the procedure of Step-1 for 1-1, a mixture of the key intermediate C08 and 1-102-B was subjected to a condensation reaction, and the reaction mixture was purified by high-pressure preparative liquid chromatography to give compound 1-102. $[M+1]^+$: 457.15. $^1H$ NMR (400 MHz, DMSO-$d_6$) δ (ppm): 7.86 (s, 1H), 7.72 (s, 1H), 7.49 (s, 1H), 7.02(d, *J* =8.4Hz, 1H), 6.88 (s, 1H), 6.49 (s, 1H), 6.32 (s, 1H), 5.38 (s, 2H), 3.90 (s, 3H), 3.72 (s, 3H).

Example 103

Compound 1-103: *N*-(6-((1H-pyrazol-1-yl)methyl)-4-methoxyisoxazolo[4,5-c]pyridin-3-yl)-5-methoxy-2,3-dihydrobenzofuran-6-sulfonamide-2,3-$d_2$;

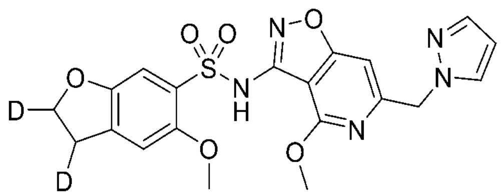

**1-103**

Synthetic route:

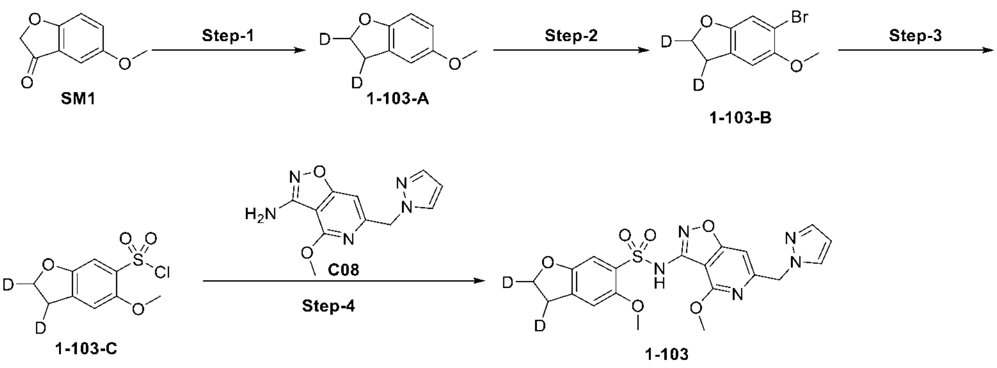


Step-1: Preparation of compound 1-103-A: SM1 (2 g, 1 eq) was dissolved in deuterated methanol (10 mL). Under nitrogen atmosphere, anhydrous palladium on carbon (130 mg, 10%, 1 eq) and deuterated formic acid (1 g, 1.71 eq) were added. The mixture was purged several times with hydrogen, reacted at room temperature for 16 h under hydrogen atmosphere (5 atm), and filtered. The filter cake was washed three times with ethyl acetate, and the filtrate was concentrated under reduced pressure. The crude product was purified by normal phase column chromatography to give compound 1-103-A (0.5 g, yield: 26.4%). $^1$H NMR (400 MHz, $CDCl_3$-*d*) δ (ppm): 6.81 (d, *J* =2.4 Hz, 1H), 6.73-6.66 (m, 2H), 4.58-4.54 (m, 1H), 3.78 (s, 3H), 3.23-3.19 (m, 1H).

Step-2: Preparation of compound 1-103-B: 1-103-A (500 mg, 1 eq) was dissolved in dichloromethane (5 mL) at room temperature. After complete dissolution, 1,3-dibromo-5,5-dimethylhydantoin (469.7 mg, 0.5 eq) was added at 0 °C, and the mixture was stirred at 0 °C for 2 h. TLC showed that the reaction was completed. A saturated aqueous sodium bisulfite solution was added to quench the reaction system, and the reaction mixture was extracted three times with dichloromethane. The organic phases were combined, washed 3 times with a saturated sodium chloride solution, dried over anhydrous sodium sulfate, subjected to suction filtration, and concentrated by rotary evaporation. The crude product was purified by normal phase column chromatography to give 1-103-B (350 mg, yield: 45.2%). $^1$H NMR (400 MHz, $CDCl_3$-*d*) δ (ppm): 7.00 (s, 1H), 6.84 (s, 1H), 4.61-4.56(m, 1H), 3.85 (s, 3H), 3.21-3.17(m, 1H). Step-3: Preparation of compound 1-103-C: Compound 1-103-C was synthesized according to the procedure of Step-3 for compound D-15, with 1-103-B as a starting material. $^1$H NMR (400 MHz, $CDCl_3$-*d*) δ (ppm): 7.34 (s, 1H), 7.03 (s, 1H), 4.68-4.64 (m, 1H), 4.02 (s, 3H), 3.35-3.31 (m, 1H).

Step-4: Preparation of compound 1-103: According to the procedure of Step-1 for 1-1, a mixture of the key intermediate C08 and 1-103-C was subjected to a condensation reaction, and the reaction mixture was purified by high-pressure preparative liquid chromatography to give compound 1-103. [M+1]$^+$: 460.00. $^1$H NMR (400 MHz, DMSO-$d_6$) δ (ppm): 10.67 (s, 1H), 7.89 (d, *J* =2.4Hz, 1H), 7.51 (d, *J* =1.6Hz, 1H), 7.24-7.06 (s, 3H), 6.57-6.52 (m, 1H), 6.32 (t, *J* = 2.0Hz, 1H), 5.42 (s, 2H), 4.54-4.49 (m, 1H), 3.95 (s, 3H), 3.69 (s, 3H), 3.21-3.18 (m, 1H).

Example 104

Compound 1-104: *N*-(6-((1H-pyrazol-1-yl)methyl-$d_2$)-4-methoxyisoxazolo[4,5-c]pyridin-3-yl)-5-methoxy-2,3-dihydrobenzofuran-6-sulfonamide

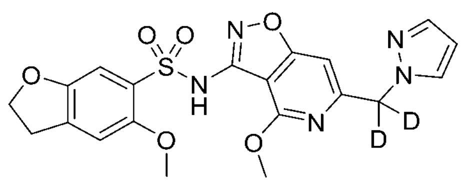

**1-104**

Synthetic route:

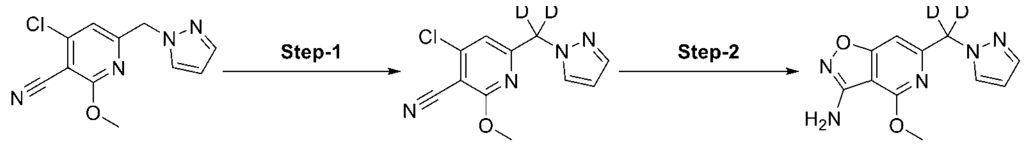


C07 1-104-A 1-104-B

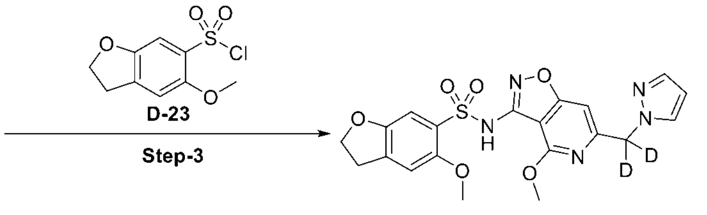


1-104

Step-1: Preparation of compound 1-104-A: C07 (250 mg, 1 eq) was dissolved in tetrahydrofuran (12.5 mL). Under nitrogen atmosphere, the mixture was cooled to -78 °C, and n-butyllithium (2.5 M in n-hexane, 1.0 mL, 2.5 eq) was slowly added dropwise for reaction. The mixture was stirred for 30 min, and deuterium oxide ($D_2O$, 0.05 mL, 3 eq) was slowly added dropwise at -78 °C under stirring. The mixture was slowly and naturally warmed to room temperature under stirring. A saturated aqueous ammonium chloride solution was added to the reaction solution to quench the reaction, and the mixture was extracted 3 times with ethyl acetate. The organic phases were combined, washed with a saturated aqueous sodium chloride solution, dried over anhydrous sodium sulfate, subjected to suction filtration, and concentrated by rotary evaporation to give a crude product. The above experimental procedures were repeated 4 times with the crude product as a starting material, and finally, the resulting crude product was purified by normal phase column chromatography to give compound 1-104-A (150 mg, yield: 59.5%). $[M+1]^+$: 251. $^1H$ NMR (400 MHz, $CDCl_3$-*d*) δ (ppm): 7.65 (s, 1H), 7.53 (s, 1H), 6.61 (s, 1H), 6.43 (s, 1H), 4.08 (s, 3H).

Step-2: Preparation of compound 1-104-B: 1-104-A (100 mg, 1 eq) was added to tert-butanol (30 mL), and the mixture was completely dissolved. Subsequently, potassium carbonate (110.3 mg, 2 eq) and acetohydroxamic acid (60 mg, 2 eq) were sequentially added. Under nitrogen atmosphere, the mixture was stirred at room temperature for 16 h. LCMS showed that the reaction was completed. The mixture was filtered, and the filter cake was washed with acetonitrile. The filtrate was concentrated under reduced pressure. The resulting crude product was purified by preparative chromatography to give compound 1-104-B (45 mg, yield: 45.9%). $[M+1]^+$: 248. $^1H$ NMR (400 MHz, DMSO-$d_6$) δ (ppm): 7.90 (d, *J* =2.0Hz, 1H), 7.52 (d, *J* =2.0Hz, 1H), 6.57 (s, 1H), 6.33-6.32 (m, 1H), 6.20 (s, 2H), 3.98 (s, 3H).

Step-3: Preparation of compound 1-104: According to the procedure of Step-1 for 1-1, a mixture of the key intermediate D-23 and 1-104-B was subjected to a condensation reaction, and the reaction mixture was purified by high-pressure preparative liquid chromatography to give compound 1-104. $[M+1]^+$: 460.20. $^1H$ NMR (400 MHz, DMSO-$d_6$) δ (ppm): 7.84 (d, *J* =2.0Hz, 1H), 7.49 (s, 1H), 7.09 (s, 1H), 7.01 (s, 1H), 6.58 (s, 1H), 6.32 (s, 1H), 4.49 (t, *J* =8.4Hz, 2H), 3.92(s, 3H), 3.66 (s, 3H), 3.17 (t, *J* =8.4Hz, 2H).

Example 105

Compound 1-105: *N*-(6-((1H-pyrazol-1-yl)methyl)-4-methoxyisoxazolo[4,5-c]pyridin-3-yl)-5-methoxy-2,2-dimethyl-2,3-dihydrobenzofuran-6-sulfonamide

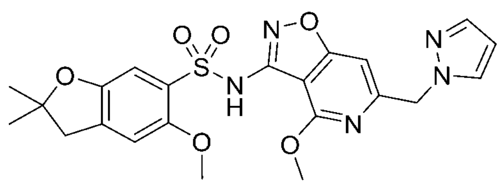

1-105

Synthetic route:

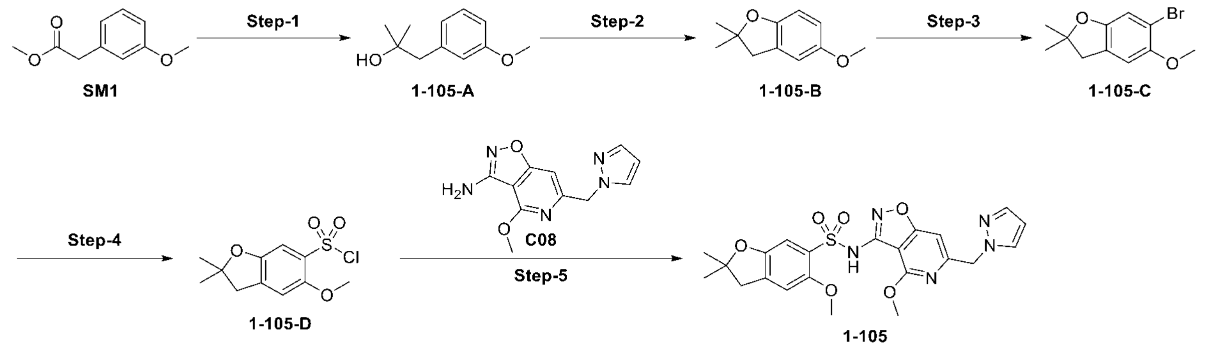


SM1 1-105-A 1-105-B 1-105-C 1-105-D 1-105

Step-1: Preparation of compound 1-105-A: SM1 (5 g, 1 eq) was dissolved in tetrahydrofuran (50 mL). Under nitrogen atmosphere, the mixture was cooled to 0 °C, and methylmagnesium bromide (3 M in diethyl ether, 27.7 mL, 3 eq) was slowly added dropwise for reaction. After the mixture was stirred at room temperature for 3 h, a saturated aqueous ammonium chloride solution was added to the reaction solution to quench the reaction, and the mixture was extracted 3 times with ethyl acetate. The organic phases were combined, washed with a saturated aqueous sodium chloride solution, dried over anhydrous sodium sulfate, subjected to suction filtration, and concentrated by rotary evaporation,. The resulting crude product was purified by normal phase column chromatography to give compound 1-105-A (4 g, yield: 80.1%). $^1H$ NMR (400 MHz, $CDCl_3$-*d*) δ (ppm): 7.27-7.23 (m, 1H), 6.84-6.79 (m, 3H), 3.82 (s, 3H), 2.76 (s, 2H), 1.25 (s, 6H).

Step-2: Preparation of compound 1-105-B: 1-105-A (2 g, 1 eq) and lithium carbonate (1.23 g, 1.5 eq) were added to hexafluorobenzene (50 mL) at room temperature, and then iodobenzene diacetate (5.36 g, 1.5 eq) and palladium(II) acetate (249.1 mg, 0.1 eq) were added. Under nitrogen atmosphere, the mixture was heated to 100 °C and stirred for 25 h. After cooling to room temperature, the reaction system was subjected to suction filtration, and the filter cake was washed with ethyl acetate. The organic phases were combined and concentrated under reduced pressure by rotary evaporation. The resulting crude product was purified by normal phase column chromatography to give compound 1-105-B (1.2 g, yield: 68.7%). $^1H$ NMR (400 MHz, $CDCl_3$-*d*) δ (ppm): 6.77 (s, 1H), 6.68-6.66 (m, 2H), 3.77 (s, 3H), 3.06 (s, 2H), 1.49 (s, 6H).

Step-3: Preparation of compound 1-105-C: 1-105-B (100 mg, 1 eq) was dissolved in acetonitrile (5 mL), and N-bromosuccinimide (71.6 mg, 1 eq) was added at 0 °C. Under nitrogen atmosphere, the mixture was slowly warmed to room temperature and stirred for 16 h. The reaction mixture was quenched with a saturated aqueous sodium thiosulfate solution at room temperature and extracted 3 times with ethyl acetate. The organic phases were combined, washed with a saturated aqueous sodium chloride solution, dried over anhydrous sodium sulfate, subjected to suction filtration, and concentrated by rotary evaporation. The resulting crude product was purified by normal phase column chromatography to give compound 1-105-C (70 mg, yield: 69.4%). $^1H$ NMR (400 MHz, $CDCl_3$-*d*) δ (ppm): 6.94 (s, 1H), 6.79 (s, 1H), 3.85 (s, 3H), 2.98 (s, 2H), 1.48 (s, 6H).

Step-4: Preparation of compound 1-105-D: Compound 1-105-D was synthesized according to the procedure of Step-3 for compound D-15, with 1-105-C as a starting material. $^1H$ NMR (400 MHz, $CDCl_3$-*d*) δ (ppm): 7.29 (s, 1H), 6.97 (s, 1H), 4.01 (s, 3H), 3.10 (s, 2H), 1.51 (s, 6H).

Step-5: Preparation of compound 1-105: According to the procedure of Step-1 for 1-1, a mixture of the key intermediate C08 and 1-105-D was subjected to a condensation reaction, and the reaction mixture was purified by high-pressure preparative liquid chromatography to give compound 1-105. $[M+1]^+$: 486.25. $^1H$ NMR (400 MHz, DMSO-$d_6$) δ (ppm): 7.86 (d, *J*=2.0Hz, 1H), 7.50 (d, *J*=1.6Hz, 1H), 7.02 (s, 1H), 6.96 (s, 1H), 6.54 (s, 1H), 6.32-6.31 (m, 1H), 5.38 (s, 2H), 3.92 (s, 3H), 3.67 (s, 3H), 2.99 (s, 2H), 1.37 (s, 6H).

Example 106

Compound 1-106: *N*-(6-((1H-pyrazol-1-yl)methyl)-4-methoxyisoxazolo[4,5-c]pyridin-3-yl)-6-cyclopropyl-3,4-dihydro-1H-benzopyran-8-sulfonamide

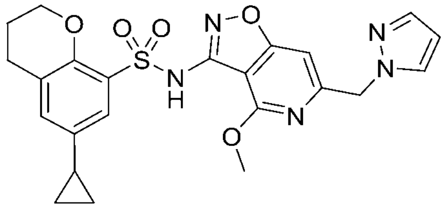

**1-106**

Synthetic route:

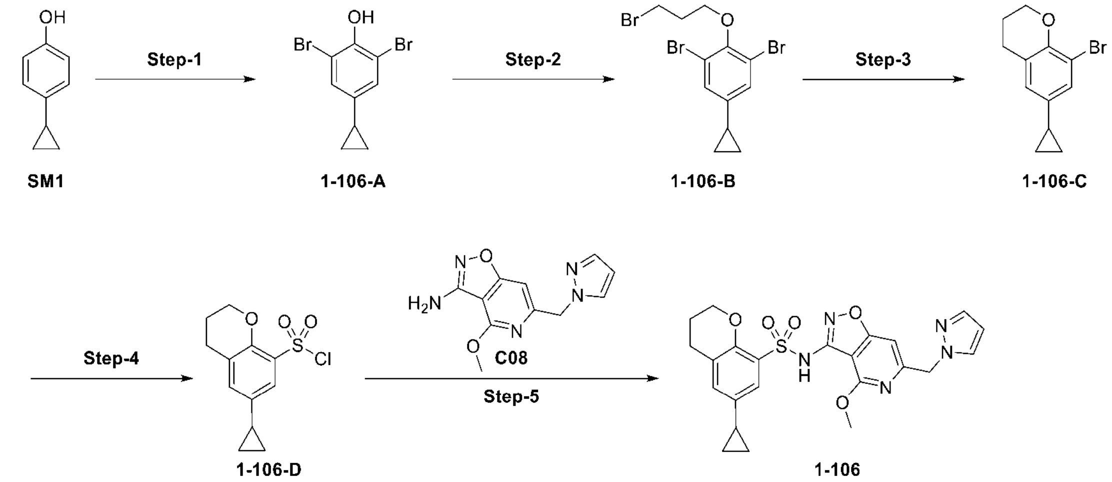


Step-1: Preparation of compound 1-106-A: SM1 (5 g, 1 eq) was dissolved in acetonitrile (150 mL), and *N*-bromosuccinimide (15 g, 2.2 eq) was added in batches at room temperature. Under nitrogen atmosphere, the mixture was stirred at room temperature for 16 h. The reaction system was placed in an ice bath and cooled to 0 °C. Then, the reaction system was quenched with a saturated aqueous sodium bisulfite solution and extracted 3 times with ethyl acetate. The organic phases were combined, washed with a saturated aqueous sodium chloride solution, dried over anhydrous sodium sulfate, subjected to suction filtration, and concentrated by rotary evaporation. The resulting crude product was purified by normal phase column chromatography to give compound 1-106-A (2.4 g, yield: 22.2%). $^1$H NMR (400 MHz, $CDCl_3$-*d*) δ (ppm): 7.19 (s, 2H), 1.85-1.79(m, 1H), 0.98-0.89 (m, 2H), 0.64-0.61(m, 2H).

Step-2 to Step-4: Preparation of compound 1-106-D: Compound 1-106-D was synthesized according to the procedures of Step-1 to Step-3 for compound D-31, with 1-106-A as a starting material. $^1$H NMR (400 MHz, $CDCl_3$-*d*) δ (ppm): 7.48 (d, *J*=1.6Hz, 1H), 7.12 (d, *J*=1.6Hz 1H), 4.44-4.41 (m, 2H), 2.87-2.84 (m, 2H), 2.14-2.11 (m, 2H), 1.89-1.87 (m, 1H), 1.01-0.97 (m, 2H), 0.69-0.65 (m, 2H).

Step-5: Preparation of compound 1-106: According to the procedure of Step-1 for 1-1, a mixture of the key intermediate C08 and 1-106-D was subjected to a condensation reaction, and the reaction mixture was purified by high-pressure preparative liquid chromatography to give compound 1-106. $[M+1]^+$: 482.30. $^1$H NMR (400 MHz, DMSO-$d_6$) δ (ppm):10.51 (s, 1H), 7.90 (d, *J*=2.0Hz, 1H), 7.52 (d, *J*=1.2Hz, 1H), 7.34 (d, *J*=2.0Hz, 1H), 6.99 (s, 1H), 6.70 (s, 1H), 6.34-6.33 (m, 1H), 5.45 (s, 2H), 4.04-4.02 (m, 2H), 3.94 (s, 3H), 2.72-2.69 (m, 2H), 1.92-1.89 (m, 1H), 1.87-1.78 (m, 2H), 0.91-0.88 (m, 2H), 0.67-0.65 (m, 2H).

Example 107

Compound 1-107: *N*-(6-((1H-pyrazol-1-yl)methyl)-4-methoxyisoxazolo[4,5-c]pyridin-3-yl)-6-cyano-3,4-dihydro-1H-benzopyran-8-sulfonamide

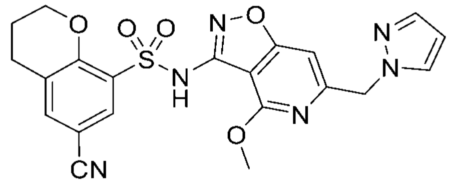

**1-107**

Synthetic route:

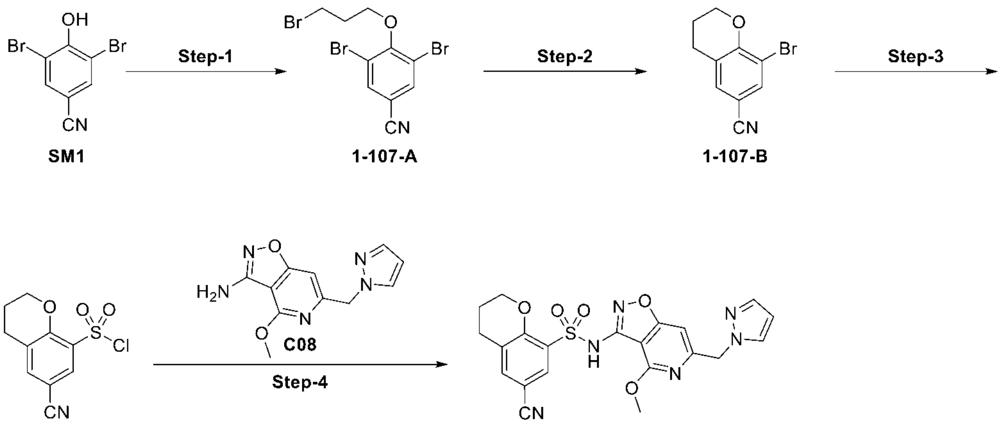


SM1 1-107-A 1-107-B 1-107-C C08 1-107

Step-1 to Step-3: Preparation of compound 1-107-C: Compound 1-107-C was synthesized according to the procedures of Step-1 to Step-3 for compound D-31, with SM1 as a starting material. $^1$H NMR (400 MHz, $CDCl_3$-*d*) δ (ppm): 8.10 (d, *J* =2.0Hz, 1H), 7.66 (d, *J* =2.0Hz, 1H), 4.59-4.56 (m, 2H), 2.97-2.91 (m, 2H), 2.22-2.16 (m, 2H). Step-4: Preparation of compound 1-107: A mixture of the key intermediate C08 and 1-107-C was subjected to a condensation reaction, and the reaction mixture was purified by high-pressure preparative liquid chromatography to give compound 1-107. $[M+1]^+$: 467.15. $^1$H NMR (400 MHz, DMSO-$d_6$) δ (ppm): 7.90 (d, *J*=1.6 Hz, 1H), 7.86 (d, *J*=2.0 Hz, 1H), 7.61 (s, 1H), 7.49 (d, *J*=1.6 Hz, 1H), 6.52 (s, 1H), 6.32-6.31 (m, 1H), 5.37 (s, 2H), 4.18-4.16 (m, 2H), 3.92 (s, 3H), 2.77-2.74 (m, 2H), 1.87-1.82 (m, 2H).

Example 108

Compound 1-108: *N*-(6-((1H-pyrazol-1-yl)methyl)-4-methoxyisoxazolo[4,5-c]pyridin-3-yl)-6-methoxybenzofuran-5-sulfonamide

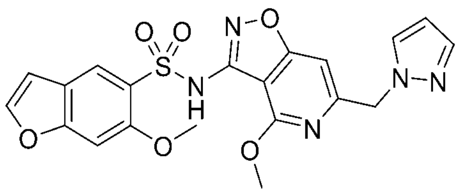

**1-108**

Synthetic route:

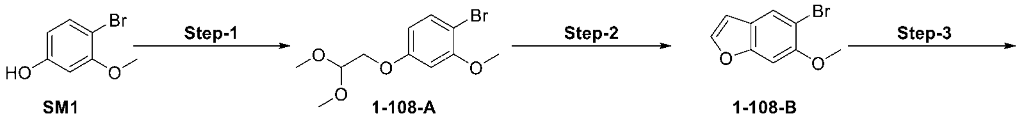


SM1 1-108-A 1-108-B

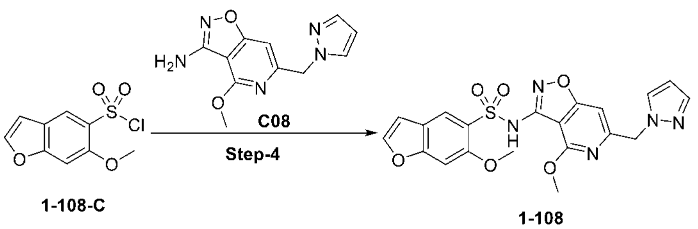


1-108-C 1-108

Step-1 to Step-3: Preparation of compound 1-108-C: Compound 1-108-C was prepared according to a preparation method similar to that for D-15, with SM1 as a starting material. $^1$H NMR (400 MHz, $CDCl_3$-*d*) δ (ppm): 8.28 (s, 1H), 7.70 (s, 1H), 7.24 (s, 1H), 6.85 (s, 1H), 4.12 (s, 3H).

Step-4: Preparation of compound 1-108: According to the procedure of Step-1 for 1-1, a mixture of the key intermediate C08 and 1-108-C was subjected to a condensation reaction, and the reaction mixture was purified by high-pressure preparative liquid chromatography to give compound 1-108. $[M+1]^+$: 456.15. $^1$H NMR (400 MHz, DMSO-$d_6$) δ (ppm): 8.13 (s, 1H), 7.95 (s, 1H), 7.87 (d, *J*=2.0 Hz, 1H), 7.50 (d, *J*=1.6 Hz, 1H), 7.39 (s, 1H), 6.99 (s, 1H), 6.63-6.61 (m, 1H), 6.32-6.31 (m, 1H), 5.41 (s, 2H), 3.93 (s, 3H), 3.81 (s, 3H).

**Pharmacological Experiment I: Enzymatic Activity Assay for Acetyltransferase**

1.1 Enzymatic activity assay for KAT6A acetyltransferase

The acetylation effect of KAT6A on histone substrate H3 was determined using the TR-FRET (time-resolved fluorescence resonance energy transfer) method.

1.2 Preparation of compounds

1) The compounds prepared in the examples of the present disclosure were each dissolved in dimethylsulfoxide to give solutions at a concentration of 10 mmol/L, and then the solutions were diluted to 0.2 mmol/L, i.e., 1 μL of the compound was added to 49 μL of DMSO, and 3-fold dilution was performed to give 10 concentrations.
2) 50 nL of the test compounds (example compounds) were transferred to 384 reaction plates using Echo, two replicates for each compound, and the plates were centrifuged at a rotation speed of 1000 rpm for 1 min, the final concentration of dimethylsulfoxide (DMSO) being 0.5%.
3) 2.5 μL of the reaction mixtures obtained in step 2 was added to the 384 reaction plates in step 2), and the plates were centrifuged at 1000 rpm for 1 min and incubated at 25 °C for 10 min.
4) 2.5 μL of Bio-H3&AcCOA (acetyl-CoA) reaction solution was added to the 384 reaction plates in step 3), and the plates were centrifuged at a rotation speed of 1000 rpm for 1 min and incubated at 25 °C for 90 min.
5) 5 μL of Eu-Ab& Ulight-SA detection solution was added to the 384 reaction plates in step 4), and the plates were centrifuged at 1000 rpm for 1 min and incubated at 25 °C for 60 min.
6) The HTRF 665 nM/615 nM signals were read using a BMG high-throughput drug-screening multi-mode microplate reader.

1.3 Data analysis

The acetylation level of the histone H3 was analyzed by the GraphPad Prism 8 software. The reading of the negative control (0.5% DMSO wells) was set as 0% inhibition rate, and the reading of the positive control (wells with the highest concentration of the control compound) was set as 100% inhibition rate. The inhibition rates were calculated, and then $IC_{50}$ values (half maximal inhibitory concentration) of test compounds were obtained by using a software nonlinear fitting formula;

$$\text{Compound inhibition rate (\%)}=\left[\frac{\overline{\text{Data}}_{\text{negative control}}\text{-Data}_{\text{compound}}}{\overline{\text{Data}}_{\text{negative control}}\text{-}\overline{\text{Data}}_{\text{positive control}}}\right]\times 100\%$$

$\overline{\text{Data}}_{\text{positive control}}$: mean of positive control well values; $\overline{\text{Data}}_{\text{negative control}}$: mean of negative control well values. The test results are shown in Table 3.

Table 3. Enzymatic inhibitory activity of example compounds

| Sample | $IC_{50}$ (nmol/L) | Sample | $IC_{50}$ (nmol/L) | Sample | $IC_{50}$ (nmol/L) |
|---|---|---|---|---|---|
| Example 1 | 7.33 | Example 45 | 9.45 | Example 68 | 2.41 |
| Example 2 | 0.72 | Example 46 | 1.67 | Example 69 | 0.87 |
| Example 3 | 1.76 | Example 47 | 4.67 | Example 70 | 0.85 |
| Example 4 | 1.51 | Example 48 | 22.67 | Example 71 | 4.49 |
| Example 5 | 4.52 | Example 49 | 67.82 | Example 72 | 5.10 |
| Example 6 | 3.11 | Example 50 | 8.67 | Example 73 | 0.97 |
| Example 7 | 5.68 | Example 51 | 18.23 | Example 74 | 1.77 |
| Example 9 | 0.92 | Example 52 | 34.32 | Example 75 | 2.41 |
| Example 10 | 0.38 | Example 53 | 4.56 | Example 76 | 1.05 |
| Example 11 | 0.77 | Example 54 | 110 | Example 77 | 1.91 |
| Example 12 | 66.67 | Example 55 | 8.79 | Example 78 | 7.97 |
| Example 17 | 8.67 | Example 56 | 16.33 | Example 83 | 4.12 |
| Example 18 | 7.23 | Example 57 | 0.95 | Example 84 | 1.21 |
| Example 21 | 75.89 | Example 58 | 0.66 | Example 86 | 10.03 |
| Example 26 | 21.78 | Example 59 | 0.95 | Example 87 | 2.67 |
| Example 28 | 3.55 | Example 60 | 1.01 | Example 89 | 16.78 |
| Example 29 | 1.57 | Example 62 | 0.84 | Example 90 | 7.18 |
| Example 31 | 68.56 | Example 63 | 5.98 | Example 91 | 17.59 |
| Example 32 | 34.68 | Example 64 | 8.85 | Example 94 | 2.37 |
| Example 33 | 3.13 | Example 65 | 2.01 | Example 95 | 1.63 |
| Example 34 | 12.58 | Example 66 | 1.22 | Example 96 | 6.67 |
| Example 44 | 8.28 | Example 67 | 1.31 | Example 97 | 0.95 |
| Example 98 | 0.84 | Example 102 | 0.83 | Example 106 | 0.94 |
| Example 99 | 2.79 | Example 103 | 1.86 | Example 107 | 5.86 |
| Example 100 | 1.06 | Example 104 | 1.77 | Example 108 | 1.26 |
| Example 101 | 2.04 | Example 105 | 2.18 | | |

The reagent information used in the experiment is shown in Table 4:

Table 4. Reagent information used in the experiment

| Material | Manufacturer | Product number |
|---|---|---|
| KAT6A | active motife | 81223 |
| AcCOA | sigma | A2056 |
| Bio-H3(1-21) | GenScript | / |
| Eu-Ab | Perkin Elmer | TRF0412-M |

(continued)

| Material | Manufacturer | Product number |
|---|---|---|
| Ulight-SA | Perkin Elmer | TRF0102-M |
| LANCE™ Detection buffer(10x) | Perkinelmer | CR97-100 |

**Pharmacological Experiment II: Enzymatic Activity Assay for Other Acetyltransferase Subtypes**

1.1 Enzymatic activity assay for KAT6B, KAT5, KAT7, and KAT8 acetyltransferases

The acetylation effects of KAT6B on histone substrate H3, as well as KAT5, KAT7, and KAT8 on histone substrate H4, were determined using the TR-FRET (time-resolved fluorescence resonance energy transfer) method. The determination was performed according to experimental procedures similar to those in "Pharmacological Experiment I":
1.2 Preparation of compounds: The compounds prepared in the examples were each dissolved in dimethylsulfoxide to give solutions at a concentration of 10 mmol/L.

1) The compounds in the examples were each diluted from 10 mmol/L stock solution to 0.2 mmol/L, i.e., 1 μL of the compound was added to 49 μL of DMSO, and 3-fold dilution was performed to give 10 concentrations.
2) 50 nL of the test compounds were transferred to 384 reaction plates using Echo, two replicates for each compound, and the plates were centrifuged at a rotation speed of 1000 rpm for 1 min, the final concentration of dimethylsulfoxide (DMSO) being 0.5%.

For KAT6B:

3) 2.5 μL of the KAT6B enzyme solution at a final concentration of 3 nM was added to the 384 reaction plates in step 2), and the plates were centrifuged at 1000 rpm for 1 min and incubated at 25 °C for 10 min.
4) 2.5 μL of Bio-H3 at a final concentration of 400 nM and an AcCOA (acetyl-CoA) reaction solution at a final concentration of 2 μM were added to the 384 reaction plates in step 3), and the plates were centrifuged at a rotation speed of 1000 rpm for 1 min and incubated at 25 °C for 90 min.

For KAT5:

3) 2.5 μL of the KAT5 enzyme solution at a final concentration of 1.5 nM was added to the 384 reaction plates in step 2), and the plates were centrifuged at 1000 rpm for 1 min and incubated at 25 °C for 10 min.
4) 2.5 μL of H4 at a final concentration of 200 nM and an AcCOA (acetyl-CoA) reaction solution at a final concentration of 2 μM were added to the 384 reaction plates in step 3), and the plates were centrifuged at a rotation speed of 1000 rpm for 1 min and incubated at 25 °C for 90 min.

For KAT7:

3) 2.5 μL of the KAT7 enzyme solution at a final concentration of 15 nM was added to the 384 reaction plates in step 2), and the plates were centrifuged at 1000 rpm for 1 min and incubated at 25 °C for 10 min.
4) 2.5 μL of H4 at a final concentration of 300 nM and an AcCOA (acetyl-CoA) reaction solution at a final concentration of 2 μM were added to the 384 reaction plates in step 3), and the plates were centrifuged at a rotation speed of 1000 rpm for 1 min and incubated at 25 °C for 90 min.

For KAT8:

3) 2.5 μL of the KAT8 enzyme solution at a final concentration of 10 nM was added to the 384 reaction plates in step 2), and the plates were centrifuged at 1000 rpm for 1 min and incubated at 25 °C for 10 min.
4) 2.5 μL of Bio-H3 at a final concentration of 300 nM and an AcCOA (acetyl-CoA) reaction solution at a final concentration of 2 μM were added to the 384 reaction plates in step 3), and the plates were centrifuged at a rotation speed of 1000 rpm for 1 min and incubated at 25 °C for 90 min.
5) 5 μL of Eu-Ab& Ulight-SA detection solution was separately added to the 384 reaction plates in step 4) of the 4 groups described above, and the plates were centrifuged at 1000 rpm for 1 min and incubated at 25 °C for 60 min.
6) The HTRF 665 nM/615 nM signals were read using a BMG high-throughput drug-screening multi-mode microplate reader.

1.3 Data analysis

The acetylation levels of the histones in the groups were analyzed by the GraphPad Prism 8 software. The reading of the negative control (0.5% DMSO wells) was set as 0% inhibition rate, and the reading of the positive control (wells with the highest concentration of the control compound) was set as 100% inhibition rate. The inhibition rates were calculated, and then $IC_{50}$ values (half maximal inhibitory concentration) of test compounds were obtained by using a software nonlinear fitting formula;

$$\% \text{ Compound inhibition rate} = \left[\frac{\overline{\text{Data}}_{\text{negative control}} - \text{Data}_{\text{compound}}}{\overline{\text{Data}}_{\text{negative control}} - \overline{\text{Data}}_{\text{positive control}}}\right] \times 100\%$$

$\overline{\text{Data}}_{\text{positive control}}$: mean of positive control well values; $\overline{\text{Data}}_{\text{negative control}}$: mean of negative control well values. The test results are shown in Table 5.

Table 5. Enzymatic inhibitory activity of example compounds

| Sample | KAT6B $IC_{50}$ (nmol/L) | KAT5 $IC_{50}$ (nmol/L) | KAT7 $IC_{50}$ (nmol/L) | KAT8 $IC_{50}$ (nmol/L) |
|---|---|---|---|---|
| Example 2 | 2.34 | 219.3 | 54.03 | 117.7 |
| Example 9 | / | / | 47.5 | / |
| Example 10 | 1.78 | 66.38 | 7.42 | 79.42 |
| Example 59 | 9.50 | 170.9 | 27.4 | 327.1 |
| Example 76 | / | / | 86.59 | / |

The reagent information used in the experiment is shown in Table 6:

Table 6. Reagent information used in the experiment

| **Material** | **Manufacturer** | **Product number** |
|---|---|---|
| KAT6B | active motife | 81224 |
| KAT5 | signalChem | K314-380G |
| KAT7 | ICE | S02210F-H21HF |
| KAT8 | active motife | 81225 |
| AcCOA | sigma | A2056 |
| Bio-H3(1-21) | GenScript | / |
| H4(1-25) | GenScript | / |
| Eu-Ab | Perkin Elmer | TRF0412-M |
| Ulight-SA | Perkin Elmer | TRF0102-M |
| LANCE™ Detection buffer(10x) | Perkinelmer | CR97-100 |

**Pharmacological Experiment III: Assay for Cell Growth Inhibitory Activity**

1.1 Cell culture

The human breast cancer cell line ZR-75-1 (1 × 10^6 cells per cell culture dish with 100-mm diameter) was cultured in a 1640 culture medium, and 1% double antibiotics (streptomycin and penicillin), 10% fetal bovine serum (FBS), and 1% glutamine (Glumax) were added simultaneously. The cell culture dish was placed at 37 °C with 5% $CO_2$ for 2 days for culture until the cell confluence reached 80%-90% to give cells in the logarithmic growth phase.

1.2 Preparation of compounds:

1) The example compounds were each dissolved in dimethylsulfoxide to give solutions at a concentration of 10 mmol/L, and the solutions were diluted to 250 μmol/L in 10-fold gradient for 5 concentrations, i.e., 0.2 nmol/L, 2 nmol/L, 20 nmol/L, 200 nmol/L, and 2000 nmol/L.
2) Blank control wells were prepared by adding 0.1% dimethylsulfoxide to the cultured cells, and were used as high-reading control wells; cell-free 3D hydrogel was used as background wells.
3) The cultured cells were separately added to the compounds in the examples at 0.2 nmol/L, 2 nmol/L, 20 nmol/L, 200 nmol/L, and 2000 nmol/L as test wells, with the same volumes as the original culture solutions.

1.3 Cell plating

1) The cells were collected, resuspended in a PBS balanced salt solution, and counted; and the cells were mixed with the 3D hydrogel stock solution and prepared into hydrogel cell suspensions with proper density.
2) The 3D hydrogel cell suspension (5 × 10^4 cells) was added to each well in 96-well cell culture plates with low surface adsorption, and the plates were incubated at 37 °C with 5% $CO_2$ for 20 min.
3) The complete culture medium containing the compound prepared in 1.2 with an equal volume to that of the cell suspension in step 2 was added to the 96-well cell culture plate.
4) The cells were cultured at 37 °C in a 5% $CO_2$ incubator for 96 h.

1.4 Cell assay

1) The complete culture medium in the upper layer above the hydrogel was pipetted from each well of the 96-well cell culture plate with a vacuum pump or a manual pipette with careful processing to avoid breaking the hydrogel lower layer.
2) The complete culture medium containing the CCK-8 substrate with an equal volume to that of the original hydrogel was added to each well.
3) The plate was cultured at 37 °C with 5% $CO_2$ for 1 h.
4) The 96-well cell culture plate was placed in a microplate reader, and the values of A450 and A630 were separately detected.

Data analysis: The relative inhibitory level was analyzed by using Excel software, and the survival rates of cells under different compound culture conditions were obtained;

Survival rate (%) = (example compound well reading-background well reading)/(high-reading control well reading-background well reading) × 100

High-reading control well: cells + dimethylsulfoxide; background well: cell-free hydrogel. The test results are shown in Table 7.

Table 7. Cell proliferation inhibitory activity of some of example compounds

| Sample | Blank | Cell survival rate at example drug concentration | | | | |
|---|---|---|---|---|---|---|
| | DMSO(%) | 0.1 nM(%) | 1 nM(%) | 10 nM(%) | 100 nM(%) | 1000 nM(%) |
| Example 1 | 100 | 95 | 80 | 54 | 40 | 30 |
| Example 2 | 100 | 63 | 51 | 43 | 43 | 40 |
| Example 3 | 100 | 71 | 55 | 43 | 40 | 29 |
| Example 4 | 100 | 75 | 61 | 41 | 37 | 29 |
| Example 5 | 100 | 80 | 85 | 69 | 54 | 35 |
| Example 6 | 100 | 74 | 66 | 45 | 38 | 25 |
| Example 9 | 100 | 63 | 48 | 41 | 38 | 32 |
| Example 10 | 100 | 62 | 45 | 39 | 33 | 29 |

As can be seen from Tables 3 and 5: The compounds in the present disclosure have relatively high cell proliferation inhibitory activity and can effectively inhibit the proliferation of the breast cancer cells ZR-75-1, indicating that the compounds in the present disclosure can well treat or prevent KAT6A-dependent cancers.

**Pharmacological Experiment IV: Assay for Plasma Protein Binding Rate**

1.1 Preparation of compounds: The compounds prepared in the examples were each dissolved in dimethylsulfoxide to give solutions at a concentration of 10 mmol/L.

1) The example compounds were each diluted from 10 mmol/L stock solution to 0.2 mmol/L, i.e., 1 μL of the compound was added to 49 μL of DMSO;
2) 3 μL of 0.2 mmol/L test compounds were added to 597 μL of blood plasma, and the mixtures were vortexed at 1000 rpm for 2 min;
3) 50 μL of the plasma containing the drug in 2) was taken, 50 μL of PBS was added, and 300 μL of methanol internal standard* was added to serve as a sample T0;
4) 120 μL of the plasma containing the drug in 2) was taken and added to one side of an HTD device membrane, and 120 μL of PBS was added to the other side of the membrane; a breathable film was covered, and the plasma and the residual sample in 2) were balanced at 37 °C (100 rpm and 5% $CO_2$) for 6 h;
5) 50 μL of the dialyzed plasma side sample was taken, 50 μL of PBS was added, and 300 μL of methanol internal standard* was added to serve as a sample P;
6) 50 μL of the dialyzed PBS side sample was taken, 50 μL of blank plasma was added, and 300 μL of methanol internal standard* was added to serve as a sample B;
7) 50 μL of the plasma containing the drug in 2) was taken, 50 μL of PBS was added, and 300 μL of methanol internal standard* was added to serve as a sample T6;
8) All samples were vortexed, mixed homogeneously, and then centrifuged at 3220 g for 40 min at 4 °C;
9) 100 μL of the supernatant and 100 μL of purified water were mixed homogenously for liquid chromatography-mass spectrometry.

Methanol internal standard* indicates that: the appropriate internal standard and concentration can be selected according to the property of the test substance.

1.2 Data analysis

An internal standard method was used, and the plasma protein binding rate, recovery rate, and residual percentage were calculated by using Excel according to the ratio of the peak area of the sample to the peak area of the internal standard.

$$\text{Free rate (\%)} = \frac{Ratio_B}{Ratio_P} \times 100\%$$

$$\text{Binding rate (\%)} = 100\ \% - \text{Free rate\%}$$

$$\text{Recovery rate (\%)} = \frac{Ratio_B + Ratio_P}{Ratio_{T0}} \times 100\%$$

$$\text{Remaining percentage (\%)} = \frac{Ratio_{T6}}{Ratio_{T0}} \times 100\%$$

*$Ratio_B$:* a ratio of the peak area of the test substance on the PBS side to the peak area of the internal standard; *$Ratio_P$:* a ratio of the peak area of the test substance on the plasma side to the peak area of the internal standard; *$Ratio_{T6}$:* a ratio of the peak area of the sample after incubation with the test substance for 6 h to the peak area of the internal standard; *$Ratio_{T0}$:* a ratio of the peak area of the sample after incubation with the test substance for 0 h to the peak area of the internal standard. The data obtained are shown in Table 8.

Table 8. Plasma protein binding rate (Fu%) data for various species of example compounds

| Sample | Human (Fu%) | Mouse (Fu%) | Rat (Fu%) | Dog (Fu%) |
|---|---|---|---|---|
| Example 2 | 0.99 | 0.65 | 1.67 | 0.13 |
| Example 9 | 0.69 | 4.78 | 2.57 | 2.50 |
| Example 10 | 0.73 | 5.12 | 1.32 | 1.45 |
| Example 28 | 0.86 | 2.05 | / | / |
| Example 44 | 1.49 | 4.80 | 1.76 | 0.23 |
| Example 45 | 0.66 | 1.45 | / | / |
| Example 57 | 2.64 | 5.65 | / | / |
| Example 59 | 2.48 | 5.00 | 4.14 | 1.43 |
| Example 60 | 1.75 | 3.58 | 5.16 | 3.45 |
| Example 75 | 0.91 | 1.63 | / | / |
| Example 76 | 1.18 | 0.72 | 1.44 | 0.65 |

As can be seen from the data in Table 8, the plasma protein binding rates of the compounds in the present disclosure exhibited relatively low differences in various species, indicating that the druggability of the compounds in the present disclosure is relatively high.

**Pharmacological Experiment V: *In-Vivo* Pharmacokinetic Study in Rats**

1.1 Instrument: high-performance liquid chromatograph: SHIMADZU LC-30AD; mass spectrum: AB SCIEX mass spectrometer Triple Quad 5500; all measured data were calculated and processed using Microsoft Excel, and relevant pharmacokinetic parameters were calculated using WinNonlin software. The main obtained kinetic parameters are $T_{max}$, $T_{1/2}$, $C_{max}$, $AUC_{0\text{-}24\,h}$, and $AUC_{inf}$. Chromatographic column: XSelect Hss T3 2.5 μm (2.1 × 50 mm) Column XP; column temperature: 40 °C; mobile phase A: water (0.1% formic acid); mobile phase B: acetonitrile; flow rate: 0.60 mL/min; gradient elution; elution gradient: 0.30 min: 5% B; 1.00 min: 98% B; 1.48 min: 98% B; 1.51 min: 5% B; 2.00 min: stop. Sample injection amount: 1 μL.

1.2 Animals: three SD female rats were used. The body weight range was 180-300 g. After purchase, the rats were bred in the experimental animal center for two days for later use. The rats were fasted 12 h before administration and 4 h after administration, and given *ad libitum* access to water during the assay. Blood samples were taken at predetermined time points after intragastric administration.

1.3 Vehicle: 0.5% HPMC (hydroxypropyl methylcellulose) + 0.4% Tween80 + 99.1% water. Preparation of intragastric administration solution: The compound was precisely weighed, the vehicle was added, and then the drug was completely dissolved by ultrasonication at room temperature for 5 min and prepared into a 0.5 mg/mL solution. After the intragastric administration, blood samples were taken at 0.083 h, 0.25 h, 0.5 h, 1.0 h, 2.0 h, 4.0 h, 7.0 h, and 24 h. The blood collection mode is as follows: 0.2 mL of the jugular vein blood was collected, EDTA-$K_2$ was used as an anticoagulation agent, the collected blood sample was transferred into a microcentrifuge tube containing the anticoagulation agent, and the tube was centrifuged at 4 °C and 4000 g for 5 min to separate the plasma. All plasma samples collected were stored at -75±15 °C until analysis.

The compound was precisely weighed and prepared into a standard curve working solution and a quality control working solution at different concentrations. The blank plasma was added and prepared into a plasma standard curve and a quality control sample. LC/MS/MS analysis was performed after pretreatment using a protein precipitation method, and then the concentration of the compounds described above in the plasma was measured. All measured data were collected and processed by the relevant software, and pharmacokinetic parameters were calculated using Winnonlin software. The kinetic parameters of some representative compounds are shown in Table 9.

Table 9. Pharmacokinetic parameters of some compounds in the examples in rats

| Example | Dose mg/kg | $T_{1/2}$ (h) | $T_{max}$ (h) | $C_{max}$ (ng/mL) | $AUC_{0\text{-}24h}$ (h*ng/mL) | $AUC_{inf}$ (h*ng/mL) |
|---|---|---|---|---|---|---|
| Example 2 | 5 | 6.85 | 6.00 | 4407 | 64774 | 71528 |
| Example 4 | 5 | 3.75 | 0.25 | 5955 | 11819 | 12002 |
| Example 5 | 5 | 5.81 | 0.25 | 5956 | 18474 | 19694 |

(continued)

| Example | Dose mg/kg | $T_{1/2}$ (h) | $T_{max}$ (h) | $C_{max}$ (ng/mL) | $AUC_{0-24h}$ (h*ng/mL) | $AUC_{inf}$ (h*ng/mL) |
|---|---|---|---|---|---|---|
| Example 6 | 5 | 24.60 | 0.33 | 31631 | 501526 | 1298674 |
| Example 9 | 5 | 2.74 | 1.83 | 22500 | 214572 | 215211 |
| Example 10 | 5 | 4.08 | 0.50 | 10534 | 72280 | 74334 |
| Example 11 | 5 | 2.08 | 0.83 | 3311 | 32006 | 32016 |
| Example 59 | 5 | 2.75 | 1.17 | 16500 | 166222 | 166662 |
| Example 60 | 5 | 4.27 | 2.67 | 2307 | 26155 | 26708 |
| Example 62 | 5 | 9.77 | 1.17 | 25577 | 356464 | 449535 |
| Example 75 | 5 | 2.29 | 0.25 | 10830 | 30570 | 30597 |
| Example 76 | 5 | 4.60 | 2.33 | 14435 | 157672 | 162006 |

As can be seen from the data in Table 9, pharmacokinetic studies in rats showed that the sulfonamide compounds of the present disclosure had relatively high exposure levels after oral intragastric administration and good oral absorption. In particular, compared with the existing known compounds, the compounds in Examples 2, 4, 5, 9, 10, 11, 59, 60, 62, 75, and 76 have more excellent pharmacokinetic parameters, that is, the $T_{1/2}$ is within 10 h while the compounds have better biological exposure levels, so that the compounds are more suitable for once-a-day administration and have less *in-vivo* accumulation, thereby effectively reducing the safety risk of such compounds caused by high accumulation.

**Pharmacological Experiment VI: *In-Vivo* Pharmacokinetic Study in Beagle Dogs**

1.1 Instrument: high-performance liquid chromatograph: SHIMADZU LC-30AD; mass spectrum: AB SCIEX mass spectrometer Triple Quad 5500; all measured data were calculated and processed using Microsoft Excel, and relevant pharmacokinetic parameters were calculated using WinNonlin software. The main obtained kinetic parameters are $T_{max}$, $T_{1/2}$, $C_{max}$, $AUC_{0-24\ h}$, and $AUC_{inf}$. Chromatographic column: Agilent EC-C18 2.7 μm (50 × 2.1 mm); column temperature: 40 °C; mobile phase A: water (0.1% formic acid); mobile phase B: acetonitrile; flow rate: 0.60 mL/min; gradient elution; elution gradient: 0.30 min: 5% B; 1.90 min: 95% B; 2.20 min: 95% B; 2.21 min: 5% B; 2.50 min: stop. Sample injection amount: 10 μL.

1.2 Animals: 3 male beagle dogs were used. The body weight range was 8-15 kg. The beagle dogs were fasted 12 h before administration and 4 h after administration, and given *ad libitum* access to water during the assay.

1.3 Vehicle: 0.5% HPMC (hydroxypropyl methylcellulose) + 0.4% Tween80 + 99.1% water.

After the intragastric administration, blood samples were taken at 0.25 h, 0.5 h, 1 h, 2 h, 4 h, 6 h, 8 h, 10 h, 12 h, 24 h, and 48 h. The blood collection mode is as follows: venipuncture was used, EDTA-$K_2$ was used as an anticoagulation agent, the collected blood sample was transferred into a microcentrifuge tube containing the anticoagulation agent, and the tube was centrifuged at 4 °C and 2000 g for 10 min to separate the plasma. All plasma samples collected were stored at -75±15 °C until analysis.

1.4 Plasma sample pretreatment: 50 μL of the plasma sample was taken, and 200 μL of an internal standard solution prepared with acetonitrile was added for precipitation; subsequently, the sample was vortexed for 1 min and mixed homogenously; the precipitated sample was centrifuged at 4 °C and 3900 rpm for 15 min; the supernatant was pipetted, and water was added for 3-fold dilution; 10 μL of the sample was injected; and quantitative analysis was performed by adopting a liquid chromatography-mass spectrometry technology.

All measured data were collected and processed by the relevant software, and pharmacokinetic parameters were calculated using Winnonlin software. The kinetic parameters of some representative compounds are shown in Table 10:

Table 10. Pharmacokinetic parameters of some compounds in the examples in beagle dogs

| Example | Dose | $T_{1/2}$ (h) | $T_{max}$ (h) | $C_{max}$ | $AUC_{0-48h}$ | $AUC_{inf}$ |
|---|---|---|---|---|---|---|
| | mg/kg | | | (ng/mL) | (h*ng/mL) | (h*ng/mL) |
| Example 9 | 1 | 12.5 | 1.33 | 9230 | 113893 | 122082 |
| Example 2 | 0.6 | 173.0 | 4.00 | 1803 | 66522 | 379089 |
| Example 59 | 1 | 7.6 | 0.67 | 8383 | 55469 | 55894 |

(continued)

| Example | Dose | $T_{1/2}$ (h) | $T_{max}$ (h) | $C_{max}$ | $AUC_{0-48h}$ | $AUC_{inf}$ |
|---|---|---|---|---|---|---|
| | mg/kg | | | (ng/mL) | (h*ng/mL) | (h*ng/mL) |
| Example 10 | 1 | 9.6 | 0.83 | 6247 | 46698 | 47546 |
| Example 76 | 1 | 45.4 | 2 | 14050 | 225295 | 749234 |

As can be seen from the data in Table 10, pharmacokinetic studies in beagle dogs showed that the sulfonamide compounds of the present disclosure had relatively high exposure levels after oral intragastric administration and good oral absorption. In particular, compared with the existing known compounds, the compounds of Examples 9, 10, and 59 have more excellent pharmacokinetic parameters, that is, the $T_{1/2}$ is within 15 h while the compounds have better biological exposure levels, so that the compounds are more suitable for once-a-day administration and have less *in-vivo* accumulation, thereby effectively reducing the safety risk of such compounds caused by high accumulation.

**Pharmacological Experiment VII: Inhibitory Effect on P450 Enzymes *In Vitro***

1.1 Preparation of compounds: The compounds prepared in the examples were each dissolved in dimethylsulfoxide to give solutions at a concentration of 2 mmol/L.

1.2 Test methods:

1) Incubation system: human liver microsomes, a $MgCl_2$ solution, a phosphate buffer solution, and a substrate solution were each added a 96-well deep-well plate and mixed homogenously. In the final incubation system, the concentration of the human liver microsomes was 0.2 mg/mL, and the final concentrations of the $MgCl_2$ solution and the phosphate buffer solution were 5 mM and 100 mM, respectively.
2) 1 μL of the substrate solution was added to the incubation system, and the final concentrations of CYP1A2 phenacetin, 2C8 paclitaxel mephenytoin, 2C19 mephenytoin, 2D6 midazolam, and 3A midazolam substrates were 40 μM (phenacetin), 5 μM (paclitaxel), 50 μM (mephenytoin), 10 μM (mephenytoin), 5 μM (midazolam), and 50 μM (midazolam), respectively.
3) 1 μL of the example compound or vehicle (DMSO) was added to the incubation system, and the incubation system was pre-warmed for 5 min in a 37 °C water bath before starting the reaction with 20 μL of 10 mM NADPH solution.

1.3 Sample treatment: Within the designated time (20 min for phenacetin, 10 min for paclitaxel, 20 min for mephenytoin, 20 min for dextromethorphan, 5 min for midazolam, and 10 min for testosterone), 400 μL of methanol containing the internal standard was added to terminate the reaction, and the mixture was vortexed and mixed homogeneously. Then, the deep-well plate was centrifuged at 3220 g and 4 °C for 40 min. 100 μL of the supernatant was transferred into a new plate, and 150 μL of purified water was added. The mixture was mixed homogenously for LC-MS/MS analysis.

Table 11. Inhibitory effects of some compounds in the examples on CYP enzymes

| Example | Final concentration (μM) | % inhibition rate | | | | | |
|---|---|---|---|---|---|---|---|
| | | CYP1A2 (phenacetin) | CYP2C8 (paclitaxel) | CYP2C19 (mephenytoin) | CYP2D6 (dextromethorphan) | CYP3A (midazolam) | CYP3A (testosterone) |
| Example 2 | 10 | 6.02 | 16.41 | 42.83 | 5.38 | -5.22 | 5.84 |
| Example 10 | 10 | -1.78 | 3.71 | 9.54 | 6.03 | -4.28 | -2.93 |
| Example 59 | 10 | 1.93 | 2.08 | 7.62 | 4.62 | -12.05 | -7.66 |

As can be seen from the data in Table 11, the example compounds all showed relatively weak inhibitory effect on CYP enzymes, with $IC_{50}$ greater than 10 μM.

**Pharmacological Experiment VIII: hERG Channel Assay**

1.1 Test methods: This experiment adopted a manual patch clamp technology to detect the blocking effect of the compounds on electric current in the HEK-293 cell line stably expressing the hERG channel, and the risk of the compounds on the inhibitory effect on the cardiac hERG potassium channel was evaluated by fitting a concentration-effect relationship.

1.2 Experimental procedures: The patch clamp operation was performed as follows: Firstly, a capillary glass tube was drawn into a recording electrode with a microelectrode drawing instrument. An electrode filled with the intracellular fluid was mounted on the microelectrode holder. Under an inverted microscope, a microelectrode manipulator was used to immerse the electrode in the extracellular fluid, and the electrode resistance (Rpip) was recorded. The electrode was then slowly brought into contact with the cell surface, and negative pressure was applied to form a GΩ seal. At this time, fast capacitance compensation was performed, and the application of negative pressure was continued to break the cell membrane, forming a whole-cell recording mode. Finally, slow capacitance compensation was performed, and experimental parameters such as series resistance (Rs) were recorded. No current leakage compensation was provided. Administration was started when the hERG current of the whole-cell record was stable. Each drug concentration was maintained for about 5 min (or until the current was stable) for action before the next concentration was measured. Multiple concentrations were measured for each test compound. The coverslip with cells applied thereon was placed in the recording chamber under the inverted microscope. Gravity perfusion was used to flow the blank control extracellular fluid and the working solutions of the test compounds sequentially from low to high concentrations over the cells in the recording chamber. A peristaltic pump was used for liquid exchange during recording. For each cell, the current detected in the compound-free extracellular fluid was used as its own control group. The test was independently repeated three times for each concentration. All electrophysiological experiments were performed at room temperature.

Data processing: First, the tail current *(Peak tail current$_{compound}$)* after the action of each drug concentration was standardized with the tail current of the blank control *(Peak tail current$_{control}$)*, and then the inhibition rate corresponding to each drug concentration was calculated ( $1 - \frac{Peak\ tail\ current_{compound}}{Peak\ tail\ current_{control}}$ ). Subsequently, the mean, standard deviation (SD), and standard error (SE) were calculated for the inhibition rate corresponding to each concentration. The data were represented by Mean ± SE. $IC_{50}$ value calculation of the compounds and dose-effect curve fitting were performed using the GraphPad Prism software. The data obtained are shown in Table 12:

Table 12. hERG channel assay results

| Sample | Example 2 | Example 4 | Example 5 | Example 9 | Example 10 | Example 59 |
|---|---|---|---|---|---|---|
| hERG $IC_{50}$ (μM) | >30 | >30 | >30 | >30 | >30 | >30 |

Conclusion: As shown in the data in Table 12, the sulfonamide compounds of the present disclosure all had $IC_{50}$ for hERG greater than 30 μM, indicating no hERG toxicity.

The above is only intended to illustrate the preferred examples of the present disclosure and is not intended to limit the protection scope of the present disclosure. Any modifications, equivalents, improvements, and the like made by those skilled in the art without departing from the spirit and principle of the present disclosure shall fall within the protection scope of the present disclosure.

## Claims

1. A sulfonamide compound represented by formula (IV) or a stereoisomer, an isotopic derivative, a hydrate, a solvate, a prodrug, or a pharmaceutically acceptable salt thereof:

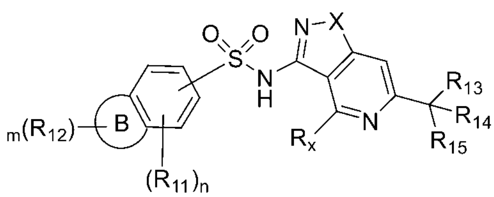

(IV)

wherein

ring B, together with the phenyl to which it is fused, forms a 9- to 10-membered fused heterocyclic ring, wherein the fused heterocyclic ring is a partially saturated or aromatic fused heterocyclic ring, and the ring B moiety contains 1-3 heteroatoms selected from N, O, and S; or the ring B moiety, together with the phenyl to which it is fused, forms a C9-C10 fused carbocyclic ring, wherein the fused carbocyclic ring is a partially saturated or aromatic fused carbocyclic ring;

$R_{11}$, $R_{12}$, $R_{13}$, $R_{14}$, and $R_x$ are identical or different, and are each independently selected from hydrogen, deuterium, halogen, hydroxyl, cyano, substituted or unsubstituted alkyl, substituted or unsubstituted alkoxy, substituted or unsubstituted cycloalkyl, and substituted or unsubstituted alkynyl; preferably, $R_{11}$, $R_{12}$, $R_{13}$, $R_{14}$, and $R_x$ are identical or different, and are each independently selected from hydrogen, deuterium, halogen, hydroxyl, cyano, substituted or unsubstituted C1-C6 alkyl, substituted or unsubstituted C1-C6 alkoxy, substituted or unsubstituted C3-C6 cycloalkyl, and substituted or unsubstituted C2-C6 alkynyl; preferably, $R_{11}$, $R_{12}$, $R_{13}$, $R_{14}$, and $R_x$ are identical or different, and are each independently selected from hydrogen, halogen, hydroxyl, substituted or unsubstituted C1-C3 alkyl, substituted or unsubstituted C1-C3 alkoxy, and substituted or unsubstituted C2-C4 alkynyl; further preferably, $R_{11}$, $R_{12}$, $R_{13}$, $R_{14}$, and $R_x$ are identical or different, and are each independently selected from hydrogen, halogen, hydroxyl, substituted or unsubstituted methyl, substituted or unsubstituted ethyl, substituted or unsubstituted methoxy, substituted or unsubstituted ethoxy, and substituted or unsubstituted ethynyl; the substituents in the groups described above are each independently selected from halogen, hydroxyl, cyano, C1-C6 alkyl, C1-C6 alkoxy, and C3-C6 cycloalkyl; preferably, when the groups described above are substituted, the substituents are identical or different, and are each independently selected from halogen, hydroxyl, cyano, C1-C3 alkyl, C1-C3 alkoxy, and C3-C4 cycloalkyl; further preferably, when the groups described above are substituted, the substituents are identical or different, and are each independently selected from halogen, hydroxyl, cyano, methyl, ethyl, methoxy, ethoxy, cyclopropyl, and cyclobutyl;

$R_{15}$ is selected from the following groups optionally substituted with an $R_6$ substituent: hydroxyl, amino, alkoxy, heterocyclyl, and heteroaryl; preferably, $R_{15}$ is selected from the following groups optionally substituted with an $R_6$ substituent: hydroxyl, amino, C1-C6 alkoxy, 4- to 6-membered heterocyclyl, and 5- to 6-membered heteroaryl, wherein the heterocyclyl contains 1-2 heteroatoms selected from N, O, and S, and the heteroaryl contains 1, 2, or 3 heteroatoms selected from N, O, and S; the $R_6$ substituent is selected from hydrogen, halogen, hydroxyl, cyano, C1-C6 alkyl, C3-C6 cycloalkyl, C1-C6 alkoxy, and C1-C6 alkylcarbonyl;

X is selected from O and S;

m and n are identical or different, and are each independently selected from integers selected from 0, 1, 2, and 3.

**2.** A sulfonamide compound represented by formula (III) or a stereoisomer, an isotopic derivative, a hydrate, a solvate, a prodrug, or a pharmaceutically acceptable salt thereof:

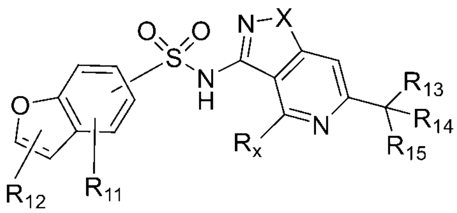

(III)

wherein $\overline{- - -}$ is selected from a single bond and a double bond;

$R_{11}$, $R_{12}$, $R_{13}$, $R_{14}$, and $R_x$ are each independently selected from hydrogen, halogen, hydroxyl, substituted or unsubstituted C1-C6 alkyl, substituted or unsubstituted C1-C6 alkoxy, and substituted or unsubstituted C2-C6 alkynyl; preferably, $R_{11}$, $R_{12}$, $R_{13}$, $R_{14}$, and $R_x$ are each independently selected from hydrogen, halogen, hydroxyl, substituted or unsubstituted C1-C3 alkyl, substituted or unsubstituted C1-C3 alkoxy, and substituted or unsubstituted C2-C4 alkynyl; further preferably, $R_{11}$, $R_{12}$, $R_{13}$, $R_{14}$, and $R_x$ are each independently selected from hydrogen, halogen, hydroxyl, substituted or unsubstituted methyl, substituted or unsubstituted ethyl, substituted or unsubstituted methoxy, substituted or unsubstituted ethoxy, and substituted or unsubstituted ethynyl; the substituents in the groups described above are each independently selected from halogen, hydroxyl, cyano, C1-C6 alkyl, C1-C6 alkoxy, and C3-C6 cycloalkyl; preferably, the substituents in the groups described above are each independently selected from halogen, hydroxyl, cyano, C1-C3 alkyl, C1-C3 alkoxy, and C3-C4 cycloalkyl; further preferably, the substituents in the groups described above are each independently selected from halogen, hydroxyl, cyano, methyl, ethyl, methoxy, ethoxy, cyclopropyl, and cyclobutyl;

$R_{15}$ is selected from 5- to 6-membered heteroaryl containing an $R_6$ substituent; preferably, $R_{15}$ is selected from 5-membered heteroaryl containing an $R_6$ substituent; the $R_6$ substituent is selected from hydrogen, halogen, hydroxyl, cyano, C1-C6 alkyl, C3-C6 cycloalkyl, and C1-C6 alkoxy; preferably, $R_6$ is selected from hydrogen, halogen, hydroxyl, cyano, C1-C3 alkyl, C3-C4 cycloalkyl, and C1-C3 alkoxy; further preferably, $R_6$ is selected from hydrogen, fluoro, methyl, ethyl, methoxy, ethoxy, cyclopropyl, and cyclobutyl;
X is selected from O and S.

**3.** The sulfonamide compound or the stereoisomer, the isotopic derivative, the hydrate, the solvate, the prodrug, or the pharmaceutically acceptable salt thereof according to claim 1, wherein ring B, together with the phenyl to which it is fused, forms a 9- to 10-membered fused heterocyclic ring, wherein the fused heterocyclic ring is a partially saturated or aromatic fused heterocyclic ring, the ring B moiety contains 1-2 heteroatoms selected from N, O, and S, and the heteroatom is linked to the fused phenyl; or the ring B moiety, together with the phenyl to which it is fused, forms a C9-C10 fused carbocyclic ring, wherein the fused carbocyclic ring is a partially saturated or aromatic fused carbocyclic ring, and preferably, the fused carbocyclic ring is a partially saturated fused carbocyclic ring.

**4.** The sulfonamide compound or the stereoisomer, the isotopic derivative, the hydrate, the solvate, the prodrug, or the pharmaceutically acceptable salt thereof according to claim 3, wherein

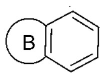

is selected from fused bicyclic ring structures as shown below:

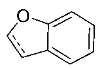 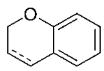 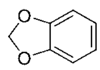 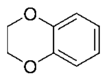 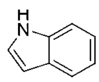 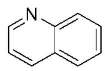

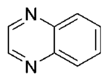 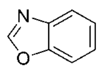 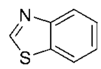 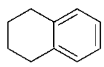 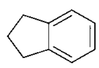

wherein is selected from a single bond and a double bond; preferably, the aminosulfonyl fragment ($-S(=O)_2-NH-$) and $R_{11}$ are located on the phenyl in the fused bicyclic ring structure

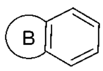;

more preferably, in the case that $R_{11}$ is not hydrogen, the aminosulfonyl fragment and at least one $R_{11}$ are bonded to ortho positions of the phenyl in the fused bicyclic ring structure

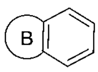.

**5.** The sulfonamide compound or the stereoisomer, the isotopic derivative, the hydrate, the solvate, the prodrug, or the pharmaceutically acceptable salt thereof according to claim 2, wherein the aminosulfonyl fragment ($-S(=O)_2-NH-$) and $R_{11}$ in formula (III) are located on the phenyl of the oxygen-containing fused bicyclic ring; preferably, the aminosulfonyl fragment is located at position 6 or 7 of the oxygen-containing fused bicyclic ring, and $R_{11}$ is located at position 5 or 6 of the fused bicyclic ring; further preferably, the aminosulfonyl fragment and $R_{11}$ are located at ortho positions of the phenyl ring in the oxygen-containing fused bicyclic ring.

**6.** The sulfonamide compound or the stereoisomer, the isotopic derivative, the hydrate, the solvate, the prodrug, or the pharmaceutically acceptable salt thereof according to claim 1 or 2, wherein:

$R_{15}$ is selected from

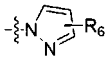
;

$R_6$ is selected from hydrogen, deuterium, halogen, C1-C6 alkyl, C1-C6 alkoxy, and C1-C6 alkylcarbonyl; preferably, $R_6$ is selected from hydrogen, F, methyl, ethyl, methoxy, ethoxy, and acetyl.

**7.** The compound or the stereoisomer, the isotopic derivative, the hydrate, the solvate, the prodrug, or the pharmaceutically acceptable salt thereof according to any one of claims 1-6, which is selected from the following compounds or stereoisomers, isotopic derivatives, hydrates, solvates, prodrugs, or pharmaceutically acceptable salts thereof:

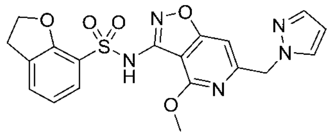
,
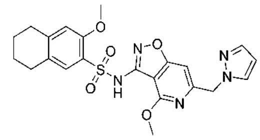
,
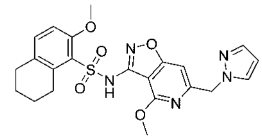
,
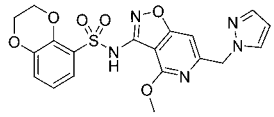
,
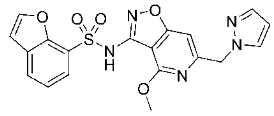
,
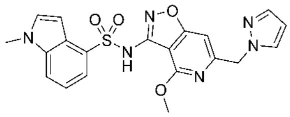
,
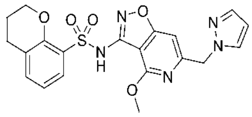
,
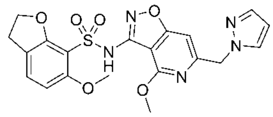
,
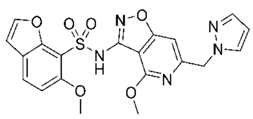
,
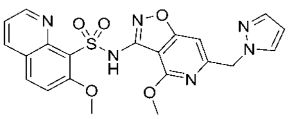
,
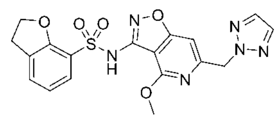
,
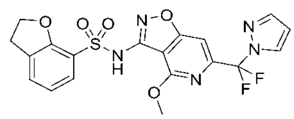

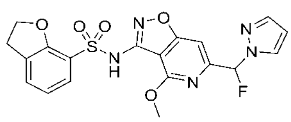
,
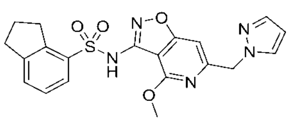
,
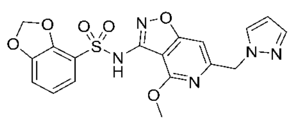
,
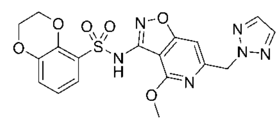

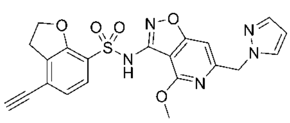
,
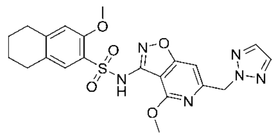
,
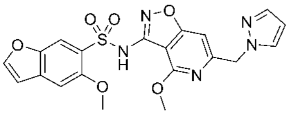
,
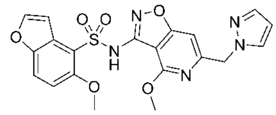

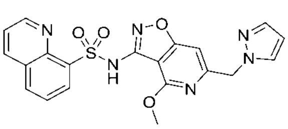
,
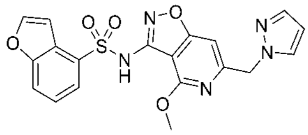
,
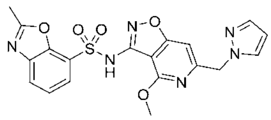
,
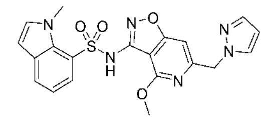

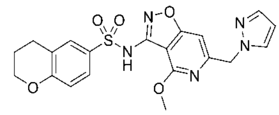
,
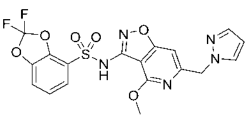
,
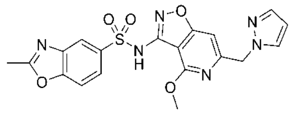
,
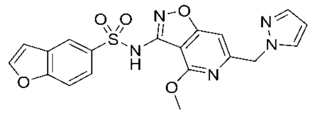

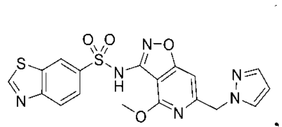
.
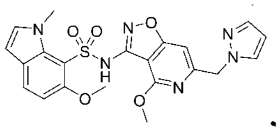
.
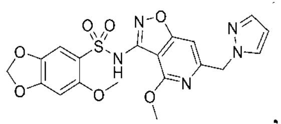
.
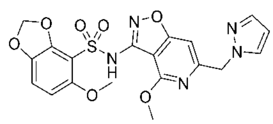

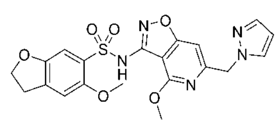, 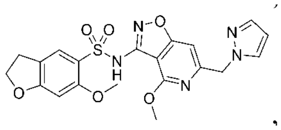, 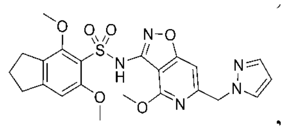, 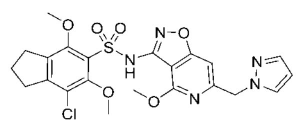,

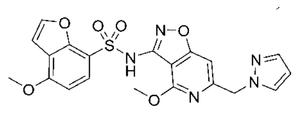, 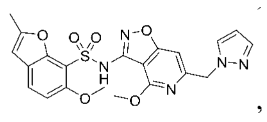, 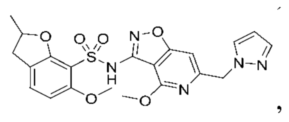, 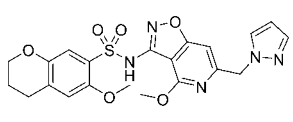,

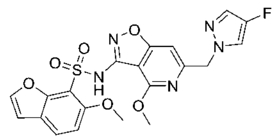, 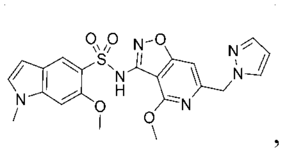, 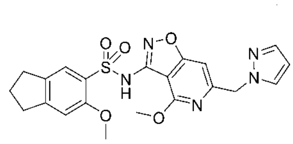, 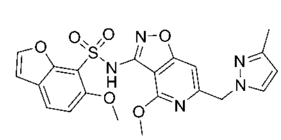,

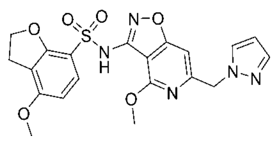, 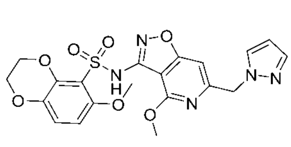, 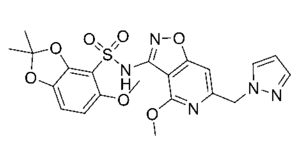, 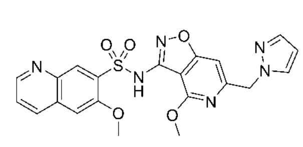,

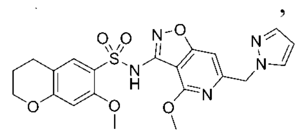, 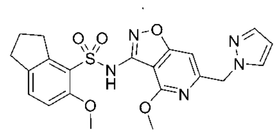, 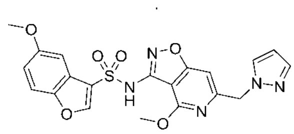, 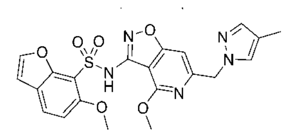,

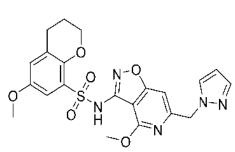, 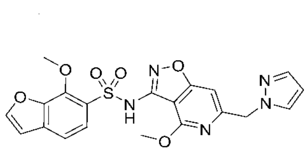, 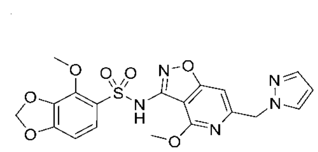, 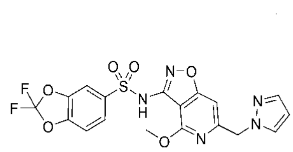,

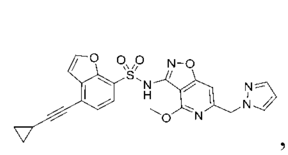, 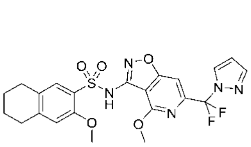, 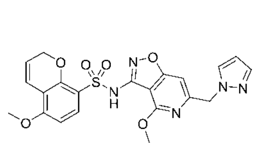, 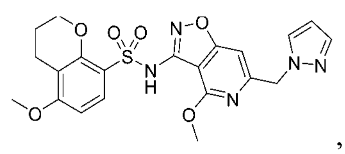,

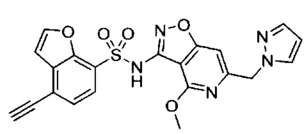, 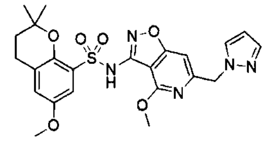, 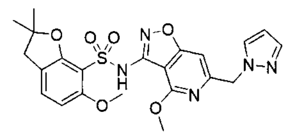, 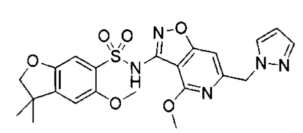,

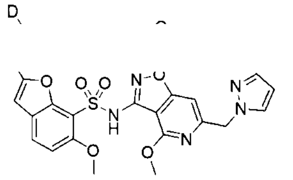, 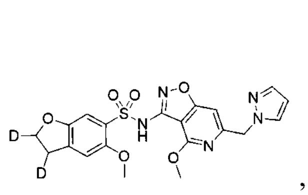, 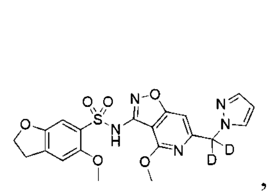, 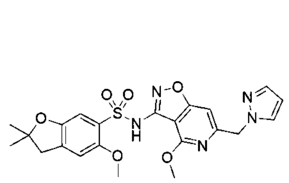,

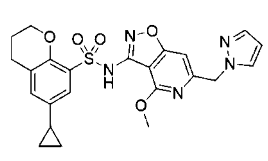, 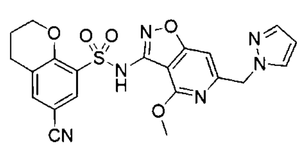, and 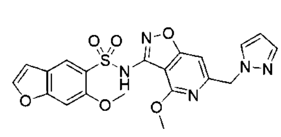.

**8.** A pharmaceutical composition, comprising any one of the compound or the stereoisomer, the isotopic derivative, the hydrate, the solvate, the prodrug, or the pharmaceutically acceptable salt thereof according to any one of claims 1-7, as an active ingredient, and a pharmaceutically acceptable carrier.

**9.** Use of any one of the compound or the stereoisomer, the isotopic derivative, the hydrate, the solvate, the prodrug, or the pharmaceutically acceptable salt thereof according to any one of claims 1-7 for manufacturing a medicament for the treatment and/or prevention of a disease associated with amplification or overexpression of KAT6.

**10.** The use according to claim 9, wherein the disease associated with amplification or overexpression of KAT6 is selected from at least one of breast cancer, triple-negative breast cancer, acute myeloid leukemia, lung adenocarcinoma, hematological tumors, digestive system tumors, reproductive system tumors, nervous system tumors, and head and neck cancer.

**INTERNATIONAL SEARCH REPORT**

International application No.
**PCT/CN2024/083976**

**A. CLASSIFICATION OF SUBJECT MATTER**

C07D498/04(2006.01)i; C07D493/00(2006.01)i; C07D487/00(2006.01)i; A61K31/5365(2006.01)i; A61P35/00(2006.01)i

According to International Patent Classification (IPC) or to both national classification and IPC

**B. FIELDS SEARCHED**

Minimum documentation searched (classification system followed by classification symbols)

IPC C07D A61K A61P

Documentation searched other than minimum documentation to the extent that such documents are included in the fields searched

Electronic data base consulted during the international search (name of data base and, where practicable, search terms used)

CNTXT, ENTXT, DWPI, STN(REGISTRY, CAPLUS, MARPAT), CNKI, Baidu, Google学术, GOOGLE SCHOLAR: 康辰, 杨阳, 张磊, 彭勇, 张贤军, 张晨, 冯开宇, 李玉花, 聂昕, 史子著, 米桢, 张音音, 异恶唑, 吡啶, 磺酰胺, 吡唑, KAT6, Isoxazo? , pyridi? , sulfonamid?, pyrazol?, structural formula search

**C. DOCUMENTS CONSIDERED TO BE RELEVANT**

| Category* | Citation of document, with indication, where appropriate, of the relevant passages | Relevant to claim No. |
|---|---|---|
| PA | WO 2023088233 A1 (INSILICO MEDICINE IP LTD.) 25 May 2023 (2023-05-25)<br>see claims 1, 83, and 90-98, in particular, the 23th compound of claim 83. | 1-10 |
| PA | WO 2023192817 A1 (ISOSTERIX, INC.) 05 October 2023 (2023-10-05)<br>see claims 1, 60, and 93-99 | 1-10 |
| A | WO 2022243983 A1 (AURIGENE DISCOVERY TECHNOLOGIES LIMITED) 24 November 2022 (2022-11-24)<br>see claims 1-28 | 1-10 |
| A | WO 2020254946 A1 (PFIZER et al.) 24 December 2020 (2020-12-24)<br>see claims 1-34, and description, embodiment | 1-10 |
| A | WO 2022013369 A1 (PFIZER et al.) 20 January 2022 (2022-01-20)<br>see description, pages 4-6 | 1-10 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 June 2024** | **17 June 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/083976**

| Patent document cited in search report | | | Publication date (day/month/year) | Patent family member(s) | | | Publication date (day/month/year) |
|---|---|---|---|---|---|---|---|
| WO | 2023088233 | A1 | 25 May 2023 | US | 2024150374 | A1 | 09 May 2024 |
| | | | | US | 2024109880 | A1 | 04 April 2024 |
| | | | | AR | 127681 | A1 | 21 February 2024 |
| | | | | CA | 3237830 | A1 | 25 May 2023 |
| | | | | TW | 202334163 | A | 01 September 2023 |
| WO | 2023192817 | A1 | 05 October 2023 | US | 2023416260 | A1 | 28 December 2023 |
| | | | | US | 11976075 | B2 | 07 May 2024 |
| | | | | US | 2023303580 | A1 | 28 September 2023 |
| | | | | TW | 202400593 | A | 01 January 2024 |
| WO | 2022243983 | A1 | 24 November 2022 | IL | 308667 | A | 01 January 2024 |
| | | | | CA | 3221048 | A1 | 24 November 2022 |
| | | | | AU | 2022278733 | A1 | 30 November 2023 |
| | | | | EP | 4341259 | A1 | 27 March 2024 |
| | | | | KR | 20240012434 | A | 29 January 2024 |
| WO | 2020254946 | A1 | 24 December 2020 | AU | 2023274178 | A1 | 15 February 2024 |
| | | | | PT | 3986890 | T | 17 January 2024 |
| | | | | KR | 20220024671 | A | 03 March 2022 |
| | | | | TW | 202115048 | A | 16 April 2021 |
| | | | | TWI | 787620 | B | 21 December 2022 |
| | | | | MD | 3986890 | T2 | 30 April 2024 |
| | | | | FI | 3986890 | T3 | 15 January 2024 |
| | | | | HUE | 064683 | T2 | 28 April 2024 |
| | | | | BR | 112021025528 | A2 | 19 April 2022 |
| | | | | RS | 64931 | B1 | 29 December 2023 |
| | | | | HRP | 20231501 | T1 | 01 March 2024 |
| | | | | LT | 3986890 | T | 11 December 2023 |
| | | | | EP | 4299135 | A2 | 03 January 2024 |
| | | | | EP | 4299135 | A3 | 28 February 2024 |
| | | | | EP | 3986890 | A1 | 27 April 2022 |
| | | | | EP | 3986890 | B1 | 15 November 2023 |
| | | | | IL | 288802 | A | 01 February 2022 |
| | | | | CR | 20210627 | A | 08 February 2022 |
| | | | | AU | 2020296361 | A1 | 06 January 2022 |
| | | | | CL | 2021003372 | A1 | 07 October 2022 |
| | | | | MX | 2021016085 | A | 20 April 2022 |
| | | | | CO | 2021017504 | A2 | 17 January 2022 |
| | | | | DK | 3986890 | T3 | 18 December 2023 |
| | | | | PL | 3986890 | T3 | 11 March 2024 |
| | | | | UY | 38752 | A | 29 January 2021 |
| | | | | US | 2020399258 | A1 | 24 December 2020 |
| | | | | US | 11492346 | B2 | 08 November 2022 |
| | | | | ECSP | 21091615 | A | 31 January 2022 |
| | | | | SI | 3986890 | T1 | 29 March 2024 |
| | | | | CA | 3143666 | A1 | 24 December 2020 |
| | | | | PE | 20220808 | A1 | 20 May 2022 |
| | | | | JP | 2022537285 | A | 25 August 2022 |
| | | | | JP | 7352662 | B2 | 28 September 2023 |
| | | | | US | 2023174522 | A1 | 08 June 2023 |
| | | | | JP | 2023175821 | A | 12 December 2023 |
| WO | 2022013369 | A1 | 20 January 2022 | KR | 20230058614 | A | 03 May 2023 |

Form PCT/ISA/210 (patent family annex) (July 2022)

**INTERNATIONAL SEARCH REPORT**
**Information on patent family members**

International application No.
**PCT/CN2024/083976**

| Patent document cited in search report | Publication date (day/month/year) | Patent family member(s) | Publication date (day/month/year) |
|---|---|---|---|
| | | JP 2022019654 A | 27 January 2022 |
| | | ZA 202300875 B | 27 September 2023 |
| | | BR 112023000687 A2 | 07 February 2023 |
| | | TW 202216131 A | 01 May 2022 |
| | | MX 2023000735 A | 13 February 2023 |
| | | AU 2021308406 A1 | 23 February 2023 |
| | | IL 299871 A | 01 March 2023 |
| | | EP 4181920 A1 | 24 May 2023 |
| | | CA 3189410 A1 | 20 January 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 202310308970 A
- WO 202311533391 A